(19) Europäisches Patentamt
European Patent Office
Office européen des brevets

(11) EP 2 142 544 B1

(12) # EUROPEAN PATENT SPECIFICATION

(45) Date of publication and mention of the grant of the patent:
**22.05.2013 Bulletin 2013/21**

(21) Application number: **08742864.5**

(22) Date of filing: **11.04.2008**

(51) Int Cl.:
***C07D 471/04*** *(2006.01)* ***A61K 31/519*** *(2006.01)*
***A61P 35/00*** *(2006.01)*

(86) International application number:
**PCT/US2008/004807**

(87) International publication number:
**WO 2008/127712 (23.10.2008 Gazette 2008/43)**

(54) **PYRIDO [2, 3-D]PYRIMIDIN-7-ONE COMPOUNDS AS INHIBITORS OF PI3K-ALPHA FOR THE TREATMENT OF CANCER**

PYRIDO [2,3-D]PYRIMIDIN-7-ONVERBINDUNGEN ALS INHIBITOREN VON PI3K-ALPHA ZUR BEHANDLUNG VON KREBS

COMPOSÉS PYRIDO [2, 3-D]PYRIMIDIN-7-ONE UTILISÉS EN TANT QU'INHIBITEURS DE PI3K-ALPHA POUR LE TRAITEMENT DU CANCER

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **11.04.2007 US 911160 P**

(43) Date of publication of application:
**13.01.2010 Bulletin 2010/02**

(73) Proprietor: **Exelixis, Inc.**
**South San Francisco, CA 94083 (US)**

(72) Inventors:
- **BUHR, Chris A.**
  **Redwood City, California 94061 (US)**
- **WANG, Longcheng**
  **Palo Alto, California 94306 (US)**

(74) Representative: **Main, Malcolm Charles**
**Murgitroyd & Company**
**Immeuble Atlantis**
**55 Allee Pierre Ziller**
**06560 Valbonne - Sophia Antipolis (FR)**

(56) References cited:
**WO-A-96/34867**
**WO-A-2005/105801**
**WO-A-2008/021389**
**WO-A1-2007/044698**
**WO-A1-2008/032162**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 142 544 B1

## Description

Cross-Reference to Related Applications

The Applicants claim priority under 35 U.S.C. 119(e) to copending Provisional Application No. 60/911,160 filed on April 11, 2007.

Field of the Invention

This invention relates to the field of protein kinases and inhibitors thereof. In particular, the invention relates to inhibitors of phosphatidylinositol 3-kinase (PI3K) signaling pathways, and methods of their use.

Background

Phosphatidylinositol 3-kinase (PI3Kα), a dual specificity lipid kinase, is composed of an 85 kDa regulatory subunit and a 110 kDa catalytic subunit. The protein encoded by this gene represents the catalytic subunit, which uses ATP to phosphorylate PtdIns, PtdIns4P and PtdIns(4,5)P2. PTEN, a tumor suppressor which inhibits cell growth through multiple mechanisms, can dephosphorylate PIP3, the major product of PIK3CA. PIP3, in turn, is required for translocation of protein kinase B (AKT1, PKB) to the cell membrane, where it is phosphorylated and activated by upstream kinases. The effect of PTEN on cell death is mediated through the PIK3CA/AKT1 pathway.

PI3Kα has been implicated in the control of cytoskeletal reorganization, apoptosis, vesicular trafficking, proliferation and differentiation processes. Increased copy number and expression of PIK3CA or activating mutations in the p110a catalytic subunit of PI3KCA are associated with a number of malignancies such as ovarian cancer (Campbell et al., Cancer Res 2004, 64, 7678-7681; Levine et al., Clin Cancer Res 2005, 11, 2875-2878; Wang et al., Hum Mutat 2005, 25, 322; Lee et al., Gynecol Oncol 2005, 97, 26-34), cervical cancer, breast cancer (Bachman, et al. Cancer Biol Ther 2004, 3, 772-775; Levine, et al., supra; Li et al., Breast Cancer Res Treat 2006, 96, 91-95; Saal et al., Cancer Res 2005, 65, 2554-2559; Samuels and Velculescu, Cell Cycle 2004, 3, 1221-1224), colorectal cancer (Samuels, et al. Science 2004, 304, 554; Velho et al. Eur J Cancer 2005, 41, 1649-1654), endometrial cancer (Oda et al. Cancer Res. 2005, 65, 10669-10673), gastric carcinomas (Byun et al., Int J Cancer 2003, 104, 318-327; Li et al., supra; Velho et al., supra; Lee et al., Oncogene 2005, 24, 1477-1480), hepatocellular carcinoma (Lee et al., *id.),* small and non-small cell lung cancer (Tang et al ., Lung Cancer 2006, 51, 181-191; Massion et al., Am J Respir Crit Care Med 2004, 170, 1088-1094), thyroid carcinoma (Wu et al., J Clin Endocrinol Metab 2005, 90, 4688-4693), acute myelogenous leukemia (AML) (Sujobert et al., Blood 1997, 106, 1063-1066), chronic myelogenous leukemia (CML) (Hickey and Cotter J Biol Chem 2006, 281, 2441-2450), and glioblastomas (Hartmann et al. Acta Neuropathol (Berl) 2005, 109, 639-642; Samuels et al., supra). WO 96/34867 describes 6-aryl pyrido [2,3-d] pyrimidines and their use as inhibitors of proteine tyrosine kinases. WO 2005/105801 describes pyrrolyl substituted pyrido [2,3-D] pyrimidin-7-ones and their use as therapeutic agents.

In view of the important role of PI3Kα in biological processes and disease states, inhibitors of this protein kinase are desirable.

## SUMMARY OF THE INVENTION

The following only summarizes certain aspects of the invention and is not intended to be limiting in nature. These aspects and other aspects and embodiments are described more fully below. In the event of a discrepancy between the express disclosure of this specification and the references incorporated by reference, the express disclosure of this specification shall control.

The invention provides compounds that inhibit, regulate, and/or modulate PI3K that are useful in the treatment of hyperproliferative diseases, such as cancer, in humans. This invention also provides methods of making the compound, such compounds for use in the treatment of hyperproliferative diseases in humans and to pharmaceutical compositions containing such compounds.

A first aspect of the invention provides a compound of Formula I:

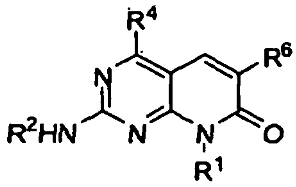

I

optionally as a pharmaceutically acceptable salt, additionally optionally as a solvate, and additionally optionally as a hydrate thereof, wherein
$R^1$ is a 5- or 6- membered heterocycloalkyl;
$R^2$ is hydrogen or alkyl;
$R^4$ is alkyl;
$R^6$ is a 5- or 6-membered heteroaryl optionally substituted with 1, 2, 3, 4, or 5 $R^9$ groups; and
each $R^9$, when present, is independently halo, alkyl, haloalkyl, alkoxy, haloalkoxy, cyano, amino, alkylamino, dialkylamino, alkoxyalkyl, carboxyalkyl, alkoxycarbonyl, aminoalkyl, cycloalkyl, aryl, arylalkyl, aryloxy, heterocycloalkyl, or heteroaryl and where the cycloalkyl, aryl, heterocycloalkyl, and heteroaryl, each either alone or as part of another group within $R^9$, are independently optionally substituted with 1, 2, 3, or 4 groups selected from halo, alkyl, haloalkyl, hydroxy, alkoxy, haloalkoxy, amino, alkylamino, and dialkylamino.

In a second aspect, the invention is directed to a pharmaceutical composition which comprises a compound of Formula I optionally as a pharmaceutically acceptable salt, solvate, and/or hydrate thereof, and a pharmaceutically acceptable carrier, excipient, or diluent.

In a third aspect, described herein is a method of inhibiting PI3K, comprising contacting a cell with a compound of Formula I or a single isomer thereof, optionally as a pharmaceutically acceptable salt, solvate, and/or hydrate thereof, or contacting a cell with a pharmaceutical composition comprising a therapeutically effective amount of a compound of Formula I or a single isomer thereof and a pharmaceutically acceptable carrier, excipient, or diluent.

In a fourth aspect described herein is a method of inhibiting the *in vivo* activity of PI3Kα, the method comprising administering to a patient an effective PI3Kα-inhibiting-inhibiting amount of a compound of Formula I or a single isomer thereof, optionally as a pharmaceutically acceptable salt, solvate, and/or hydrate thereof, or administering a pharmaceutical composition thereof.

In a fifth aspect, the Invention provides a compound of the first aspect of the invention optionally as a pharmaceutically acceptable salt, solvate, and/or hydrate thereof for use in medicine.

In a sixth aspect, the Invention is directed to a compound of the first aspect of the invention optionally as a pharmaceutically acceptable acceptable salt, solvate, and/or hydrate thereof for use in the treatment of cancer. In one embodiment, said use is in combination with one or more treatments selected from surgery, one or more chemotherapeutic agents, one or more hormone therapies, one or more antibodies, one or more immunotherapies, radioactive iodine therapy, and radiation.

A seventh aspect of the invention is directed to a process of preparing a compound of the first aspect of the invention, comprising:

(a) reacting an intermediate of formula 1:

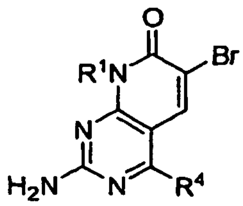

1

where $R^1$, $R^2$, and $R^4$ are as defined in the first aspect of the invention; with an intermediate of formula $R^6Sn(n\text{-}Bu)_3$ or $R^6B(OH)_2$ where $R^6$ and $R^9$ are as defined in the first aspect of the invention, to yield a Compound of Formula I:

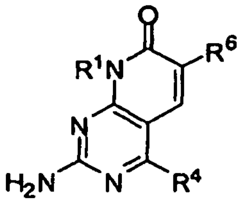

I

;

(b) optionally further modifying one of the $R^1$, $R^2$, $R^4$, and $R^6$ groups; and
(c) optionally forming a pharmaceutically acceptable salt, solvate, and/or hydrate thereof.

## DETAILED DESCRIPTION OF THE INVENTION

### Abbreviations and Definitions

The following abbreviations and terms have the indicated meanings throughout:

| Abbreviation | Meaning |
|---|---|
| Ac | acetyl |
| br | broad |
| °C | degrees Celsius |
| c- | cyclo |
| CBZ | CarboBenZoxy = benzyloxycarbonyl |
| d | doublet |
| dd | doublet of doublet |
| dt | doublet of triplet |
| DBU | 1,8-diazabicyclo[5.4.0]undec-7-ene |
| DCM | dichloromethane |
| DIEA | *N,N*-diisopropylethylamine |
| DME | 1,2-dimethoxyethane |
| DMF | *N,N*-dimethylformamide |
| DMSO | dimethyl sulfoxide |
| dppf | 1,1'-bis(diphenylphosphano)ferrocene |
| EI | Electron Impact ionization |
| EtOAc | ethyl acetate |
| g | gram(s) |
| h or hr | hour(s) |
| HPLC | high pressure liquid chromatography |
| L | liter(s) |
| M | molar or molarity |
| m | Multiplet |
| MeOH | methanol |
| mg | milligram(s) |
| MHz | megahertz (frequency) |

(continued)

| Abbreviation | Meaning |
|---|---|
| Min | minute(s) |
| mL | milliliter(s) |
| μL | microliter(s) |
| μM | Micromole(s) or micromolar |
| mM | Millimolar |
| mmol | millimole(s) |
| mol | mole(s) |
| MS | mass spectral analysis |
| N | normal or normality |
| nM | Nanomolar |
| NMR | nuclear magnetic resonance spectroscopy |
| q | Quartet |
| RT | Room temperature |
| s | Singlet |
| sat. | saturated |
| t or tr | Triplet |
| TEA | triethylamine |
| TFA | trifluoroacetic acid |
| THF | tetrahydrofuran |
| TLC | thin layer chromatography |

The symbol "-" means a single bond, "=" means a double bond, "≡" means a triple bond, "⎓" means a single or double bond. The symbol "∿∿∿" refers to a group on a double-bond as occupying either position on the terminus of a double bond to which the symbol is attached; that is, the geometry, E- or Z-, of the double bond is ambiguous. When a group is depicted removed from its parent formula, the "∼" symbol will be used at the end of the bond which was theoretically cleaved in order to separate the group from its parent structural formula.

When chemical structures are depicted or described, unless explicitly stated otherwise, all carbons are assumed to have hydrogen substitution to conform to a valence of four. For example, in the structure on the left-hand side of the schematic below there are nine hydrogens implied. The nine hydrogens are depicted in the right-hand structure. Sometimes a particular atom in a structure is described in textual formula as having a hydrogen or hydrogens as substitution (expressly defined hydrogen), for example, -CH CH -. It is understood by one of ordinary skill in the art that the aforementioned descriptive techniques are common in the chemical arts to provide brevity and simplicity to description of otherwise complex structures.

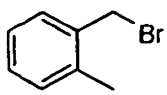
=
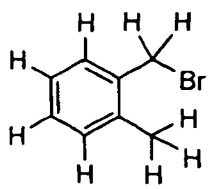

If a group "R" is depicted as "floating" on a ring system, as for example in the formula:

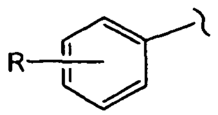

then, unless otherwise defined, a substituent "R" may reside on any atom of the ring system, assuming replacement of a depicted, implied, or expressly defined hydrogen from one of the ring atoms, so long as a stable structure is formed.

If a group "R" is depicted as floating on a fused ring system, as for example in the formulae:

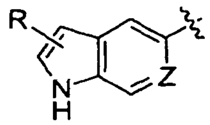 , or 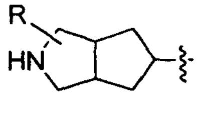 , or 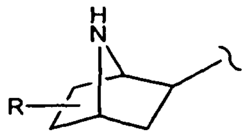

then, unless otherwise defined, a substituent "R" may reside on any atom of the fused ring system, assuming replacement of a depicted hydrogen (for example the -NH- in the formula above), implied hydrogen (for example as in the formula above, where the hydrogens are not shown but understood to be present), or expressly defined hydrogen (for example where in the formula above, "Z" equals =CH-) from one of the ring atoms, so long as a stable structure is formed. In the example depicted, the "R" group may reside on either the 5-membered or the 6-membered ring of the fused ring system. In the formula depicted above, when y is 2 for example, then the two "R's" may reside on any two atoms of the ring system, again assuming each replaces a depicted, implied, or expressly defined hydrogen on the ring.

When a group "R" is depicted as existing on a ring system containing saturated carbons, as for example in the formula:

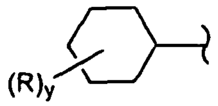

where, in this example, "y" can be more than one, assuming each replaces a currently depicted, implied, or expressly defined hydrogen on the ring; then, unless otherwise defined, where the resulting structure is stable, two "R's" may reside on the same carbon. A simple example is when R is a methyl group; there can exist a geminal dimethyl on a carbon of the depicted ring (an "annular" carbon).

"Acyl" means a -C(O)R radical where R is optionally substituted alkyl, optionally substituted alkenyl, cycloalkyl, cycloalkylalkyl, aryl, aralkyl, heteroaryl, heteroaralkyl, heterocycloalkyl, or heterocycloalkylalkyl, as defined herein, e.g., acetyl, trifluoromethylcarbonyl, or 2-methoxyethylcarbonyl, and the like.

"Acylamino" means a -NRR' radical where R is hydrogen, hydroxy, alkyl, or alkoxy and R' is acyl, as defined herein.

"Acyloxy" means an -OR radical where R is acyl, as defined herein, e.g. cyanomethylcarbonyloxy, and the like.

"Administration" and variants thereof (e.g., "administering" a compound) in reference to a compound of the invention means introducing the compound or a prodrug of the compound into the system of the animal in need of treatment. When a compound of the invention or prodrug thereof is provided in combination with one or more other active agents (e.g., surgery, radiation, and chemotherapy, etc.), "administration" and its variants are each understood to include concurrent and sequential introduction of the compound or prodrug thereof and other agents.

"Alkenyl" means a means a linear monovalent hydrocarbon radical of two to six carbon atoms or a branched monovalent hydrocarbon radical of three to 6 carbon atoms which radical contains at least one double bond, e.g., ethenyl, propenyl, 1-but-3-enyl, and 1-pent-3-enyl, and the like.

"Alkoxy" means an -OR group where R is alkyl group as defined herein. Examples include methoxy, ethoxy, propoxy, isopropoxy, and the like.

"Alkoxyalkyl" means an alkyl group, as defined herein, substituted with at least one, preferably one, two, or three, alkoxy groups as defined herein. Representative examples include methoxymethyl and the like.

"Alkoxycarbonyl" means a -C(O)R group where R is alkoxy, as defined herein.

"Alkyl" means a linear saturated monovalent hydrocarbon radical of one to six carbon atoms or a branched saturated monovalent hydrocarbon radical of three to 6 carbon atoms, e.g., methyl, ethyl, propyl, 2-propyl, butyl (including all isomeric forms), or pentyl (including all isomeric forms), and the like.

"Alkylamino" means an -NHR group where R is alkyl, as defined herein.

"Alkylaminoalkyl" means an alkyl group substituted with one or two alkylamino groups, as defined herein.

"Alkylaminoalkyloxy" means an -OR group where R is alkylaminoalkyl, as defined herein.

"Alkylcarbonyl" means a -C(O)R group where R is alkyl, as defined herein.

"Alkynyl" means a linear monovalent hydrocarbon radical of two to six carbon atoms or a branched monovalent hydrocarbon radical of three to 6 carbon atoms which radical contains at least one triple bond, e.g., ethynyl, propynyl, butynyl, pentyn-2-yl and the like.

"Amino" means $-NH_2$.

"Aminoalkyl" means an alkyl group substituted with at least one, specifically one, two or three, amino groups.

"Aminoalkyloxy" means an -OR group where R is aminoalkyl, as defined herein.

"Aryl" means a monovalent six- to fourteen-membered, mono- or bi-carbocyclic ring, wherein the monocyclic ring is aromatic and at least one of the rings in the bicyclic ring is aromatic. Unless stated otherwise, the valency of the group may be located on any atom of any ring within the radical, valency rules permitting. Representative examples include phenyl, naphthyl, and indanyl, and the like.

"Arylalkyl" means an alkyl radical, as defined herein, substituted with one or two aryl groups, as defined herein, e.g., benzyl and phenethyl, and the like.

"Aryloxy" means an -OR gorup where R is aryl, as defined herein.

"Carboxyalkyl" means an alkyl group, as defined herein, substituted with at least one, specifically one or two, -C(O)OH group(s).

"Cycloalkyl" means a monocyclic or fused bicyclic, saturated or partially unsaturated (but not aromatic), monovalent hydrocarbon radical of three to ten carbon ring atoms. Fused bicyclic hydrocarbon radical includes bridged ring systems. Unless stated otherwise, the valency of the group may be located on any atom of any ring within the radical, valency rules permitting. One or two ring carbon atoms may be replaced by a -C(O)-, -C(S)-, or -C(=NH)- group. More specifically, the term cycloalkyl includes, but is not limited to, cyclopropyl, cyclobutyl, cyclopentyl, cyclohexyl, cyclohexyl, or cyclohex-3-enyl, and the like.

"Cycloalkylalkyl" means an alkyl group substituted with at least one, specifically one or two, cycloalkyl group(s) as defined herein.

"Dialkylamino" means a -NRR' radical where R and R' are alkyl as defined herein, or an N-oxide derivative, or a protected derivative thereof, e.g., dimethylamino, diethylamino, *N,N*-methylpropylamino or *N,N*-methylethylamino, and the like.

"Dialkylaminoalkyl" means an alkyl group substituted with one or two dialkylamino groups, as defined herein.

"Dialkylaminoalkyloxy" means an -OR group where R is dialkylaminoalkyl, as defined herein. Representative examples include 2-(*N,N*-diethylamino)-ethyloxy, and the like.

"Halogen" or "halo" refers to fluorine, chlorine, bromine and iodine.

"Haloalkoxy" means an -OR' group where R' is haloalkyl as defined herein, e.g., trifluoromethoxy or 2,2,2-trifluoroethoxy, and the like.

"Haloalkyl" mean an alkyl group substituted with one or more halogens, specifically one to five halo atoms, e.g., trifluoromethyl, 2-chloroethyl, and 2,2-difluoroethyl, and the like.

"Heteroaryl" means a monocyclic, fused bicyclic, or fused tricyclic, monovalent radical of 5 to 14 ring atoms containing one or more, specifically one, two, three, or four ring heteroatoms independently selected from -O-, $-S(O)_n$ (n is 0, 1, or 2), -N-, $-N(R^x)-$, and the remaining ring atoms being carbon, wherein the ring comprising a monocyclic radical is aromatic and wherein at least one of the fused rings comprising a bicyclic or tricyclic radical is aromatic. One or two ring carbon atoms of any nonaromatic rings comprising a bicyclic or tricyclic radical may be replaced by a -C(O)-, -C(S)-, or -C(=NH)- group. $R^x$ is hydrogen, alkyl, hydroxy, alkoxy, acyl, or alkylsulfonyl. Fused bicyclic radical includes bridged ring systems. Unless stated otherwise, the valency may be located on any atom of any ring of the heteroaryl group, valency rules permitting. When the point of valency is located on the nitrogen, $R^x$ is absent. More specifically, the term heteroaryl includes, but is not limited to, 1,2,4-triazolyl, 1,3,5-triazolyl, phthalimidyl, pyridinyl, pyrrolyl, imidazolyl, thienyl, furanyl, indolyl, 2,3-dihydro-1*H*-indolyl (including, for example, 2,3-dihydro-1*H*-indol-2-yl or 2,3-dihydro-1*H*-indol-5-yl, and the like), isoindolyl, indolinyl, isoindolinyl, benzimidazolyl, benzodioxol-4-yl, benzofuranyl, cinnolinyl, indolizinyl, naphthyridin-3-yl, phthalazin-3-yl, phthalazin-4-yl, pteridinyl, purinyl, quinazolinyl, quinoxalinyl, tetrazoyl, pyrazolyl, pyrazinyl, pyrimidinyl, pyridazinyl, oxazolyl, isooxazolyl, oxadiazolyl, benzoxazolyl, quinolinyl, isoquinolinyl, tetrahydroisoquinolinyl (including, for example, tetrahydroisoquinolin-4-yl or tetrahydroisoquinolin-6-yl, and the like), pyrrolo[3,2-c]pyridinyl (including, for example, pyrrolo[3,2-c]pyridin-2-yl or pyrrolo[3,2-c]pyridin-7-yl, and the like), benzopyranyl, thiazolyl, isothiazolyl, thiadiazolyl, benzothiazolyl, benzothienyl, and the derivatives thereof, or N-oxide or a protected derivative thereof. The term "heteroaryl" includes, but is not limited to, the term "5- or 6-membered heteroaryl."

"Heteroatom" refers to O, S, N, and P.

"Heterocycloalkyl" means a saturated or partially unsaturated (but not aromatic) monovalent monocyclic group of 3 to 8 ring atoms or a saturated or partially unsaturated (but not aromatic) monovalent fused bicyclic group of 5 to 12 ring atoms in which one or more, specifically one, two, three, or four ring heteroatoms independently selected from O, $S(O)_n$ (n is 0, 1, or 2), N, $N(R^y)$ (where $R^y$ is hydrogen, alkyl, hydroxy, alkoxy, acyl, or alkylsulfonyl), the remaining ring

atoms being carbon. One or two ring carbon atoms may be replaced by a -C(O)-, -C(S)-, or -C(=NH)- group. Fused bicyclic radical includes bridged ring systems. Unless otherwise stated, the valency of the group may be located on any atom of any ring within the radical, valency rules permitting. When the point of valency is located on a nitrogen atom, $R^y$ is absent. More specifically the term heterocycloalkyl includes, but is not limited to, azetidinyl, pyrrolidinyl, 2-oxopyrrolidinyl, 2,5-dihydro-1*H*-pyrrolyl, piperidinyl, 4-piperidonyl, morpholinyl, piperazinyl, 2-oxopiperazinyl, tetrahydropyranyl, 2-oxopiperidinyl, thiomorpholinyl, thiamorpholinyl, perhydroazepinyl, pyrazolidinyl, imidazolinyl, imidazolidinyl, dihydropyridinyl, tetrahydropyridinyl, oxazolinyl, oxazolidinyl, isoxazolidinyl, thiazolinyl, thiazolidinyl, quinuclidinyl, isothiazolidinyl, octahydroindolyl, octahydroisoindolyl, decahydroisoquinolyl, tetrahydrofuryl, and tetrahydropyranyl, and the derivatives thereof and N-oxide or a protected derivative thereof. The term "heterocycloalkyl" includes, but is not limited to, the term "5- or 6-membered heterocycloalkyl;" however "5- or 6-membered heterocycloalkyl," when used, specifically means a heterocycloalkyl ring which contains five or six ring members and which may include single isomers and mixtures of isomers.

"Heterocycloalkylalkyl" means an alkyl radical, as defined herein, substituted with one or two heterocycloalkyl groups, as defined herein, e.g., morpholinylmethyl, *N*-pyrrolidinylethyl, and 3-(*N*-azetidinyl)propyl, and the like.

"Optional" or "optionally" means that the subsequently described event or circumstance may or may not occur, and that the description includes instances where said event or circumstance occurs and instances in which it does not. One of ordinary skill in the art would understand that with respect to any molecule described as containing one or more optional substituents, only sterically practical and/or synthetically feasible compounds are meant to be included.

"Optionally substituted alkyl" means an alkyl radical, as defined herein, optionally substituted with one or more groups, in another example one, two, three, four, or five groups, independently selected from alkylcarbonyl, alkenylcarbonyl, cycloalkylcarbonyl, alkylcarbonyloxy, alkenylcarbonyloxy, amino, alkylamino, dialkylamino, aminocarbonyl, alkylaminocarbonyl, dialkylaminocarbonyl, cyano, cyanoalkylaminocarbonyl, alkoxy, alkenyloxy, hydroxy, hydroxyalkoxy, halo, carboxy, alkylcarbonylamino, alkylcarbonyloxy, alkyl-$S(O)_2$-, alkenyl-$S(O)_2$-, aminosulfonyl, alkylaminosulfonyl, dialkylaminosulfonyl, alkylsulfonyl-$NR^c$- (where $R^c$ is hydrogen, alkyl, optionally substituted alkenyl, hydroxy, alkoxy, alkenyloxy, or cyanoalkyl), alkylaminocarbonyloxy, dialkylaminocarbonyloxy, alkylaminoalkyloxy, dialkylaminoalkyloxy, alkoxycarbonyl, alkenyloxycarbonyl, alkoxycarbonylamino, alkylaminocarbonylamino, dialkylaminocarbonylamino, alkoxyalkyloxy, and -C(O)$NR^aR^b$ (where $R^a$ and $R^b$ are independently hydrogen, alkyl, optionally substituted alkenyl, hydroxy, alkoxy, alkenyloxy, or cyanoalkyl).

"Optionally substituted alkenyl" means an alkenyl radical, as defined herein, optionally substituted with one or more group(s), specifically one, two, three, four, or five groups, independently selected from alkylcarbonyl, alkenylcarbonyl, cycloalkylcarbonyl, alkylcarbonyloxy, alkenylcarbonyloxy, amino, alkylamino, dialkylamino, aminocarbonyl, alkylaminocarbonyl, dialkylaminocarbonyl, cyano, cyanoalkylaminocarbonyl, alkoxy, alkenyloxy, hydroxy, hydroxyalkoxy, halo, carboxy, alkylcarbonylamino, alkylcarbonyloxy, alkyl-$S(O)_2$-, alkenyl-$S(O)_2$-, aminosulfonyl, alkylaminosulfonyl, dialkylaminosulfonyl, alkylsulfonyl-$NR^c$- (where $R^c$ is hydrogen, alkyl, optionally substituted alkenyl, hydroxy, alkoxy, alkenyloxy, or cyanoalkyl), alkylaminocarbonyloxy, dialkylaminocarbonyloxy, alkylaminoalkyloxy, dialkylaminoalkyloxy, alkoxycarbonyl, alkenyloxycarbonyl, alkoxycarbonylamino, alkylaminocarbonylamino, dialkylaminocarbonylamino, alkoxyalkyloxy, and -C(O)$NR^aR^b$ (where $R^a$ and $R^b$ are independently hydrogen, alkyl, optionally substituted alkenyl, hydroxy, alkoxy, alkenyloxy, or cyanoalkyl).

"Optionally substituted 5- or 6-membered heterocycloalkyl" means a heterocycloalkyl group, as defined herein and where the ring comprises five or six atoms, optionally substituted with one, two, or three substituents independently selected from acyl, acylamino, acyloxy, optionally substituted alkyl, optionally substituted alkenyl, alkoxy, alkenyloxy, halo, hydroxy, alkoxycarbonyl, alkenyloxycarbonyl, amino, alkylamino, dialkylamino, nitro, aminocarbonyl, alkylaminocarbonyl, dialkylaminocarbonyl, carboxy, cyano, alkylthio, alkylsulfinyl, alkylsulfonyl, aminosulfonyl, alkylaminosulfonyl, dialkylaminosulfonyl, alkylsulfonylamino, aminoalkoxy, or aryl is pentafluorophenyl. Within the optional substituents on "heterocycloalkyl", the alkyl and alkenyl, either alone or as part of another group (including, for example, the alkyl in alkoxycarbonyl), are independently optionally substituted with one, two, three, four, or five halo.

"Yield" for each of the reactions described herein is expressed as a percentage of the theoretical yield.

"AKT inhibitor" includes, for example, LY294002, PKC 412, perifosine, compounds in Table 2a, compounds in Table 2b, and compounds described in WO 2006/071819 and WO05/117909. These references also describe in vitro assays that can be used to determine the inhibitory activity of AKT.

"Alkylating agent" includes, for example, one or more of the following: Chlorambucil, Chlormethine, Cyclophosphamide, Ifosfamide, Melphalan, Carmustine, Streptozocin, Fotemustine, Lomustine, Streptozocin, Carboplatin, Cisplatin, Oxaliplatin, BBR3464, Busulfan, Dacarbazine, Mechlorethamine, Procarbazine, Temozolomide, ThioTEPA, and Uramustine.

"Antibody" includes, for example, one or more of the following: an IGF1R antibody (including, for example, $^{\alpha}$IGF-1R A12 MoAb, 19D12, h7C10 and CP-751871), an EGFR antibody (including, for example, Cetuximab (Erbitux®) and Panitumumab), an ErbB2 antibody (including, for example, Trastuzumab (Herceptin®)), a VEGF antibody (including, for example, Bevacizumab (Avastin®)), an IgG1 antibody (including, for example, Ibritumomab (tiuxetan)), a CD20 antibody

(including, for example, Rituximab and Tositumomab), a CD33 antibody (including, for example, Gemtuzumab and Gemtuzumab ozogamicin), and a CD52 antibody (including, for example, Alemtuzumab).

"Antimetabolite" includes, for example, methotrexate, Pemetrexed, Raltitrexed, Cladribine, Clofarabine, Fludarabine, Mercaptopurine, Thioguanine, Capecitabine, Cytarabine, fluorouracil (administered with or without leucovorin or folinic acid), and Gemcitabine.

"Antimicrotubule agent" includes, for example, Vincristine, Vinblastine, Vinorelbine, Vinflunine, and Vindesine.

"Aromatase inhibitor" includes, for example, one or more of the following: Aminoglutethimide, Anastrozole (Arimidex®), Letrozole (Femara®), Exemestane (Aromasin®), and Formestane (Lentaron®).

"Cancer" refers to cellular-proliferative disease states, including but not limited to: <u>Cardiac:</u> sarcoma (angiosarcoma, fibrosarcoma, rhabdomyosarcoma, liposarcoma), myxoma, rhabdomyoma, fibroma, lipoma and teratoma; <u>Lung</u>: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hanlartoma, inesothelioma; <u>Gastrointestinal:</u> esophagus (squamous cell carcinoma, adenocarcinoma, leiomyosarcoma, lymphoma), stomach (carcinoma, lymphoma, leiomyosarcoma), pancreas (ductal adenocarcinoma, insulinorna, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); <u>Genitourinary tract:</u> kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); <u>Liver</u>: hepatoma (hepatocellular carcinoma), cholangiocarcinoma, hepatoblastoma, angiosarcoma, hepatocellular adenoma, hemangioma; <u>Bone:</u> osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; <u>Nervous system:</u> skull (osteoma, hemangioma, granuloma, xanthoma, osteitis defornians), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); <u>Gynecological</u>: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); <u>Hematologic:</u> blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; <u>Skin:</u> malignant melanoma, basal cell carcinoma, squamous cell carcinoma, Karposi's sarcoma, moles dysplastic nevi, lipoma, angioma, dermatofibroma, keloids, psoriasis; <u>Adrenal Glands:</u> neuroblastoma; and breast cancer. Thus, the term "cancerous cell" as provided herein, includes a cell afflicted by any one of the above-identified conditions.

"Chemotherapeutic agent" includes, but is not limited to, an AKT inhibitor, an alkylating agent, an antimetabolite, an antimicrotubule agent, an aromatase inhibitor, a c-KIT inhibitor, a cMET inhibitor, an EGFR inhibitor, an ErbB2 inhibitor, a Flt-3 inhibitor, an HSP90 inhibitor, an IGF1R inhibitor, a platin, a Raf inhibitor, rapamycin, a Rapamycin analogue, a Receptor Tyrosine Kinase inhibitor, a taxane, a topoisomerase inhibitor, a SRC and/or ABL kinase inhibitor, and a VEGFR inhibitor. A pharmaceutically acceptable salt, solvate, and/or hydrate of a chemotherapeutic agent can be prepared by one of ordinary skill in the art and such salt, solvate, and/or hydrates thereof can be used to practice the invention.

"c-KIT inhibitor" includes, for example, imatinib, sunitinib, nilotinib, AMG 706, sorafenib, compounds in Table 3b, compounds in Table 3c, compounds in Table 8, compounds in Table 9, and compounds described in WO 2006/108059, WO/2005/020921, WO/2006/033943, and WO 2005/030140.

"cMET inhibitor" includes, for example, compounds in Table 3a, compounds in Table 3b, compounds in Table 3c, compounds described in WO06/108059, WO 2006/014325, and WO 2005/030140.

"EGFR inhibitor"includes, for example, one or more of the following: pelitinib, lapatinib (Tykerb®), gefitinib (Iressa®), erlotinib (Tarceva®), Zactima (ZD6474, vandetinib), AEE788 and HKI-272, EKB-569, CI-1033, N-(3,4-dichloro-2-fluorophenyl)-7-({[(3a*R*,5r,6a*S*)-2-methyloctahydrocyclopenta[c]pyrrol-5-yl]methyl}oxy)-6-(methyloxy)quinazolin-4-amine *N*-(4-bromo-3-chloro-2-fluorophenyl)-7-({[(3a*R*,5r,6a*S*)-2-methyloctahydrocyclo-penta[c]pyrrol-5-yl]methyl}oxy)-6-(methyloxy)quinazolin-4-amine, *N*-(3,4-dichloro-2-fluorophenyl)-7-({[(3a*R*,5s,6a*S*)-2-methyloctahydrocyclopenta[c]pyrrol-5-yl]methyl}oxy)-6-(methyloxy)quinazolin-4-amine, *N*-(4-bromo-3-chloro-2-fluorophenyl)-7-({[(3a*R*,5s,6a*S*)-2-methyloctahydrocyclo-penta[c]pyrrol-5-yl]methyl}oxy)-6-(methyloxy)quinazolin-4-amine, compounds in Table 4, compounds in Table 7, and compounds described in WO 2004/006846 and WO 2004/050681.

"ErbB2 inhibitor" includes, for example, lapatinib (GW572016), PKI-166, canertinib, CI-1033, HKI272, and EKB-569.

"Flt-3 inhibitor" includes, for example, CEP-701, PKC 412, MLN 518, sunitinib, sorafenib, compounds in Table 3a, compounds in Table 3b, compounds in Table 3c, compounds in Table 9, and compounds described in WO 2006/108059, WO/2006/033943, WO 2006/014325, and WO 2005/030140.

"Hormone therapy" and "hormonal therapy" include, for example, treatment with one or more of the following: steroids (e.g. dexamethasone), finasteride, tamoxifen, and an aromatase inhibitor.

"HSP90 inhibitor" includes, for example, 17-AAG, 17-DMAG, Geldanamycin, 5-(2,4-dihydroxy-5-isopropylphenyl)-N-ethyl-4-(4-(morpholinomethyl)phenyl)isoxazole-3-carboxamide [NVP-AUY922 (VER 52296)], 6-chloro-9-((4-methoxy-3,5-dimethylpyridin-2-yl)methyl)-9H-purin-2-amine (CNF2024, also named BIIB021), compounds disclosed in WO2004072051 (which is herein incorporated by reference), compounds disclosed in WO2005028434 (which is herein incorporated by reference), compounds disclosed in WO2007035620 (which is herein incorporated by reference) and compounds disclosed in WO2006091963 (which is herein incorporated by reference).

"IGF1R inhibitor" includes, for example, Tyrphostin AG 1024, compounds in Table 5a, compounds in Table 5b, and compounds described in WO06/074057.

"Kinase-dependent diseases or conditions" refer to pathologic conditions that depend on the activity of one or more lipid kinases. Kinases either directly or indirectly participate in the signal transduction pathways of a variety of cellular activities including proliferation, adhesion, migration, differentiation and invasion. Diseases associated with kinase activities include tumor growth, the pathologic neovascularization that supports solid tumor growth, and associated with other diseases where excessive local vascularization is involved such as ocular diseases (diabetic retinopathy, age-related macular degeneration, and the like) and inflammation (psoriasis, rheumatoid arthritis, and the like).

While not wishing to be bound to theory, phosphatases can also play a role in "kinase-dependent diseases or conditions" as cognates of kinases; that is, kinases phosphorylate and phosphatases dephosphorylate, for example lipid substrates. Therefore compounds of the invention, while modulating kinase activity as described herein, may also modulate, either directly or indirectly, phosphatase activity. This additional modulation, if present, may be synergistic (or not) to activity of compounds of the invention toward a related or otherwise interdependent kinase or kinase family. In any case, as stated previously, the compounds of the invention are useful for treating diseases characterized in part by abnormal levels of cell proliferation (i.e. tumor growth), programmed cell death (apoptosis), cell migration and invasion and angiogenesis associated with tumor growth.

"Metabolite" refers to the break-down or end product of a compound or its salt produced by metabolism or biotransformation in the animal or human body; for example, biotransformation to a more polar molecule such as by oxidation, reduction, or hydrolysis, or to a conjugate (see Goodman and Gilman, "The Pharmacological Basis of Therapeutics" 8.sup.th Ed., Pergamon Press, Gilman et al. (eds), 1990 for a discussion of biotransformation). As used herein, the metabolite of a compound of the invention or its salt may be the biologically active form of the compound in the body. In one example, a prodrug may be used such that the biologically active form, a metabolite, is released *in vivo*. In another example, a biologically active metabolite is discovered serendipitously, that is, no prodrug design *per se* was undertaken. An assay for activity of a metabolite of a compound of the present invention is known to one of skill in the art in light of the present disclosure.

"Patient" for the purposes of the present invention includes humans and other animals, particularly mammals, and other organisms. Thus the methods are applicable to both human therapy and veterinary applications. In a preferred embodiment the patient is a mammal, and in a most preferred embodiment the patient is human.

A "pharmaceutically acceptable salt" of a compound means a salt that is pharmaceutically acceptable and that possesses the desired pharmacological activity of the parent compound. It is understood that the pharmaceutically acceptable salts are non-toxic. Additional information on suitable pharmaceutically acceptable salts can be found in Remington's Pharmaceutical Sciences, 17th ed., Mack Publishing Company, Easton, PA, 1985, or S. M. Berge, et al., "Pharmaceutical Salts," J. Pharm. Sci., 1977;66:1-19.

Examples of pharmaceutically acceptable acid addition salts include those formed with inorganic acids such as hydrochloric acid, hydrobromic acid, sulfuric acid, nitric acid, phosphoric acid, and the like; as well as organic acids such as acetic acid, trifluoroacetic acid, propionic acid, hexanoic acid, cyclopentanepropionic acid, glycolic acid, pyruvic acid, lactic acid, oxalic acid, maleic acid, malonic acid, succinic acid, fumaric acid, tartaric acid, citric acid, benzoic acid, cinnamic acid, 3-(4-hydroxybenzoyl)benzoic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, p-toluenesulfonic acid, and salicylic acid and the like.

Examples of a pharmaceutically acceptable base addition salts include those formed when an acidic proton present in the parent compound is replaced by a metal ion, such as sodium, potassium, lithium, ammonium, calcium,

magnesium, iron, zinc, copper, manganese, aluminum salts and the like. Preferable salts are the ammonium, potassium, sodium, calcium, and magnesium salts. Salts derived from pharmaceutically acceptable organic non-toxic bases include, but are not limited to, salts of primary, secondary, and tertiary amines, substituted amines including naturally occurring substituted amines, cyclic amines and basic ion exchange resins. Examples of organic bases include isopropylamine, trimethylamine, diethylamine, triethylamine, tripropylamine, ethanolamine, 2-dimethylaminoethanol, 2-diethylaminoethanol, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, tromethamine, N-methylglucamine, polyamine resins, and the like. Exemplary organic bases are isopropylamine, diethylamine, ethanolamine, trimethylamine, dicyclohexylamine, choline, and caffeine.

"Platin(s)," and "platin-containing agent(s)" include, for example, cisplatin, carboplatin, and oxaliplatin.

"Prodrug" refers to compounds that are transformed (typically rapidly) *in vivo* to yield the parent compound of the above formulae, for example, by hydrolysis in blood. Common examples include, but are not limited to, ester and amide forms of a compound having an active form bearing a carboxylic acid moiety. Examples of pharmaceutically acceptable esters of the compounds of this invention include, but are not limited to, alkyl esters (for example with between about one and about six carbons) the alkyl group is a straight or branched chain. Acceptable esters also include cycloalkyl esters and arylalkyl esters such as, but not limited to benzyl. Examples of pharmaceutically acceptable amides of the compounds of this invention include, but are not limited to, primary amides, and secondary and tertiary alkyl amides (for example with between about one and about six carbons). Amides and esters of the compounds of the present invention may be prepared according to conventional methods. A thorough discussion of prodrugs is provided in T. Higuchi and V. Stella, "Pro-drugs as Novel Delivery Systems," Vol 14 of the A.C.S. Symposium Series, and in Bioreversible Carriers in Drug Design, ed. Edward B. Roche, American Pharmaceutical Association and Pergamon Press, 1987.

"Raf inhibitor" includes, for example, sorafenib, RAF 265 (CHIR 265), compounds in Table 6, and compounds described in WO 2005/112932. These references also describe in vitro assays that can be used to determine the inhibitory activity of RAF.

"Rapamycin analogue" includes for example, CCI-779, AP 23573, RAD 001 , TAFA 93, and compounds described in WO 2004/101583 and US 7,160,867.

"Receptor Tyrosine Kinase inhibitor" includes, for example, inhibitors of AKT, EGFR, ErbB2, IGF1R, KIT, Met, Raf, and VEGFR2. Examples of receptor tyrosine kinase inhibitors can be found in WO 2006/108059 (US Nat'l Stage Application Serial No. 11/910,720), WO 2006/074057 (US Nat'l Stage Application Serial No. 11/722,719), WO 2006/071819 (US Nat'l Stage Application Serial No. 11/722,291), WO 2006/014325 (US Nat'l Stage Application Serial No. 11/571,140), WO 2005/117909 (US Nat'l Stage Application Serial No. 11/568,173), WO 2005/030140 (US Nat'l Stage Application Serial No. 10/573,336), WO 2004/050681 US. Nat'l Stage Application Serial No. 10/533,555), WO 2005/112932 (US Nat'l Stage Application Serial No. 11/568,789), and WO 2004/006846 (US Nat'l Stage Application Serial No. 10/522,004). In particular, the applications cited in this paragraph are incorporated for the purpose of providing specific examples and generic embodiments (and the definitions associated with the terms used in the embodiments) of compounds that are useful in the practice of the invention. These references also describe in vitro assays useful in the practice of this invention.

"SRC and/or ABL kinase inhibitor" includes, for example, dasatinib, imatinib (Gleevec®), and compounds described in WO 2006/074057.

"Taxane(s)" includes, for example, one or more of the following: Paclitaxel (Taxol®) and Docetaxel (Taxotere®).

"Therapeutically effective amount" is an amount of a compound of the invention, that when administered to a patient, ameliorates a symptom of the disease. The amount of a compound of the invention which constitutes a "therapeutically effective amount" will vary depending on the compound, the disease state and its severity, the age of the patient to be treated, and the like. The therapeutically effective amount can be determined routinely by one of ordinary skill in the art having regard to their knowledge and to this disclosure.

"Topoisomerase inhibitor" includes, for example, one or more of the following: amsacrine, camptothecin, etoposide, etoposide phosphate, exatecan, irinotecan, lurtotecan, and teniposide, and topotecan.

"Treating" or "treatment" of a disease, disorder, or syndrome, as used herein, includes (i) preventing the disease, disorder, or syndrome from occurring in a human, i.e. causing the clinical symptoms of the disease, disorder, or syndrome not to develop in an animal that may be exposed to or predisposed to the disease, disorder, or syndrome but does not yet experience or display symptoms of the disease, disorder, or syndrome; (ii) inhibiting the disease, disorder, or syndrome, *i.e.,* arresting its development; and (iii) relieving the disease, disorder, or syndrome, i.e., causing regression of the disease, disorder, or syndrome. As is known in the art, adjustments for systemic versus localized delivery, age, body weight, general health, sex, diet, time of administration, drug interaction and the severity of the condition may be necessary, and will be ascertainable with routine experimentation by one of ordinary skill in the art. In another embodiment, "treating" or "treatment" of a disease, disorder, or syndrome, as used herein, includes (i) inhibiting the disease, disorder, or syndrome, *i.e.,* arresting its development; and (ii) relieving the disease, disorder, or syndrome, i.e., causing regression of the disease, disorder, or syndrome.

"VEGFR inhibitor" includes, for example, one or more of the following: VEGF Trap, ZD6474 (vandetanib, Zactima), sorafenib, Angiozyme, AZD2171 (cediranib), pazopanib, sorafenib, axitinib, SU5416 (semaxanib), PTK787 (vatalanib), AEE778, RAF 265, sunitinib (Sutent), *N*-(3,4-dichloro-2-fluorophenyl)-7-({[(3a*R*,5r,6a*S*)-2-methyloctahydrocyclopenta[c]pyrrol-5-yl]methyl}oxy)-6-(methyloxy)quinazolin-4-amine, *N*-(4-bromo-3-chloro-2-fluorophenyl)-7-({[(3a*R*,5r,6a*S*)-2-methyloctahydrocyclo-penta[c]pyrrol-5-yl]methyl}oxy)-6-(methyloxy)quinazolin-4-amine, *N*-(3,4-dichloro-2-fluorophenyl)-7-([(3a*R*,5s,6a*S*)-2-methyloctahydrocyclopenta[c]pyrrol-5-yl]methyl}oxy)-6-(methyloxy)quinazolin-4-amine, *N*-(4-bromo-3-chloro-2-fluorophenyl)-7-({[(3a*R*,5s,6a*S*)-2-methyloctahydrocyclo-penta[c]pyrrol-5-yl]methyl}oxy)-6-(methyloxy)quinazolin-4-amine, compounds in Table 7, and compounds described in WO 2004/050681 and WO 2004/006846.

**Embodiments of the Invention**

The following paragraphs present a number of embodiments of compounds that can be used to practice the invention. In each instance, the embodiment includes both the recited compounds as well as individual isomers and mixtures of isomers. In addition, in each instance, the embodiment includes the pharmaceutically acceptable salts, hydrates, and/or solvates of the recited compounds and any individual isomers or mixture of isomers thereof.

One embodiment (A) of the Invention is directed to a Compound of Formula I where $R^1$ is optionally substituted 5- or 6-membered heterocycloalkyl where the heterocycloalkyl comprises one or two heteroatoms selected from -O-, -S-, and -NH-; and all other groups are as defined in the Summary of the Invention for a Compound of Formula I. In another embodiment, the Invention is directed to a Compound of Formula I where $R^1$ is optionally substituted 5- or 6-membered heterocycloalkyl where the heterocycloalkyl comprises -0- and -NH-; and all other groups are as defined in the Summary of the Invention for a Compound of Formula I. In another embodiment, the Invention is directed to a Compound of Formula I where $R^1$ is optionally substituted 5- or 6-membered heterocycloalkyl where the heterocycloalkyl comprises one -O-; and all other groups are as defined in the Summary of the Invention for a Compound of Formula I.

In another embodiment (B1), the Invention is directed to a Compound of Formula I where $R^1$ is optionally substituted tetrahydrofuranyl or optionally substituted tetrahydropyranyl; and all other groups are as defined in the Summary of the Invention for a Compound of Formula I.

Another embodiment (B2) of the Invention is a Compound of Formula I, where $R^1$ is optionally substituted pyrrolidinyl or optionally substituted piperidinyl; and all other groups are as defined in the Summary of the Invention for a Compound of Formula I.

Another embodiment (C) of the Invention is a Compound of Formula I, where $R^6$ is a 5-membered heteroaryl optionally substituted with 1, 2, or 3 $R^9$ groups; and $R^9$ (when $R^9$ is present) and all other groups are as defined in the Summary of the Invention for a Compound of Formula I.

Another embodiment (D) of the Invention is a Compound of Formula I where $R^6$ is 6-membered heteroaryl optionally substituted with 1, 2, or 3 $R^9$ groups; and $R^9$ (when $R^9$ is present) and all other groups are as defined in the Summary of the Invention for a Compound of Formula I. In another embodiment of embodiment (D) the Compound of Formula I is that where $R^6$ is an unsubstituted 6-membered heteroaryl; and all other groups are as defined in the Summary of the Invention for a Compound of Formula I.

Another embodiment (E) of the Invention is a Compound of Formula I, where $R^6$ is a 5-membered heteroaryl optionally substituted with 1, 2, or 3 $R^9$ groups, where the heteroaryl comprises one or two heteroatoms selected from -O-, -S-, -N=, NH-, and -$NR^9$-; and $R^9$ (when $R^9$ is present) and all other groups are as defined in the Summary of the Invention for a Compound of Formula I. In another embodiment of embodiment (E) the Compound of Formula I is that where $R^6$ is an unsubstituted 5-membered heteroaryl; and all other groups are as defined in the Summary of the Invention for a Compound of Formula I.

Another embodiment (F) of the Invention is a Compound of Formula I, where $R^6$ is pyrazolyl, imidazolyl, thienyl, pyrrolyl, furanyl, or thiazolyl, each of which is optionally substituted with 1, 2, or 3 $R^9$ groups; and $R^9$ (when $R^9$ is present) and all other groups are as defined in the Summary of the Invention for a Compound of Formula I. In another embodiment, $R^6$ is pyrazol-1-yl, pyrazol-3-yl, pyrazol-4-yl, pyrazol-5-yl, imidazol-2-yl, imidazol-4-yl, imidazol-5-yl, thien-2-yl, thien-3-yl, pyrrol-1-yl, pyrrol-2-yl, pyrrol-3-yl, furan-2-yl, furan-3-yl, thiazol-2-yl, thiazol-4-yl, or thiazol-5-yl, each of which is optionally substituted with one or two $R^9$; and $R^9$ (when $R^9$ is present) and all other groups are as defined in the Summary of the Invention for a Compound of Formula I. In another embodiment of the Invention is a Compound of Formula I, where $R^6$ is pyrazolyl, imidazolyl, or thiazolyl, each of which is optionally substituted with 1, 2, or 3 $R^9$ groups; and $R^9$ (when $R^9$ is present) and all other groups are as defined in the Summary of the Invention for a Compound of Formula I. In another embodiment, $R^6$ is pyrazol-1-yl, pyrazol-3-yl, pyrazol-4-yl, pyrazol-5-yl, imidazol-2-yl, imidazol-4-yl, imidazol-5-yl, thiazol-2-yl, thiazol-4-yl, or thiazol-5-yl, each of which is optionally substituted with one or two $R^9$; and $R^9$ (when $R^9$ is present) and all other groups are as defined in the Summary of the Invention for a Compound of Formula I. In another embodiment of the Invention,, the Compound of Formula I is that where $R^6$ is unsubstituted pyrazolyl, unsubstituted imidazolyl, or unsubstituted thiazolyl; and all other groups are as defined in the Summary of the Invention for a

Compound of Formula I. In another embodiment, $R^6$ is unsubstituted pyrazol-1-yl, unsubstituted pyrazol-3-yl, unsubstituted pyrazol-4-yl, unsubstituted pyrazol-5-yl, unsubstituted imidazol-2-yl, unsubstituted imidazol-4-yl, unsubstituted imidazol-5-yl, unsubstituted thiazol-2-yl, unsubstituted thiazol-4-yl, or unsubstituted thiazol-5-yl; and all other groups are as defined in the Summary of the Invention for a Compound of Formula I. In another embodiment of the Invention, the Compound of Formula I is that where $R^6$ is imidazolyl optionally substituted with one $R^9$ where $R^9$ (when $R^9$ is present) is alkoxyalkyl; and all other groups are as defined in the Summary of the Invention for a Compound of Formula I.

Another embodiment (CC) of the Invention is a Compound of Formula I, where $R^6$ is unsubstituted pyrazolyl, unsubstituted imidazolyl, unsubstituted thienyl, unsubstituted pyrrolyl, unsubstituted furanyl, or unsubstituted thiazolyl; and all other groups are as defined in the Summary of the Invention for a Compound of Formula I. In another embodiment, $R^6$ is unsubstituted pyrazol-1-yl, unsubstituted pyrazol-3-yl, unsubstituted pyrazol-4-yl, unsubstituted pyrazol-5-yl, unsubstituted imidazol-2-yl, unsubstituted imidazol-4-yl, unsubstituted imidazol-5-yl, unsubstituted thien-2-yl, unsubstituted thien-3-yl, unsubstituted pyrrol-1-yl, unsubstituted pyrrol-2-yl, unsubstituted pyrrol-3-yl, unsubstituted furan-2-yl, unsubstituted furan-3-yl, unsubstituted thiazol-2-yl, unsubstituted thiazol-4-yl, or unsubstituted thiazol-5-yl. In another embodiment of the Invention is a Compound of Formula I, where $R^6$ is unsubstituted pyrazolyl, unsubstituted imidazolyl, or unsubstituted thiazolyl; and all other groups are as defined in the Summary of the Invention for a Compound of Formula I. In another embodiment, $R^6$ is unsubstituted pyrazol-1-yl, unsubstituted pyrazol-3-yl, unsubstituted pyrazol-4-yl, unsubstituted pyrazol-5-yl, unsubstituted imidazol-2-yl, unsubstituted imidazol-4-yl, unsubstituted imidazol-5-yl, unsubstituted thiazol-2-yl, unsubstituted thiazol-4-yl, or unsubstituted thiazol-5-yl; and all other groups are as defined in the Summary of the Invention for a Compound of Formula I. In another embodiment, $R^6$ is unsubstituted pyrazol-1-yl, unsubstituted pyrazol-3-yl, unsubstituted pyrazol-4-yl, or unsubstituted pyrazol-5-yl; and all other groups are as defined in the Summary of the Invention for a Compound of Formula I. In another embodiment, $R^6$ is unsubstituted imidazol-2-yl, unsubstituted imidazol-4-yl, unsubstituted imidazol-5-yl; and all other groups are as defined in the Summary of the Invention for a Compound of Formula I. In another embodiment, $R^6$ is unsubstituted thiazol-2-yl, unsubstituted thiazol-4-yl, or unsubstituted thiazol-5-yl; and all other groups are as defined in the Summary of the Invention for a Compound of Formula I.

Another embodiment (G) of the Invention is a Compound of Formula I, where $R^4$ is alkyl; and all other groups are as defined in the Summary of the Invention for a Compound of Formula I. In another embodiment, $R^4$ is methyl or ethyl; and all other groups are as defined in the Summary of the Invention for a Compound of Formula I. In another embodiment of the Invention $R^4$ is methyl; and all other groups are as defined in the Summary of the Invention for a Compound of Formula I.

Another embodiment (H) of the Invention is a Compound of Formula I, where $R^4$ is methyl; $R^1$ is optionally substituted 5- or 6-membered heterocycloalkyl where the heterocycloalkyl comprises one or two heteroatoms selected from -O-, -S-, and -NH-; and all other groups are as defined in the Summary of the Invention for a Compound of Formula I. Another embodiment of embodiment (H) is a Compound of Formula I, where $R^4$ is methyl; $R^1$ is optionally substituted 5- or 6-membered heterocycloalkyl where the heterocycloalkyl comprises one heteroatom and the heteroatom is -O-; and all other groups are as defined in the Summary of the Invention for a Compound of Formula I.

Another embodiment (J) of the Invention is a Compound of Formula I, where $R^2$ is hydrogen; $R^4$ is methyl; $R^6$ is a 5-membered heteroaryl optionally substituted with 1, 2, or 3 $R^9$ groups; and $R^9$ (when $R^9$ is present) and all other groups are as defined in the Summary of the Invention for a Compound of Formula I.

Another embodiment (K) of the Invention is a Compound of Formula I, where $R^2$ is hydrogen; $R^4$ is methyl; $R^6$ is a 5- membered heteroaryl optionally substituted with 1, 2, or 3 $R^9$ groups, where the heteroaryl comprises one or two heteroatoms selected from -O-, -S-, -N=, NH-, and -$NR^9$-; and $R^9$ (when $R^9$ is present) and all other groups are as defined in the Summary of the Invention for a Compound of Formula I.

Another embodiment (L) of the Invention is a Compound of Formula I, where $R^2$ is hydrogen; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ (when $R^9$ is present) all other groups are as defined in the Summary of the Invention for a Compound of Formula I. In another embodiment the Compound of Formula I is that where $R^2$ is hydrogen; $R^4$ is methyl; $R^6$ is pyrazolyl, imidazolyl, or thiazolyl, each of which is optionally substituted with 1, 2, or 3 $R^9$ groups; and $R^9$ (when $R^9$ is present) all other groups are as defined in the Summary of the Invention for a Compound of Formula I.

Another embodiment (M) of the Invention is a Compound of Formula I, where $R^2$ is hydrogen; $R^4$ is methyl; $R^1$ is optionally substituted 5- or 6-membered heterocycloalkyl where the heterocycloalkyl comprises one or two heteroatoms selected from -O-, -S-, and -NH-; $R^6$ is a 5-membered heteroaryl optionally substituted with 1, 2, or 3 $R^9$ groups; and $R^9$ (when $R^9$ is present) is as defined in the Summary of the Invention for a Compound of Formula I. In another embodiment of embodiment (M), the Compound is that where $R^2$ is hydrogen; $R^4$ is methyl; $R^1$ is optionally substituted tetrahydrofuranyl or tetrahydropyranyl; $R^6$ is a 5-membered heteroaryl optionally substituted with 1, 2, or 3 $R^9$ groups; and $R^9$ (when $R^9$ is present) is as defined in the Summary of the Invention for a Compound of Formula I.

Another embodiment (N) of the Invention is a Compound of Formula I, where $R^2$ is hydrogen; $R^4$ is methyl; $R^1$ is optionally substituted 5- or 6-membered heterocycloalkyl where the heterocycloalkyl comprises one or two heteroatoms

selected from -O-, -S-, and -NH-; $R^6$ is pyrazolyl, imidazolyl, thienyl, pyrrolyl, furanyl, or thiazolyl, each of which is optionally substituted with 1, 2, or 3 $R^9$ groups; and $R^9$ (when $R^9$ is present) is as defined in the Summary of the Invention for a Compound of Formula I. In another embodiment of embodiment (M) the Compound of Formula I is that where $R^2$ is hydrogen; $R^4$ is methyl; $R^1$ is optionally substituted 5- or 6-membered heterocycloalkyl where the heterocycloalkyl comprises one or two heteroatoms selected from -O-, -S-, and -NH-; $R^6$ is pyrazolyl, imidazolyl, or thiazolyl, each of which is optionally substituted with 1, 2, or 3 $R^9$ groups; and $R^9$ (when $R^9$ is present) is as defined in the Summary of the Invention for a Compound of Formula I.

Another embodiment (P) of the Invention is a Compound of Formula I, where $R^2$ is hydrogen; $R^4$ is methyl; $R^1$ is optionally substituted tetrahydrofuranyl or tetrahydropyranyl; $R^6$ is pyrazolyl, imidazolyl, thienyl, pyrrolyl, furanyl, or thiazolyl, each of which is optionally substituted with 1, 2, or 3 $R^9$ groups; and $R^9$ (when $R^9$ is present) is as defined in the Summary of the Invention for a Compound of Formula I. In another embodiment of embodiment (P) the Compound of Formula I is that where $R^2$ is hydrogen; $R^4$ is methyl; $R^1$ is optionally substituted tetrahydrofuranyl or tetrahydropyranyl; $R^6$ is pyrazolyl, imidazolyl, or thiazolyl, each of which is optionally substituted with 1, 2, or 3 $R^9$ groups; and $R^9$ (when $R^9$ is present) is as defined in the Summary of the Invention for a Compound of Formula I.

Another embodiment (Q) of the Invention is a Compound of Formula I where $R^1$ is optionally substituted 5-membered heterocycloalkyl; and all other groups are as defined in the Summary of the Invention for a Compound of Formula I.

Another embodiment (R) of the Invention is a Compound of Formula I where $R^1$ is optionally substituted 6-membered heterocycloalkyl; and all other groups are as defined in the Summary of the Invention for a Compound of Formula I.

Another embodiment (S) of the Invention is a Compound of Formula I where $R^2$ is alkyl; and all other groups are as defined in the Summary of the Invention for a Compound of Formula I. Another embodiment is where $R^2$ is methyl or ethyl; and all other groups are as defined in the Summary of the Invention for a Compound of Formula I. In another embodiment of the Invention $R^2$ is methyl; and all other groups are as defined in the Summary of the Invention for a Compound of Formula I.

Another embodiment (T) of the Invention is a Compound of Formula I where $R^2$ is hydrogen; and all other groups are as defined in the Summary of the Invention for a Compound of Formula I.

Another embodiment (U) of the Invention is a Compound of Formula I where $R^6$ is optionally substituted with one $R^9$; and $R^9$ (when $R^9$ is present) is alkyl, alkoxyalkyl, or alkoxycarbonyl; and all other groups are as defined in the Summary of the Invention for a Compound of Formula I. Another embodiment of the Invention is a Compound of Formula I where $R^6$ is optionally substituted with one $R^9$; and $R^9$ (when $R^9$ is present) is methyl, ethyl, isopropyl, methoxymethyl, ethoxymethyl, or *tert*-butoxycarbonyl; and all other groups are as defined in the Summary of the Invention for a Compound of Formula I.

Another embodiment (V) of the Invention is directed to a Compound of Formula I(a)

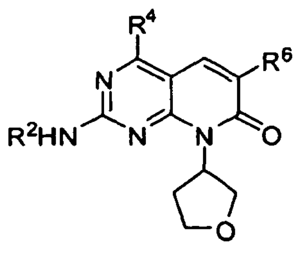

I(a)

where $R^2$, $R^4$, $R^6$, and all other groups are as defined in the Summary of the Invention for a Compound of Formula I.

In another embodiment of embodiment (V), the Compound of Formula I(a) is that where $R^2$ is hydrogen; and all other groups are as defined for a Compound of Formula I(a).

In another embodiment of embodiment (V), the Compound of Formula I(a) is that where $R^4$ is methyl; and all other groups are as defined for a Compound of Formula I(a).

In another embodiment of embodiment (V), the Compound of Formula I(a) is that where $R^2$ is hydrogen; $R^4$ is methyl; and all other groups are as defined for a Compound of Formula I(a).

In another embodiment of embodiment (V), the Compound of Formula I(a) is that where $R^2$ is alkyl; and all other groups are as defined for a Compound of Formula I(a). In another embodiment, the Compound of Formula I(a) is that where $R^2$ is methyl or ethyl; and all other groups are as defined for a Compound of Formula I(a). In another embodiment, the Compound of Formula I(a) is that where $R^2$ is methyl; and all other groups are as defined for a Compound of Formula I(a).

In another embodiment of embodiment (V), the Compound of Formula I(a) is that where $R^2$ is alkyl; $R^4$ is methyl; and all other groups are as defined for a Compound of Formula I(a). In another embodiment, the Compound of Formula

I(a) is that where $R^2$ is methyl or ethyl; $R^4$ is methyl; and all other groups are as defined for a Compound of Formula I(a).

In another embodiment of embodiment (V), the Compound of Formula I(a) is that where $R^6$ is pyrazolyl, imidazolyl, thienyl, pyrrolyl, furanyl, or thiazolyl, each of which is optionally substituted with 1, 2, or 3 $R^9$ groups; and $R^9$ and all other groups are as defined for a Compound of Formula I(a). In another embodiment, the Compound of Formula I(a) is that where $R^6$ is pyrazolyl, imidazolyl, or thiazolyl, each of which is optionally substituted with 1, 2, or 3 $R^9$ groups; and $R^9$ and all other groups are as defined for a Compound of Formula I(a). In another embodiment, the Compound of Formula I(a) is that where $R^6$ is unsubstituted pyrazolyl, unsubstituted imidazolyl, or unsubstituted thiazolyl; and all other groups are as defined in the Summary of the Invention for a Compound of Formula I. In another embodiment, the Compound of Formula I(a) is that where $R^6$ is unsubstituted pyrazolyl; and all other groups are as defined in the Summary of the Invention for a Compound of Formula I. In another embodiment, the Compound of Formula I(a) is that where $R^6$ is unsubstituted imidazolyl; and all other groups are as defined in the Summary of the Invention for a Compound of Formula I. In another embodiment, the Compound of Formula I(a) is that where $R^6$ is unsubstituted thiazolyl; and all other groups are as defined in the Summary of the Invention for a Compound of Formula I.

In another embodiment of embodiment (V), the Compound of Formula I(a) is that where $R^6$ is unsubstituted; and $R^2$ and $R^4$ are as defined in the Summary of the Invention for a Compound of Formula I.

In another embodiment of embodiment (V), the Compound of Formula I(a) is that where $R^2$ is hydrogen; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(a). In another embodiment of embodiment (V), the Compound of Formula I(a) is that where $R^2$ is hydrogen; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(a). In another embodiment, the Compound of Formula I(a) is that where $R^2$ is hydrogen; $R^4$ is methyl; $R^6$ is unsubstituted pyrazolyl, unsubstituted imidazolyl, or unsubstituted thiazolyl.

In another embodiment of embodiment (V), the Compound of Formula I(a) is that where $R^2$ is methyl or ethyl; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(a). In another embodiment of embodiment (V), the Compound of Formula I(a) is that where $R^2$ is methyl or ethyl; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(a).

Another embodiment (W) of the Invention is directed to a Compound of Formula I(b)

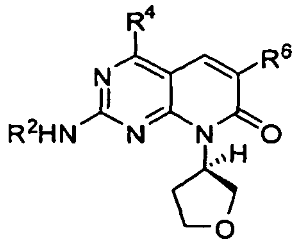

I(b)

where $R^2$, $R^4$, $R^6$, and all other groups are as defined in the Summary of the Invention for a Compound of Formula I.

In another embodiment of embodiment (W), the Compound of Formula I(b) is that where $R^2$ is hydrogen; and all other groups are as defined for a Compound of Formula I(b).

In another embodiment of embodiment (W), the Compound of Formula I(b) is that where $R^4$ is methyl; and all other groups are as defined for a Compound of Formula I(b).

In another embodiment of embodiment (W), the Compound of Formula I(b) is that where $R^2$ is hydrogen; $R^4$ is methyl; and all other groups are as defined for a Compound of Formula I(b).

In another embodiment of embodiment (W), the Compound of Formula I(b) is that where $R^2$ is alkyl; and all other groups are as defined for a Compound of Formula I(b). In another embodiment, the Compound of Formula I(b) is that where $R^2$ is methyl or ethyl; and all other groups are as defined for a Compound of Formula I(b). In another embodiment, the Compound of Formula I(b) is that where $R^2$ is methyl; and all other groups are as defined for a Compound of Formula I(b).

In another embodiment of embodiment (W), the Compound of Formula I(b) is that where $R^2$ is alkyl; $R^4$ is methyl; and all other groups are as defined for a Compound of Formula I(b). In another embodiment, the Compound of Formula I(b) is that where $R^2$ is methyl or ethyl; $R^4$ is methyl; and all other groups are as defined for a Compound of Formula I(b).

In another embodiment of embodiment (W), the Compound of Formula I(b) is that where $R^6$ is pyrazolyl, imidazolyl, thienyl, pyrrolyl, furanyl, or thiazolyl, each of which is optionally substituted with 1, 2, or 3 $R^9$ groups; and $R^9$

and all other groups are as defined for a Compound of Formula I(b). In another embodiment, the Compound of Formula I(b) is that where $R^6$ is pyrazolyl, imidazolyl, or thiazolyl, each of which is optionally substituted with 1, 2, or 3 $R^9$ groups; and $R^9$ and all other groups are as defined for a Compound of Formula I(b). In another embodiment, the Compound of Formula I(b) is that where $R^6$ is unsubstituted pyrazolyl, unsubstituted imidazolyl, or unsubstituted thiazolyl; and all other groups are as defined in the Summary of the Invention for a Compound of Formula I. In another embodiment, the Compound of Formula I(b) is that where $R^6$ is unsubstituted pyrazolyl; and all other groups are as defined in the Summary of the Invention for a Compound of Formula I. In another embodiment, the Compound of Formula I(b) is that where $R^6$ is unsubstituted imidazolyl; and all other groups are as defined in the Summary of the Invention for a Compound of Formula I. In another embodiment, the Compound of Formula I(b) is that where $R^6$ is unsubstituted thiazolyl; and all other groups are as defined in the Summary of the Invention for a Compound of Formula I.

In another embodiment of embodiment (W), the Compound of Formula I(b) is that where $R^6$ is unsubstituted; and $R^2$ and $R^4$ are as defined in the Summary of the Invention for a Compound of Formula I..

In another embodiment of embodiment (W), the Compound of Formula I(b) is that where $R^2$ is hydrogen; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(b). In another embodiment, the Compound of Formula I(b) is that where $R^2$ is hydrogen; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(b). In another embodiment, the Compound of Formula I(b) is that where $R^2$ is hydrogen; $R^4$ is methyl; $R^6$ is unsubstituted pyrazolyl, unsubstituted imidazolyl, or unsubstituted thiazolyl.

In another embodiment of embodiment (W), the Compound of Formula I(b) is that where $R^2$ is methyl or ethyl; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(b). In another embodiment, the Compound of Formula I(b) is that where $R^2$ is methyl or ethyl; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(b).

Another embodiment (X) of the Invention is directed to a Compound of Formula I(c)

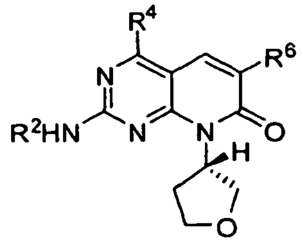

I(c)

where $R^2$, $R^4$, $R^6$, and all other groups are as defined in the Summary of the Invention for a Compound of Formula I.

In another embodiment of embodiment (X), the Compound of Formula I(c) is that where $R^2$ is hydrogen; and all other groups are as defined for a Compound of Formula I(c).

In another embodiment of embodiment (X), the Compound of Formula I(c) is that where $R^4$ is methyl; and all other groups are as defined for a Compound of Formula I(c).

In another embodiment of embodiment (X), the Compound of Formula I(c) is that where $R^2$ is hydrogen; $R^4$ is methyl; and all other groups are as defined for a Compound of Formula I(c).

In another embodiment of embodiment (X), the Compound of Formula I(c) is that where $R^2$ is alkyl; and all other groups are as defined for a Compound of Formula I(c). In another embodiment, the Compound of Formula I(c) is that where $R^2$ is methyl or ethyl; and all other groups are as defined for a Compound of Formula I(c). In another embodiment, the Compound of Formula I(c) is that where $R^2$ is methyl; and all other groups are as defined for a Compound of Formula I(c).

In another embodiment of embodiment (X), the Compound of Formula I(c) is that where $R^2$ is alkyl; $R^4$ is methyl; and all other groups are as defined for a Compound of Formula I(c). In another embodiment, the Compound of Formula I(c) is that where $R^2$ is methyl or ethyl; $R^4$ is methyl; and all other groups are as defined for a Compound of Formula I(c).

In another embodiment of embodiment (X), the Compound of Formula I(c) is that where $R^6$ is pyrazolyl, imidazolyl, thienyl, pyrrolyl, furanyl, or thiazolyl, each of which is optionally substituted with 1, 2, or 3 $R^9$ groups; and $R^9$ and all other groups are as defined for a Compound of Formula I(c). In another embodiment, the Compound of Formula I(c) is that where $R^6$ is pyrazolyl, imidazolyl, or thiazolyl, each of which is optionally substituted with 1, 2, or 3 $R^9$ groups; and $R^9$ and all other groups are as defined for a Compound of Formula I(c). In another embodiment, the Compound of Formula I(c) is that where $R^6$ is unsubstituted pyrazolyl, unsubstituted imidazolyl, or unsubstituted thiazolyl; and all other groups are as defined in the Summary of the Invention for a Compound of Formula I. In another embodiment, the

Compound of Formula I(c) is that where $R^6$ is unsubstituted pyrazolyl; and all other groups are as defined in the Summary of the Invention for a Compound of Formula I. In another embodiment, the Compound of Formula I(c) is that where $R^6$ is unsubstituted imidazolyl; and all other groups are as defined in the Summary of the Invention for a Compound of Formula I. In another embodiment, the Compound of Formula I(c) is that where $R^6$ is unsubstituted thiazolyl; and all other groups are as defined in the Summary of the Invention for a Compound of Formula I.

In another embodiment of embodiment (X), the Compound of Formula I(c) is that where $R^6$ is unsubstituted; and $R^2$ and $R^4$ are as defined in the Summary of the Invention for a Compound of Formula I..

In another embodiment of embodiment (X), the Compound of Formula I(c) is that where $R^2$ is hydrogen; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(c). In another embodiment, the Compound of Formula I(c) is that where $R^2$ is hydrogen; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(c). In another embodiment, the Compound of Formula I(c) is that where $R^2$ is hydrogen; $R^4$ is methyl; $R^6$ is unsubstituted pyrazolyl, unsubstituted imidazolyl, or unsubstituted thiazolyl.

In another embodiment of embodiment (X), the Compound of Formula I(c) is that where $R^2$ is methyl or ethyl; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(c). In another embodiment, the Compound of Formula I(c) is that where $R^2$ is methyl or ethyl; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(c).

Another embodiment (Y) of the Invention is directed to a Compound of Formula I(d)

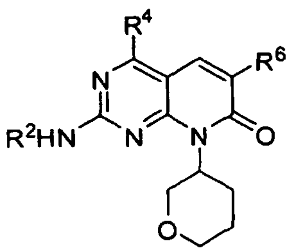

I(d)

where $R^2$, $R^4$, $R^6$, and all other groups are as defined in the Summary of the Invention for a Compound of Formula I.

In another embodiment of embodiment (Y), the Compound of Formula I(d) is that where $R^2$ is hydrogen; and all other groups are as defined for a Compound of Formula I(d).

In another embodiment of embodiment (Y), the Compound of Formula I(d) is that where $R^4$ is methyl; and all other groups are as defined for a Compound of Formula I(d).

In another embodiment of embodiment (Y), the Compound of Formula I(d) is that where $R^2$ is hydrogen; $R^4$ is methyl; and all other groups are as defined for a Compound of Formula I(d).

In another embodiment of embodiment (Y), the Compound of Formula I(d) is that where $R^2$ is alkyl; and all other groups are as defined for a Compound of Formula I(d). In another embodiment, the Compound of Formula I(d) is that where $R^2$ is methyl or ethyl; and all other groups are as defined for a Compound of Formula I(d). In another embodiment, the Compound of Formula I(d) is that where $R^2$ is methyl; and all other groups are as defined for a Compound of Formula I(d).

In another embodiment of embodiment (Y), the Compound of Formula I(d) is that where $R^2$ is alkyl; $R^4$ is methyl; and all other groups are as defined for a Compound of Formula I(d). In another embodiment, the Compound of Formula I(d) is that where $R^2$ is methyl or ethyl; $R^4$ is methyl; and all other groups are as defined for a Compound of Formula I(d).

In another embodiment of embodiment (Y), the Compound of Formula I(d) is that where $R^6$ is pyrazolyl, imidazolyl, thienyl, pyrrolyl, furanyl, or thiazolyl, each of which is optionally substituted with 1, 2, or 3 $R^9$ groups; and $R^9$ and all other groups are as defined for a Compound of Formula I(d). In another embodiment, the Compound of Formula I(d) is that where $R^6$ is pyrazolyl, imidazolyl, or thiazolyl, each of which is optionally substituted with 1, 2, or 3 $R^9$ groups; and $R^9$ and all other groups are as defined for a Compound of Formula I(d). In another embodiment, the Compound of Formula I(d) is that where $R^6$ is unsubstituted pyrazolyl, unsubstituted imidazolyl, or unsubstituted thiazolyl; and all other groups are as defined in the Summary of the Invention for a Compound of Formula I. In another embodiment, the Compound of Formula I(d) is that where $R^6$ is unsubstituted pyrazolyl; and all other groups are as defined in the Summary of the Invention for a Compound of Formula I. In another embodiment, the Compound of Formula I(d) is that where $R^6$ is unsubstituted imidazolyl; and all other groups are as defined in the Summary of the Invention for a Compound of Formula I. In another embodiment, the Compound of Formula I(d) is that where $R^6$ is unsubstituted thiazolyl; and all

other groups are as defined in the Summary of the Invention for a Compound of Formula I.

In another embodiment of embodiment (Y), the Compound of Formula Id) is that where $R^6$ is unsubstituted; and $R^2$ and $R^4$ are as defined in the Summary of the Invention for a Compound of Formula I.

In another embodiment of embodiment (Y), the Compound of Formula I(d) is that where $R^2$ is hydrogen; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(d). In another embodiment, the Compound of Formula I(d) is that where $R^2$ is hydrogen; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(d). In another embodiment, the Compound of Formula I(d) is that where $R^2$ is hydrogen; $R^4$ is methyl; $R^6$ is unsubstituted pyrazolyl, unsubstituted imidazolyl, or unsubstituted thiazolyl.

In another embodiment of embodiment (Y), the Compound of Formula I(d) is that where $R^2$ is methyl or ethyl; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(d). In another embodiment, the Compound of Formula I(d) is that where $R^2$ is methyl or ethyl; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(d).

Another embodiment (Z) of the Invention is directed to a Compound of Formula I(e)

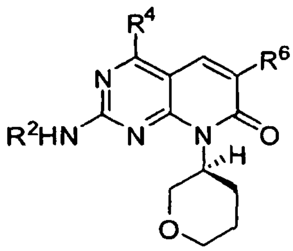

I(e)

where $R^2$, $R^4$, $R^6$, and all other groups are as defined in the Summary of the Invention for a Compound of Formula I.

In another embodiment of embodiment (Z), the Compound of Formula I(e) is that where $R^2$ is hydrogen; and all other groups are as defined for a Compound of Formula I(e).

In another embodiment of embodiment (Z), the Compound of Formula I(e) is that where $R^4$ is methyl; and all other groups are as defined for a Compound of Formula I(e).

In another embodiment of embodiment (Z), the Compound of Formula I(e) is that where $R^2$ is hydrogen; $R^4$ is methyl; and all other groups are as defined for a Compound of Formula I(e).

In another embodiment of embodiment (Z), the Compound of Formula I(e) is that where $R^2$ is alkyl; and all other groups are as defined for a Compound of Formula I(e). In another embodiment, the Compound of Formula I(e) is that where $R^2$ is methyl or ethyl; and all other groups are as defined for a Compound of Formula I(e). In another embodiment, the Compound of Formula I(e) is that where $R^2$ is methyl; and all other groups are as defined for a Compound of Formula I(e).

In another embodiment of embodiment (Z), the Compound of Formula I(e) is that where $R^2$ is alkyl; $R^4$ is methyl; and all other groups are as defined for a Compound of Formula I(e). In another embodiment, the Compound of Formula I(e) is that where $R^2$ is methyl or ethyl; $R^4$ is methyl; and all other groups are as defined for a Compound of Formula I(e).

In another embodiment of embodiment (Z), the Compound of Formula I(e) is that where $R^6$ is pyrazolyl, imidazolyl, thienyl, pyrrolyl, furanyl, or thiazolyl, each of which is optionally substituted with 1, 2, or 3 $R^9$ groups; and $R^9$ and all other groups are as defined for a Compound of Formula I(e). In another embodiment, the Compound of Formula I(e) is that where $R^6$ is pyrazolyl, imidazolyl, or thiazolyl, each of which is optionally substituted with 1, 2, or 3 $R^9$ groups; and $R^9$ and all other groups are as defined for a Compound of Formula I(e). In another embodiment, the Compound of Formula I(e) is that where $R^6$ is unsubstituted pyrazolyl, unsubstituted imidazolyl, or unsubstituted thiazolyl; and all other groups are as defined in the Summary of the Invention for a Compound of Formula I. In another embodiment, the Compound of Formula I(e) is that where $R^6$ is unsubstituted pyrazolyl; and all other groups are as defined in the Summary of the Invention for a Compound of Formula I. In another embodiment, the Compound of Formula I(e) is that where $R^6$ is unsubstituted imidazolyl; and all other groups are as defined in the Summary of the Invention for a Compound of Formula I. In another embodiment, the Compound of Formula I(e) is that where $R^6$ is unsubstituted thiazolyl; and all other groups are as defined in the Summary of the Invention for a Compound of Formula I.

In another embodiment of embodiment (Z), the Compound of Formula I(e) is that where $R^6$ is unsubstituted;

and $R^2$ and $R^4$ are as defined in the Summary of the Invention for a Compound of Formula I..

In another embodiment of embodiment (Z), the Compound of Formula I(e) is that where $R^2$ is hydrogen; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(e). In another embodiment, the Compound of Formula I(e) is that where $R^2$ is hydrogen; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(e). In another embodiment, the Compound of Formula I(e) is that where $R^2$ is hydrogen; $R^4$ is methyl; $R^6$ is unsubstituted pyrazolyl, unsubstituted imidazolyl, or unsubstituted thiazolyl.

In another embodiment of embodiment (Z), the Compound of Formula I(e) is that where $R^2$ is methyl or ethyl; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(e). In another embodiment, the Compound of Formula I(e) is that where $R^2$ is methyl or ethyl; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(e).

Another embodiment (AA) of the Invention is directed to a Compound of Formula I(f)

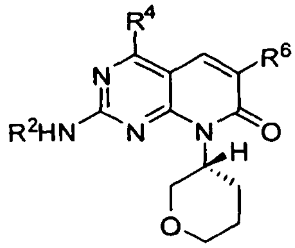

I(f)

where $R^2$, $R^4$, $R^6$, and all other groups are as defined in the Summary of the Invention for a Compound of Formula I.

In another embodiment of embodiment (AA), the Compound of Formula I(f) is that where $R^2$ is hydrogen; and all other groups are as defined for a Compound of Formula I(f).

In another embodiment of embodiment (AA), the Compound of Formula I(f) is that where $R^4$ is methyl; and all other groups are as defined for a Compound of Formula I(f).

In another embodiment of embodiment (AA), the Compound of Formula I(f) is that where $R^2$ is hydrogen; $R^4$ is methyl; and all other groups are as defined for a Compound of Formula I(f).

In another embodiment of embodiment (AA), the Compound of Formula I(f) is that where $R^2$ is alkyl; and all other groups are as defined for a Compound of Formula I(f). In another embodiment, the Compound of Formula I(f) is that where $R^2$ is methyl or ethyl; and all other groups are as defined for a Compound of Formula I(f). In another embodiment, the Compound of Formula I(f) is that where $R^2$ is methyl; and all other groups are as defined for a Compound of Formula I(f).

In another embodiment of embodiment (AA), the Compound of Formula I(f) is that where $R^2$ is alkyl; $R^4$ is methyl; and all other groups are as defined for a Compound of Formula I(f). In another embodiment, the Compound of Formula I(f) is that where $R^2$ is methyl or ethyl; $R^4$ is methyl; and all other groups are as defined for a Compound of Formula I(f).

In another embodiment of embodiment (AA), the Compound of Formula I(f) is that where $R^6$ is pyrazolyl, imidazolyl, thienyl, pyrrolyl, furanyl, or thiazolyl, each of which is optionally substituted with 1, 2, or 3 $R^9$ groups; and $R^9$ and all other groups are as defined for a Compound of Formula I(f). In another embodiment, the Compound of Formula I(f) is that where $R^6$ is pyrazolyl, imidazolyl, or thiazolyl, each of which is optionally substituted with 1, 2, or 3 $R^9$ groups; and $R^9$ and all other groups are as defined for a Compound of Formula I(f). In another embodiment, the Compound of Formula I(f) is that where $R^6$ is unsubstituted pyrazolyl, unsubstituted imidazolyl, or unsubstituted thiazolyl; and all other groups are as defined in the Summary of the Invention for a Compound of Formula I. In another embodiment, the Compound of Formula I(f) is that where $R^6$ is unsubstituted pyrazolyl; and all other groups are as defined in the Summary of the Invention for a Compound of Formula I. In another embodiment, the Compound of Formula I(f) is that where $R^6$ is unsubstituted imidazolyl; and all other groups are as defined in the Summary of the Invention for a Compound of Formula I. In another embodiment, the Compound of Formula I(f) is that where $R^6$ is unsubstituted thiazolyl; and all other groups are as defined in the Summary of the Invention for a Compound of Formula I.

In another embodiment of embodiment (AA), the Compound of Formula I(f) is that where $R^6$ is unsubstituted; and $R^2$ and $R^4$ are as defined in the Summary of the Invention for a Compound of Formula I..

In another embodiment of embodiment (AA), the Compound of Formula I(f) is that where $R^2$ is hydrogen; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(f). In another embodiment, the Compound of

Formula I(f) is that where $R^2$ is hydrogen; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(f). In another embodiment, the Compound of Formula I(f) is that where $R^2$ is hydrogen; $R^4$ is methyl; $R^6$ is unsubstituted pyrazolyl, unsubstituted imidazolyl, or unsubstituted thiazolyl.

In another embodiment of embodiment (AA), the Compound of Formula I(f) is that where $R^2$ is methyl or ethyl; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(f). In another embodiment, the Compound of Formula I(f) is that where $R^2$ is methyl or ethyl; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(f).

Another embodiment (BB) of the Invention is directed to a Compound of Formula I(g)

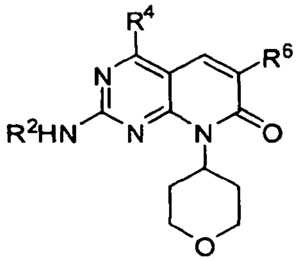

I(g)

where $R^2$, $R^4$, $R^6$, and all other groups are as defined in the Summary of the Invention for a Compound of Formula I.

In another embodiment of embodiment (BB), the Compound of Formula I(g) is that where $R^2$ is hydrogen; and all other groups are as defined for a Compound of Formula I(g).

In another embodiment of embodiment (BB), the Compound of Formula I(g) is that where $R^4$ is methyl; and all other groups are as defined for a Compound of Formula I(g).

In another embodiment of embodiment (BB), the Compound of Formula I(g) is that where $R^2$ is hydrogen; $R^4$ is methyl; and all other groups are as defined for a Compound of Formula I(g).

In another embodiment of embodiment (BB), the Compound of Formula I(g) is that where $R^2$ is alkyl; and all other groups are as defined for a Compound of Formula I(g). In another embodiment, the Compound of Formula I(g) is that where $R^2$ is methyl or ethyl; and all other groups are as defined for a Compound of Formula I(g). In another embodiment, the Compound of Formula I(g) is that where $R^2$ is methyl; and all other groups are as defined for a Compound of Formula I(g).

In another embodiment of embodiment (BB), the Compound of Formula I(g) is that where $R^2$ is alkyl; $R^4$ is methyl; and all other groups are as defined for a Compound of Formula I(g). In another embodiment, the Compound of Formula I(g) is that where $R^2$ is methyl or ethyl; $R^4$ is methyl; and all other groups are as defined for a Compound of Formula I(g).

In another embodiment of embodiment (BB), the Compound of Formula I(g) is that where $R^6$ is pyrazolyl, imidazolyl, thienyl, pyrrolyl, furanyl, or thiazolyl, each of which is optionally substituted with 1, 2, or 3 $R^9$ groups; and $R^9$ and all other groups are as defined for a Compound of Formula I(g). In another embodiment, the Compound of Formula I(g) is that where $R^6$ is pyrazolyl, imidazolyl, or thiazolyl, each of which is optionally substituted with 1, 2, or 3 $R^9$ groups; and $R^9$ and all other groups are as defined for a Compound of Formula I(g). In another embodiment, the Compound of Formula I(g) is that where $R^6$ is unsubstituted pyrazolyl, unsubstituted imidazolyl, or unsubstituted thiazolyl; and all other groups are as defined in the Summary of the Invention for a Compound of Formula I. In another embodiment, the Compound of Formula I(g) is that where $R^6$ is unsubstituted pyrazolyl; and all other groups are as defined in the Summary of the Invention for a Compound of Formula I. In another embodiment, the Compound of Formula I(g) is that where $R^6$ is unsubstituted imidazolyl; and all other groups are as defined in the Summary of the Invention for a Compound of Formula I. In another embodiment, the Compound of Formula I(g) is that where $R^6$ is unsubstituted thiazolyl; and all other groups are as defined in the Summary of the Invention for a Compound of Formula I.

In another embodiment of embodiment (BB), the Compound of Formula I(g) is that where $R^6$ is unsubstituted; and $R^2$ and $R^4$ are as defined in the Summary of the Invention for a Compound of Formula I..

In another embodiment of embodiment (BB), the Compound of Formula I(g) is that where $R^2$ is hydrogen; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(g). In another embodiment, the Compound of Formula I(g) is that where $R^2$ is hydrogen; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(g). In another embodiment, the

Compound of Formula I(g) is that where $R^2$ is hydrogen; $R^4$ is methyl; $R^6$ is unsubstituted pyrazolyl, unsubstituted imidazolyl, or unsubstituted thiazolyl.

In another embodiment of embodiment (BB), the Compound of Formula I(g) is that where $R^2$ is methyl or ethyl; $R^4$ is methyl; $R^6$ is pyrazolyl, imidazolyl, thienyl, pyrrolyl, furanyl, or thiazolyl, each of which is optionally substituted with 1, 2, or 3 $R^9$ groups; and $R^9$ is as defined for a Compound of Formula I(g). In another embodiment, the Compound of Formula I(g) is that where $R^2$ is methyl or ethyl; $R^4$ is methyl; $R^6$ is pyrazolyl, imidazolyl, or thiazolyl, each of which is optionally substituted with 1, 2, or 3 $R^9$ groups; and $R^9$ is as defined for a Compound of Formula I(g).

Another embodiment of the Invention provides a pharmaceutical composition which comprises a compound of Formula I, I(a), I(b), I(c), I(d), I(e), I(f), or I(g) optionally as a pharmaceutically acceptable salt, solvate, and/or hydrate thereof and a pharmaceutically acceptable carrier, excipient, or diluent.

Also described herein is a method of inhibiting proliferative activity in a cell, the method comprising administering to a cell or a plurality of cells an effective amount of a compound of Formula I, I(a), I(b), I(c), I(d), I(e), I(f), or I(g)or a Compound selected from Table 1 optionally as a pharmaceutically acceptable salt, solvate, and/or hydrate thereof, and additionally optionally as pharmaceutical composition thereof.

Described as embodiment (CC) is a method of treating disease, disorder, or syndrome where the disease is associated with uncontrolled, abnormal, and/or unwanted cellular activities effected directly or indirectly by PI3Kα which method comprises administering to a human in need thereof a therapeutically effective amount of a compound of Formula I (as defined in the Summary of the Invention) or a pharmaceutically acceptable salt, solvate, or a pharmaceutical composition thereof. Specifically, the Compound is of Formula I (as defined in the Summary of the Invention).

Described as embodiment (DD) is a method of treating a disease, disorder, or syndrome which method comprises administering to a patient a therapeutically effective amount of a compound of Formula I, I(a), I(b), I(c), I(d), I(e), I(f), or I(g) or a pharmaceutically acceptable salt, solvate, and/or hydrate thereof, or administering a pharmaceutical composition comprising a therapeutically effective amount of a compound of Formula I, I(a), I(b), I(c), I(d), I(e), I(f), or I (g) and a pharmaceutically acceptable carrier, excipient, or diluent.

In another embodiment of (EE), the disease is cancer.

In another embodiment of embodiment (EE) or an embodiment of the sixth aspect of the invention, the cancer is breast cancer, colon cancer, rectal cancer, endometrial cancer, gastric carcinoma (including gastrointestinal carcinoid tumors and gastrointestinal stromal tumors), glioblastoma, hepatocellular carcinoma, small cell lung cancer, non-small cell lung cancer, melanoma, ovarian cancer, cervical cancer, pancreatic cancer, prostate carcinoma, acute myelogenous leukemia (AML), chronic myelogenous leukemia (CML), non-Hodgkin's lymphoma, or thyroid carcinoma. In another embodiment, the cancer is ovarian cancer, cervical cancer, breast cancer, colon cancer, rectal cancer, or glioblastoma. In another embodiment of embodiment (T), the Compound of Formula I is selected from Table 1.

Another embodiment (GG) of the invention is a compound of Formula I, I(a), I(b), I(c), I(d), I(e), I(f), or I(g) or a Compound selected from Table 1, optionally as a pharmaceutically acceptable salt, solvate, and/or hydrate thereof, or a pharmaceutical composition comprising a therapeutically effective amount of a compound of Formula I, I(a), I(b), I (c), I(d), I(e), I(f), or I(g) and a pharmaceutically acceptable carrier, excipient, or diluent in combination with one or more chemotherapeutic agents for use in the treatment of cancer.

In another embodiment of embodiment (GG), one or more of the chemotherapeutic agents are independently selected from rapamycin, a rapamycin analogue, an alkylating agent, a taxane, a platin, an EGFR inhibitor, and an ErbB2 inhibitor. In another embodiment, one or two of the chemotherapeutic agents is independently selected from rapamycin, temozolomide, paclitaxel, docetaxel, carboplatin, cisplatin, oxaliplatin, gefitinib (Iressa®), erlotinib (Tarceva®), Zactima (ZD6474), HKI-272, pelitinib, canertinib, and lapatinib. In another embodiment, one or two of the chemotherapeutic agents are independently selected from rapamycin, temozolomide, paclitaxel, docetaxel, carboplatin, trastuzumab, erlotinib, and lapatinib. In another embodiment, one or two of the chemotherapeutic agents are independently selected from rapamycin, paclitaxel, carboplatin, and erlotinib.

In another embodiment of embodiment (GG), one or more of the chemotherapeutic agents are independently selected from a platin and a taxane. In another embodiment, one or two of the chemotherapeutic agents is independently selected from carboplatin, cisplatin, oxaliplatin, and paclitaxel.

In another embodiment of embodiment (GG), one or more of the chemotherapeutic agents is an EGFR inhibitor. In another embodiment, one or two of the chemotherapeutic agents is an EGFR inhibitor. In another embodiment, one of the chemotherapeutic agent is an EGFR inhibitor selected from lapatinib (Tykerb®), gefitinib (Iressa®), erlotinib (Tarceva®), Zactima (ZD6474), AEE778, HKI-272, EKB-569, and CI1033.

In another embodiment of embodiment (GG), one or more of the chemotherapeutic agents is an ErbB2 inhibitor. In another embodiment, one of the chemotherapeutic agents is an ErbB2 inhibitor selected from lapatinib, EXB-569, HKI272, CI1033, and PKI-166.

In another embodiment of embodiment (GG), one or more of the chemotherapeutic agents is selected from rapamycin, CCI-779, AP23573, RAD 001, TAFA 93, PI103, and SF1126. In another embodiment, one of the chemotherapeutic agents is rapamycin.

Another embodiment of the invention is a compound of Formula I, I(a), I(b), I(c), I(d), I(e), I(f), or I(g) or a Compound selected from Table 1 optionally additionally as a pharmaceutically acceptable salt, solvate, and/or hydrate thereof, or a pharmaceutical composition comprising a therapeutically effective amount of a compound of Formula I, I (a), I(b), I(c), I(d), I(e), I(f), or I(g) or a Compound selected from Table 1 and a pharmaceutically acceptable carrier, excipient, or diluent in combination with radiation for use in the treatment of cancer.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more antibodies for use in the treatment of cancer.

Another embodiment of the invention is a compound of Formula I, I(a), I(b), I(c), I(d), I(e), I(f), or I(g) or a Compound selected from Table 1 optionally as a pharmaceutically acceptable salt, solvate and/or hydrate thereof, or a pharmaceutical composition comprising a therapeutically effective amount of a compound of Formula I, I(a), I(b), I(c), I (d), I(e), I(f), or I(g) or a Compound selected from Table 1 and a pharmaceutically acceptable carrier, excipient, or diluent in combination with surgery for use in the treatment of cancer.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more hormone therapies for use in the treatment of cancer.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent; in combination with one or more of treatments selected from bone marrow or peripheral blood stem cell transplantation, radiation, one or more antibodies, and one or more chemotherapeutic agents for use in the treatment of acute myelogenous leukemia. In another embodiment, the antibody is selected from Gemtuzumab ozogamicin (Mylotarg), $^{\alpha}$IGF-1R A12 MoAb, $^{\alpha}$IGF-1R 19D12 MoAb, $^{\alpha}$IGF-1R h7C10 MoAb, $^{\alpha}$IGF-1R CP-751871 MoAb and trastuzumab. In another embodiment, one or two of the chemotherapeutic agents is selected from Imatinib (i.e. Gleevec®), PKC412, CEP-701, daunorubicin, doxorubicin, cytarabine (ara-C), an anthracycline drug such as daunorubicin or idarubicin (Daunomycin, Idamycin), 6-thioguanine, and a granulocyte colony-stimulating factor such as Neupogen or Leukine.

Another embodiment of the invention is a therapeutically effective amount of a compound of Formula I, I(a), I (b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from bone marrow or peripheral blood stem cell transplantation, radiation one or more chemotherapeutic agents, immunotherapy, and one or more antibodies for use in the treatment of chronic myelogenous leukemia (CML). In another embodiment, one or two of the chemotherapeutic agents is selected from Imatinib (i.e. Gleevec®), PKC412, hydroxyurea (Hydrea), cytosine, cytosine arabinoside, dasatinib, AMN107, VX680 (MK0457), and cytarabine (ara-C); in another embodiment, one or more of the chemotherapeutic agents is selected from Imatinib (i.e. Gleevec®) and dasatinib. In another embodiment, the immunotherapy is selected from interferon therapy such as interferon-a.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from surgery (including cryosurgery), radiation, one or more chemotherapeutic agents, one or more antibodies, and one or more hormone therapies for use in the treatment of prostate cancer. In another embodiment, one or two of the antibodies is $^{\alpha}$IGF-1R A12 MoAb, $^{\alpha}$IGF-1R 19D12 MoAb, $^{\alpha}$IGF-1R h7C10 MoAb, $^{\alpha}$IGF-1R CP-751871 MoAb. In another embodiment, one or two of the chemotherapeutic agents is selected from rapamycin, mitoxantrone, prednisone, docetaxel (Taxotere), doxorubicin, etoposide, vinblastine, paclitaxel, and carboplatin. In another embodiment, one or two of the hormone therapies is androgen deprivation therapy or androgen suppression therapy. In another embodiment, one or two of the treatments is a taxanes.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent

in combination with one or more of treatments selected from surgery, radiation, one or more immunotherapies, one or more hormone therapies, and one or more chemotherapeutic agents for use in the treatment of melanoma. In another embodiment, one or two of the chemotherapeutic agents is independently selected from an alkylating agent, a taxane, a platin, and a Raf inhibitor. In another embodiment, one or two chemotherapeutic agent is selected from sorafenib, Paclitaxel (Taxol®), Docetaxel (Taxotere®), dacarbazine, rapamycin, imatinib mesylate (Gleevec®), sorafenib, cisplatin, carboplatin, dacarbazine (DTIC), carmustine (BCNU), vinblastine, temozolomide (Temodar), Melphalan, and imiquimod (Aldara). In another embodiment, one or two of the immunotherapies is selected from ipilimumab, interferon-alpha and/or interleukin-2. In another embodiment, one or two of the hormone therapies is tamoxifen.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from surgery, radiation, one or more antibodies, and one or more chemotherapeutic agents for use in the treatment of colon or rectal cancer. In another embodiment, the surgery is selected from local excision, electrofulguration, segmental colon resection, polypectomy, local transanal resection, low anterior resection, abdominoperineal resection, and pelvic exenteration. In another embodiment, one or two of the chemotherapeutic agents is selected from a platinum-containing compound (including cisplatin, oxaliplatin, and carboplatin), 5-fluorouracil (5-FU), leucovorin, capecitabine (Xeloda), irinotecan (Camptosar), FOLFOX (Folinic acid, 5-FU, Oxaliplatin), and leucovorin. In another embodiment, one or two of the antibodies is selected from cetuximab (Erbitux) and bevacizumab (Avastin).

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier excipient, or diluent in combination with one or more of treatments selected from surgery, radiation, and one or more chemotherapeutic agents for use in the treatment of pancreatic cancer. In another embodiment, one or two of the chemotherapeutic agents is selected from platinum-containing compound (including cisplatin, oxaliplatin, and carboplatin), 5-fluorouracil (5-FU), gemcitabine, a taxane (including paclitaxel and docetaxel), topotecan, irinotecan, capecitabine, streptozocin, erlotinib (Tarceva), cetuximab, leucovorin, and capecitabine (Xeloda).

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from surgery, radiation, one or more chemotherapeutic agents, one or more hormone therapies, and one or more antibodies for use in the treatment of breast cancer. In another embodiment, one or two of the chemotherapeutic agents is selected from lapatinib (Tykerb®), Paclitaxel (Taxol®), docetaxel, capecitabine, Cyclophosphamide (Cytoxan), CMF (cyclophosphamide, fluoruracil, and methotrexate), methotrexate, fluorouracil, doxorubicin, epirubicin, gemcitabine, carboplatin (Paraplatin), cisplatin (Platinol), vinorelbine (Navelbine), capecitabine (Xeloda), pegylated liposomal doxorubicin (Doxil), albumin-bound paclitaxel (Abraxane), AC (adriamycin and Cyclophosphamide), adriamyclin, and pamidronate or zoledronic acid (to treat bone weakness). In another embodiment, one or two of the hormone therapyies is selected from tamoxifen, Toremifene (Fareston), Fulvestrant (Faslodex), Megestrol acetate (Megace), ovarian ablation, Raloxifene, a luteinizing hormone-releasing hormone (LHRH) analogs (including goserelin and leuprolide), Megestrol acetate (Megace), and one or two aromatase inhibitor(s); in another embodiment, one or two of the aromatase inhibitor(s) is selected from letrozole (Femara), anastrozole (Arimidex), and exemestane (Aromasin). In another embodiment, one or more of the antibodyies is selected from $^{\alpha}$IGF-1R A12 MoAb, $^{\alpha}$IGF-1R 19D 12 MoAb, $^{\alpha}$IGF-1R h7C10 MoAb, $^{\alpha}$IGF-1R CP-751871 MoAb, bevacizumab (Avastin), and trastuzumab.

Another embodiment of the invention is a compound of Formula I, I(a), I(b), I(c), I(d), I(e), I(f), or I(g) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from one or more chemotherapeutic agents and one or more antibodies for use in the treatment of breast cancer. In another embodiment, one or two of the chemotherapeutic agents is selected from rapamycin, lapatinib, and erlotinib. In another embodiment, one of the antibodies is trastuzumab.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c),

I(d), I(e), I(f), or I(g) or a Compound selected from Table 1 and a pharmaceutically acceptable carrier, excipient, or diluent in combination with one or more of treatments selected from surgery, radiation, one or more antibodies, and one or more chemotherapeutic agents for use in the treatment of non-small cell lung cancer. In another embodiment, one or two of the chemotherapeutic agents is independently selected from cisplatin, oxaliplatin, carboplatin, Zactima (ZD6474), Paclitaxel, Docetaxel (Taxotere®), Gemcitabine (Gemzar®), Vinorelbine, Irinotecan, Etoposide, Vinblastine, Erlotinib (Tarceva®), gefitinib (Iressa), and Pemetrexed. In another embodiment, one of the antibodies is Bevacizumab. In another embodiment, the chemotherapeutic agent is selected from cisplatin, oxaliplatin, carboplatin, Paclitaxel, Docetaxel (Taxotere®), and erlotinib (Tarceva®).

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from surgery, radiation, and one or more chemotherapeutic agents for use in the treatment of small cell lung cancer. In another embodiment, one or two of the chemotherapy agents is selected from a platin (such as cisplatin, oxaliplatin, and carboplatin), gefitinib, vinorelbine, docetaxel, paclitaxel, etoposide, fosfamide, ifosfamide, cyclophosphamide, cyclophosphamide/doxorubicin/vincristine (CAV), doxorubicin, vincristine, gemcitabine, paclitaxel, vinorelbine, topotecan, irinotecan, methotrexate, and docetaxel.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from surgery, radiation, radioactive iodine therapy, one or more hormone therapies, and one or more chemotherapeutic agents for use in the treatment of papillary or anaplastic thyroid cancer. In another embodiment, one or two of the chemotherapeutic agents is selected from thyroid hormone pills, Doxorubucin and a platin. In another embodiment, one of the hormone therapies is radioiodine ablation.

Another embodiment of the invention is a compound of Formula I, I(a), I(b), I(c), I(d), I(e), I(f), or I(g)or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from surgery, radiation, one or more hormone therapies, and one or more chemotherapeutic agents for use in the treatment of endometrial cancer. In another embodiment, one or two of the hormone therapies is selected from megestrol acetate, Tamoxifen, and a progestin including medroxyprogesterone acetate (Provera) and megestrol acetate (Megace). In another embodiment, one or two of the chemotherapeutic agents is selected from a platinum-containing compound (including cisplatin, oxaliplatin, and carboplatin; in another example, cisplatin), a taxane (including paclitaxel), doxorubicin (Adriamycin), cyclophosphamide, fluorouracil (5-FU), methotrexate, and vinblastine.

Another embodiment of the invention is a compound of Formula I, I(a), I(b), I(c), I(d), I(e), I(f), or I(g) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from surgery, radiation, one or more antibodies, and one or more chemotherapeutic agents for use in the treatment of ovarian cancer. In another embodiment, one of the antibodies is selected from bevacizumab. In another embodiment, one or two of the chemotherapeutic agents is selected from a platinum-containing compound (including cisplatin, oxaliplatin and carboplatin), a taxane (including paclitaxel and docetaxel), topotecan, an anthracyclines (including doxorubicin and liposomal doxorubicin), gemcitabine, cyclophosphamide, vinorelbine (Navelbine), hexamethylmelamine, ifosfamide, etoposide, bleomycin, vinblastine, ifosfamide, vincristine, and cyclophosphamide. In another embodiment, one or more of the treatments is selected from one or two chemotherapeutic agents where the chemotherapeutic agents are independently selected from a platin and a taxane.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from surgery, radiation, one or more chemotherapeutic agents, one or more anti-seizure agents, and one or more agents to reduce swelling for use in the treatment of glioblastoma. In another embodiment, the radiation treatment is selected from external beam radiation, interstitial radiotherapy, and stereotactic radiosurgery. In another embodiment, one or two of the chemotherapeutic agents is selected from carmustine (BCNU), Erlotinib (Tarceva), bevacizumab, gefitinib (Iressa), rapamycin, temozolomide, cisplatin, BCNU, lomustine,

procarbazine, and vincristine. In another embodiment, one or two of the anti-seizure agents is selected from diphenylhydantoin (Dilantin). In another embodiment, one or two of the agents to reduce swelling is selected from dexamethasone (Decadron). In another embodiment, one of the treatments is one or two chemotherapeutic agents; in another embodiment, one or two of the chemotherapeutic agents are independently selected from erlotinib and temozolomide.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from surgery, radiation, and one or more chemotherapeutic agents for use in the treatment of cervical cancer. In another embodiment, the surgery is selected from cryosurgery, laser surgery, loop electrosurgical excision, conization, simple hysterectomy, and radical hysterectomy and pelvic lymph node dissection. In another embodiment, the radiation is selected from called external beam radiation therapy and brachytherapy. In another embodiment, one or two of the chemotherapeutic agents is selected from a platinum compound (such as cisplatin, carboplatin, and oxaliplatin), paclitaxel, topotecan, ifosfamide, gemcitabine, vinorelbine, and fluorouracil.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from surgery, radiation, immunotherapy, and one or more chemotherapeutic agents for use in the treatment of gastrointestinal carcinoid tumor. In another embodiment, the surgery is selected from excision and electrofulguration. In another embodiment, one or two of the chemotherapeutic agents is selected from cyproheptadine, SOM230, octreotide and lanreotide. In another embodiment, the immunotherapy is an interferon.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from surgery, radiation, and one or more chemotherapeutic agents for use in the treatment of gastrointestinal stromal tumor. In another embodiment, one or two of the chemotherapeutic agents are independently selected from imatinib mesylate (Gleevec), sunitinib (Sutent), and nilotinib (AMN107).

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from surgery, radiofrequency ablation, ethanol ablation, cryosurgery, hepatic artery embolization, chemoembolization, radiation, and one or more chemotherapeutic agents for use in the treatment of hepatocellular carcinoma. In another embodiment, the surgery is selected from resection and transplantation. In another embodiment, one or two of the chemotherapeutic agents are independently selected from sorafenib, 5-fluorouracil and cisplatin.

Another embodiment of the invention is a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with one or more of treatments selected from radiation, one or more chemotherapeutic agents, interferon therapy, one or more antibodies, and bone marrow or peripheral blood stem cell transplantation for use in the treatment of non-Hodgkin's lymphoma. In another embodiment, one or two of the chemotherapeutic agents are independently selected from CHOP (cyclophosphamide, doxorubicin, vincristine and prednisone), chlorambucil, fludarabine, and etoposide. In another embodiment, the antibody is selected from rituximab, ibritumomab tiuxetan, tositumomab, and alemtuzumab; in another embodiment, the antibody is rituximab.

Another embodiment of the invention is a compound of Formula I, I(a), I(b), I(c), I(d), I(e), I(f), or I(g)) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c), I(d), I(e), I(f), or I(g) or a Compound selected from Table 1 and a pharmaceutically acceptable carrier, excipient, or diluent in combination with radiation and surgery for use in the treatment of cancer.

Another embodiment of the invention is a a compound of Formula I, I(a), I(b), I(c), I(d), I(e), I(f), or I(g) or a Compound selected from Table 1 optionally as a pharmaceutically acceptable salt, solvate, and/or hydrate thereof, or a pharmaceutical composition comprising a therapeutically effective amount of a compound of Formula I, I(a), I(b), I(c),

I(d), I(e), I(f), or I(g) or a Compound selected from Table 1 and a pharmaceutically acceptable carrier, excipient, or diluent in combination with radiation and one or two chemotherapeutic agents for use in the treatment of cancer.

**Representative Compounds**

Representative compounds of Formula I are depicted below. The examples are merely illustrative. Compounds of the invention are named according to systematic application of the nomenclature rules agreed upon by the International Union of Pure and Applied Chemistry (IUPAC), International Union of Biochemistry and Molecular Biology (IUBMB), and the Chemical Abstracts Service (CAS). Names were generated using ACD/Labs naming software 8.00 release, product version 8.08. Each of the recited compounds in Table 1 can be prepared as an individual isomer (where applicable) as well as a pharmaceutically acceptable salt, solvate, and/or hydrate, thereof.

**Table 1**

| Example | Structure | Name |
|---|---|---|
| 1 | 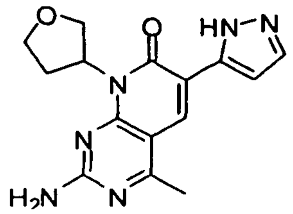 | 2-amino-4-methyl-6-(1*H*-pyrazol-5-yl)-8-(tetrahydr ofuran-3-yl) pyrido[2,3-*d*]pyrimidin-7(8*H*)-one |
| 2 | 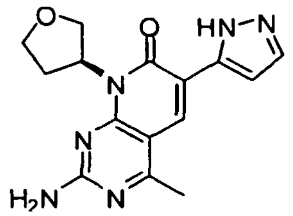 | 2-amino-4-methyl-6-(1*H*-pyrazol-5-yl)-8-[(3*S*')-tetr ahydrofuran-3-yl]pyrido[2,3-*d*]pyrimidin-7(8*H*)-on e |
| 3 | 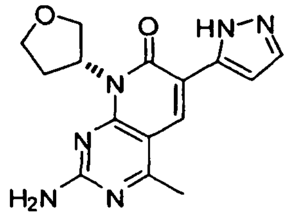 | 2-amino-4-methyl-6-(1*H*-pyrazol-5-yl)-8-[(3*R*)-tetr ahydrofuran-3-yl]pyrido[2,3-*d*]pyrimidin-7(8*H*)-on e |
| 4 | 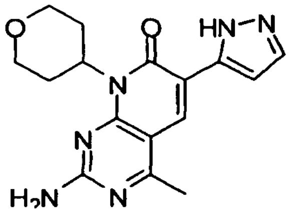 | 2-amino-4-methyl-6-(1*H*-pyrazol-5-yl)-8-(tetrahydr o-2*H*-pyran-4-yl)pyrido[2,3-*d*]pyrimidin-7(8*H*)-one |
| 5 | 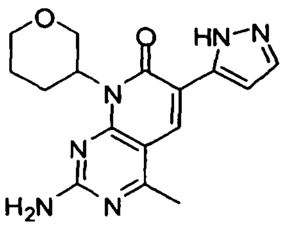 | 2-amino-4-methyl-6-(1*H*-pyrazol-5-yl)-8-(tetrahydr o-2H-pyran-3-yl)pyrido[2,3-*d*]pyrimidin-7(8*H*)-one |

(continued)

| Example | Structure | Name |
|---|---|---|
| 6 | 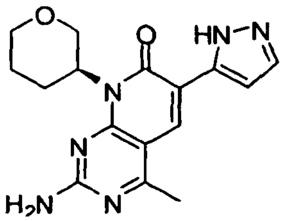 | 2-amino-4-methyl-6-(1*H*-pyrazol-5-yl)-8-[(3*S*)-tetr ahydro-2*H*-pyran-3-yl]pyrido[2,3-*d*]pyrimidin-7(8 *H*)-one |
| 7 | 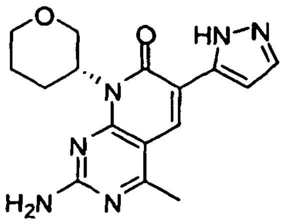 | 2-amino-4-methyl-6-(1*H*-pyrazol-5-yl)-8-[(3*R*)-tetr ahydro-2*H*-pyran-3-yl]pyrido[2,3-*d*]pyrimidin-7(8 *H*)-one |
| 8 | 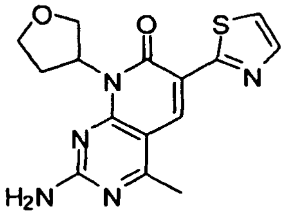 | 2-amino-4-methyl-8-(tetrahydrofuran-3-yl)-6-(1,3-t hiazol-2-yl) pyrido[2,3-*d*]pyrimidin-7(8*H*)-one |
| 9 | 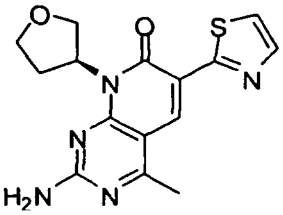 | 2-amino-4-methyl-8-[(3*S*)-tetrahydrofuran-3-yl]-6-(1,3-thiazol-2-yl)pyrido[2,3-*d*]pyrimidin-7(8*H*)-one |
| 10 | 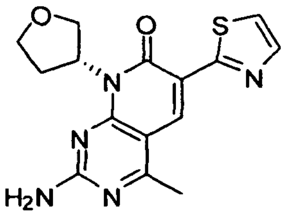 | 2-amino-4-methyl-8-[(3*R*)-tetrahydrofuran-3-yl]-6-(1,3-thiazol-2-yl)pyrido[2,3-*d*]pyrimidin-7(8*H*)-one |
| 11 | 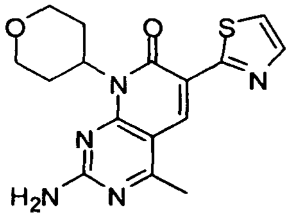 | 2-amino-4-methyl-8-(tetrahydro-2*H*-pyran-4-yl)-6-(1,3-thiazol-2-yl)pyrido[2,3-*d*]pyrimidin-7(8*H*)-one |
| 12 | 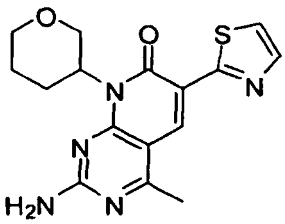 | 2-amino-4-methyl-8-(tetrahydro-2*H*-pyran-3-yl)-6-(1,3-thiazol-2-yl)pyrido[2,3-*d*]pyrimidin-7(8*H*)-one |

(continued)

| Example | Structure | Name |
|---|---|---|
| 13 | 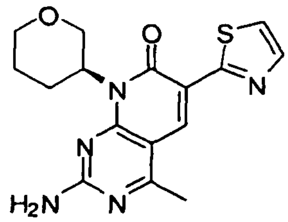 | 2-amino-4-methyl-8-[(3*S*)-tetrahydro-2*H*-pyran-3-y l]-6-(1,3-thiazol-2-yl)pyrido[2,3-*d*]pyrimidin-7(8*H*) -one |
| 14 | 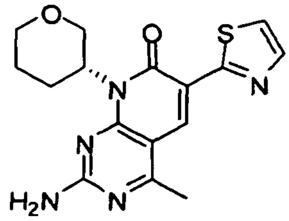 | 2-amino-4-methyl-8-[(3*R)*-tetrahydro-2*H*-pyran-3-yl]-6-(1,3-thiazol-2-yl)pyrido[2,3-d]pyrimidin-7(8 *H*)-one |
| 15 | 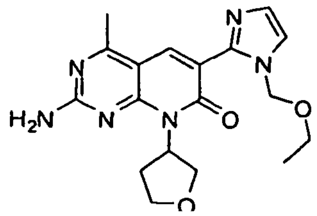 | 2-amino-6-{1-[(ethyloxy)methyl]-1*H*-imidazol-2-yl}-4-methyl-8-(tetrahydrofuran-3-yl)pyrido[2,3-*d*]pyrimidin-7(8*H*)-one |
| 16 | 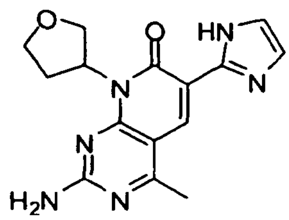 | 2-amino-6-(1*H*-imidazol-2-yl)-4-methyl-8-(tetrahy drofuran-3-yl) pyrido[2,3-*d*]-pyrimidin-7(8*H*)-one |
| 17 | 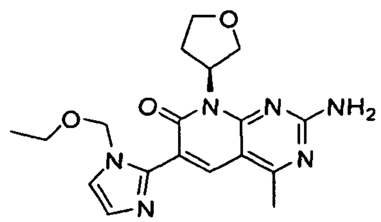 | 2-amino-6-{1-[(ethyloxy)methyl]-1*H*-imidazol-2-yl}-4-methyl-8-[(3*S*)-tetrahydrofuran-3-yl]pyrido[2,3-*d*]pyrimidin-7(8*H*)-one |
| 18 | 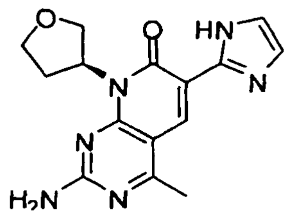 | 2-amino-6-(1*H*-imidazol-2-yl)-4-methyl-8-[(3*S*)-tet rahydrofuran-3-yl]pyrido[2,3-*d*]pyrimidin-7(8*H*)-o ne |
| 19 | 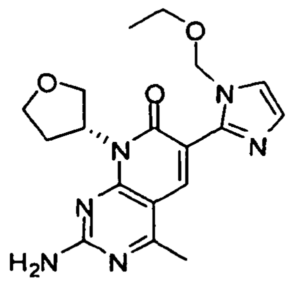 | 2-amino-6-{1-[(ethyloxy)methyl]-1*H*-imidazol-2-yl}-4-methyl-8-[(3*R*)-tetrahydrofuran-3-yl]pyrido[2,3-*d*]pyrimidin-7(8*H*)-one |

(continued)

| Example | Structure | Name |
|---|---|---|
| 20 | 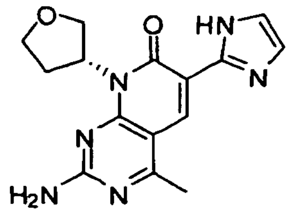 | 2-amino-6-(1*H*-imidazol-2-yl)-4-methyl-8-[(3*R*)-tet rahydrofuran-3-yl]pyrido[2,3-*d*]pyrimidin-7(8*H*)-o ne |
| 21 | 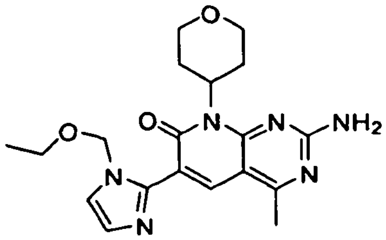 | 2-amino-6-{1-[(ethyloxy)methyl]-1*H*-imidazol-2-yl}-4-methyl-8-(tetrahydro-2*H*-pyran-4-yl)pyrido[2,3-*d*]pyrimidin-7(8*H*)-one |
| 22 | 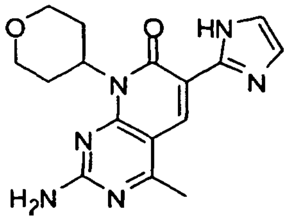 | 2-amino-6-(1*H*-imidazol-2-yl)-4-methyl-8-(tetrahy dro-2*H*-pyran-4-yl)pyrido[2,3-*d*]pyrimidin-7(8*H*)-o ne |
| 23 | 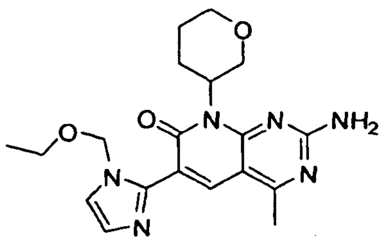 | 2-Amino-6-{1-[(ethyloxy)methyl]-1*H*-imidazol-2-yl}-4-methyl-8-(tetrahydro-2*H*-pyran-3-yl)pyrido[2,3-*d*]pyrimidin-7(8*H*)-one |
| 24 | 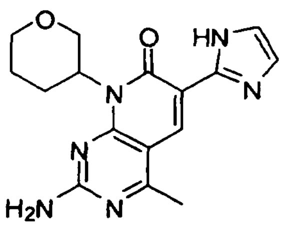 | 2-amino-6-(1*H*-imidazol-2-yl)-4-methyl-8-(tetrahy dro-2*H*-pyran-3-yl)pyrido[2,3-*d*]pyrimidin-7(8*H*)-o ne |
| 25 | 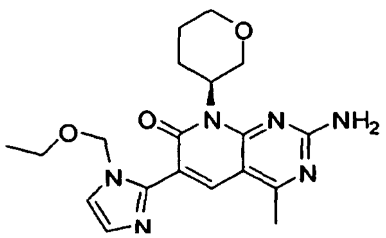 | 2-amino-6-{1-[(ethyloxy)methyl]-1*H*-imidazol-2-yl}-4-methyl-8-[(3*S*)-tetrahydro-2*H*-pyran-3-yl]pyrido[2,3-*d*]pyrimidin-7 (8*H*)-one |
| 26 | 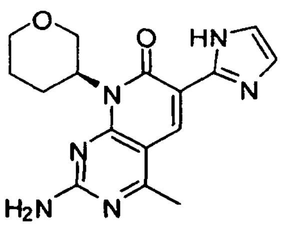 | 2-amino-6-(1*H*-imidazol-2-yl)-4-methyl-8-[(3*S*)-tet rahydro-2*H*-pyran-3-yl]pyrido[2,3-*d*]pyrimidin-7(8 *H*)-one |

(continued)

| Example | Structure | Name |
|---|---|---|
| 27 | 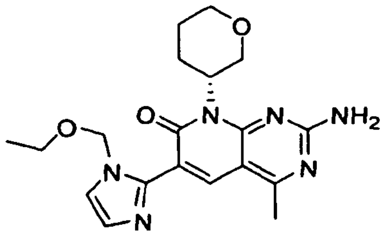 | 2-amino-6-{1-[(ethyloxy)methyl]-1*H*-imidazol-2-yl}-4-methyl-8-[(3*R*)-tetrahydro-2*H*-pyran-3-yl]pyrido[2,3-*d*]pyrimidin-7(8*H*)-one |
| 28 | 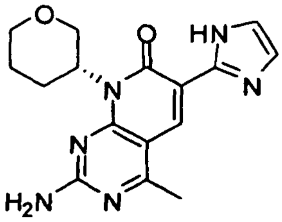 | 2-amino-6-(1*H*-imidazol-2-yl)-4-methyl-8-[(3*R*)-tet rahydro-2*H*-pyran-3-yl]pyrido[2,3-*d*]pyrimidin-7(8 *H*)-one |
| 29 | 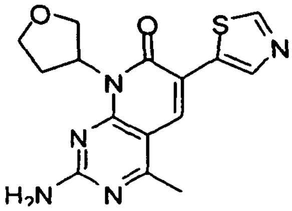 | 2-amino-4-methyl-8-(tetrahydrofuran-3-yl)-6-(1,3-t hiazol-5-yl) pyrido[2,3-*d*]pyrimidin-7(8*H*)-one |
| 30 | 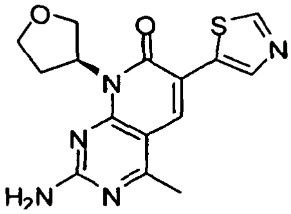 | 2-amino-4-methyl-8-[(3*S*)-tetrahydrofuran-3-yl]-6-(1,3-thiazol-5-yl)pyrido[2,3-*d*]pyrimidin-7(8*H*)-one |
| 31 | 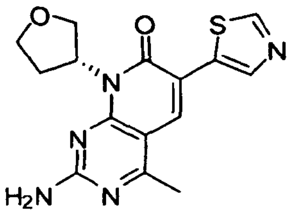 | 2-amino-4-methyl-8-[(3*R*)-tetrahydrofuran-3-yl]-6-(1,3-thiazol-5-yl)pyrido[2,3-*d*]pyrimidin-7(8*H*)-one |
| 32 | 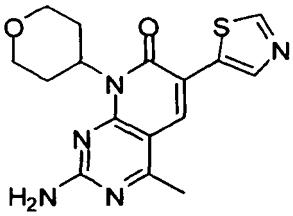 | 2-amino-4-methyl-8-(tetrahydro-2*H*-pyran-4-yl)-6-(1,3-thiazol-5-yl)pyrido[2,3-*d*]pyrimidin-7(8*H*)-one |
| 33 | 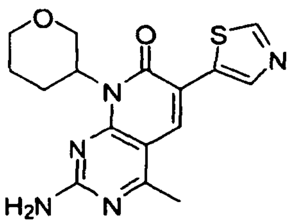 | 2-amino-4-methyl-8-(tetrahydro-2*H*-pyran-3-yl)-6-(1,3-thiazol-5-yl)pyrido[2,3-*d*]pyrimidin-7(8*H*)-one |

(continued)

| Example | Structure | Name |
|---|---|---|
| 34 | 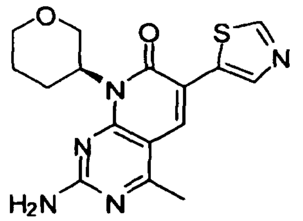 | 2-amino-4-methyl-8-[(3*S*)-tetrahydro-2*H*-pyran-3-y 1]-6-(1,3-thiazol-5-yl)pyrido[2,3-*d*]pyrimidin-7(8*H*) -one |
| 35 | 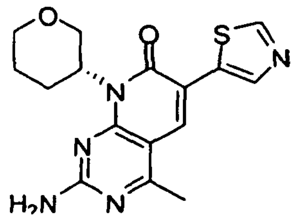 | 2-amino-4-methyl-8-[(3*R*)-tetrahydro-2*H*-pyran-3-yl]-6-(1,3-thiazol-5-yl)pyrido[2,3-*d*]pyrimidin-7(8 *H*)-one |

**General Administration**

In one aspect, the invention provides pharmaceutical compositions comprising an inhibitor of PI3K according to the invention and a pharmaceutically acceptable carrier, excipient, or diluent. In certain other specific embodiments, administration is by the oral route. Administration of the compounds of the invention, or their pharmaceutically acceptable salts, in pure form or in an appropriate pharmaceutical composition, can be carried out via any of the accepted modes of administration or agents for serving similar utilities. Thus, administration can be, for example, orally, nasally, parenterally (intravenous, intramuscular, or subcutaneous), topically, transdermally, intravaginally, intravesically, intracistemally, or rectally, in the form of solid, semi-solid, lyophilized powder, or liquid dosage forms, such as for example, tablets, suppositories, pills, soft elastic and hard gelatin capsules, powders, solutions, suspensions, or aerosols, or the like, specifically in unit dosage forms suitable for simple administration of precise dosages.

The compositions will include a conventional pharmaceutical carrier or excipient and a compound of the invention as the/an active agent, and, in addition, may include carriers and adjuvants, etc.

Adjuvants include preserving, wetting, suspending, sweetening, flavoring, perfuming, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

If desired, a pharmaceutical composition of the invention may also contain minor amounts of auxiliary substances such as wetting or emulsifying agents, pH buffering agents, antioxidants, and the like, such as, for example, citric acid, sorbitan monolaurate, triethanolamine oleate, butylalted hydroxytoluene, etc.

The choice of formulation depends on various factors such as the mode of drug administration (e.g., for oral administration, formulations in the form of tablets, pills or capsules) and the bioavailability of the drug substance. Recently, pharmaceutical formulations have been developed especially for drugs that show poor bioavailability based upon the principle that bioavailability can be increased by increasing the surface area i.e., decreasing particle size. For example, U.S. Pat. No. 4,107,288 describes a pharmaceutical formulation having particles in the size range from 10 to 1,000 nm in which the active material is supported on a crosslinked matrix of macromolecules. U.S. Pat. No. 5,145,684 describes the production of a pharmaceutical formulation in which the drug substance is pulverized to nanoparticles (average particle size of 400 nm) in the presence of a surface modifier and then dispersed in a liquid medium to give a pharmaceutical formulation that exhibits remarkably high bioavailability.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

One specific route of administration is oral, using a convenient daily dosage regimen that can be adjusted according to the degree of severity of the disease-state to be treated.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, cellulose derivatives, starch, alignates, gelatin, polyvinylpyrrolidone, sucrose, and gum acacia, (c) humectants, as for example, glycerol, (d) disintegrating agents, as for example, agar-agar, calcium carbonate, potato or tapioca starch, alginic acid, croscarmellose sodium, complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, magnesium stearate and the like (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid dosage forms as described above can be prepared with coatings and shells, such as enteric coatings and others well known in the art. They may contain pacifying agents, and can also be of such composition that they release the active compound or compounds in a certain part of the intestinal tract in a delayed manner. Examples of embedded compositions that can be used are polymeric substances and waxes. The active compounds can also be in microencapsulated form, if appropriate, with one or more of the above-mentioned excipients.

Liquid dosage forms for oral administration include pharmaceutically acceptable emulsions, solutions, suspensions, syrups, and elixirs. Such dosage forms are prepared, for example, by dissolving, dispersing, etc., a compound(s) of the invention, or a pharmaceutically acceptable salt thereof, and optional pharmaceutical adjuvants in a carrier, such as, for example, water, saline, aqueous dextrose, glycerol, ethanol and the like; solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide; oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan; or mixtures of these substances, and the like, to thereby form a solution or suspension.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administrations are, for example, suppositories that can be prepared by mixing the compounds of the present invention with for example suitable non-irritating excipients or carriers such as cocoa butter, polyethyleneglycol or a suppository wax, which are solid at ordinary temperatures but liquid at body temperature and therefore, melt while in a suitable body cavity and release the active component therein.

Dosage forms for topical administration of a compound of this invention include ointments, powders, sprays, and inhalants. The active component is admixed under sterile conditions with a physiologically acceptable carrier and any preservatives, buffers, or propellants as may be required. Ophthalmic formulations, eye ointments, powders, and solutions are also contemplated as being within the scope of this invention.

Compressed gases may be used to disperse a compound of this invention in aerosol form. Inert gases suitable for this purpose are nitrogen, carbon dioxide, etc.

Generally, depending on the intended mode of administration, the pharmaceutically acceptable compositions will contain about 1% to about 99% by weight of a compound(s) of the invention, or a pharmaceutically acceptable salt thereof, and 99% to 1% by weight of a suitable pharmaceutical excipient. In one example, the composition will be between about 5% and about 75% by weight of a compound(s) of the invention, or a pharmaceutically acceptable salt thereof, with the rest being suitable pharmaceutical excipients.

Actual methods of preparing such dosage forms are known, or will be apparent, to those skilled in this art; for example, see Remington's Pharmaceutical Sciences, 18th Ed., (Mack Publishing Company, Easton, Pa., 1990). The composition to be administered will, in any event, contain a therapeutically effective amount of a compound of the invention, or a pharmaceutically acceptable salt thereof, for treatment of a disease-state in accordance with the teachings of this invention.

The compounds of the invention, or their pharmaceutically acceptable salts or solvates, are administered in a therapeutically effective amount which will vary depending upon a variety of factors including the activity of the specific compound employed, the metabolic stability and length of action of the compound, the age, body weight, general health, sex, diet, mode and time of administration, rate of excretion, drug combination, the severity of the particular disease-states, and the host undergoing therapy. The compounds of the present invention can be administered to a patient at dosage levels in the range of about 0.1 to about 1,000 mg per day. For a normal human adult having a body weight of about 70 kilograms, a dosage in the range of about 0.01 to about 100 mg per kilogram of body weight per day is an example. The specific dosage used, however, can vary. For example, the dosage can depend on a number of factors including the requirements of the patient, the severity of the condition being treated, and the pharmacological activity of the compound being used. The determination of optimum dosages for a particular patient is well known to one of ordinary skill in the art.

If formulated as a fixed dose, such combination products employ the compounds of this invention within the

dosage range described above and the other pharmaceutically active agent(s) within its approved dosage range. Compounds of the instant invention may alternatively be used sequentially with known pharmaceutically acceptable agent (s) when a combination formulation is inappropriate.

## UTILITY

Compounds of this invention have been tested using the assay described in Biological Example I to determine PI3K-inhibitory activity. Compounds of Formula I are useful for treating diseases; particularly cancer in which PI3K activity contributes to the pathology and/or symptomatology of the disease. For example, cancer in which PI3K activity contributes to its pathology and/or symptomatology include breast cancer, colon cancer, rectal cancer, endometrial cancer, gastric carcinoma, glioblastoma, hepatocellular carcinoma, small cell lung cancer, non-small cell lung cancer, melanoma, ovarian cancer, cervical cancer, pancreatic cancer, prostate carcinoma, acute myelogenous leukemia (AML), chronic myelogenous leukemia (CML), or thyroid carcinoma

Suitable *in vitro* assays for measuring PI3K activity and the inhibition thereof by compounds are known in the art. For further details of an *in vitro* assay for measuring PI3K activity see Biological Examples, Example 1 *infra.* Following the examples disclosed herein, as well as that disclosed in the art, a person of ordinary skill in the art can determine the inhibitory activity of a compound of this invention.

Assays for measurement of *in vitro* efficacy in treatment of cancer are known in the art. For further details of cell-based assays see Biological Examples, Example 2, 3, and 4 *infra.* Following the examples disclosed herein, as well as that disclosed in the art, a person of ordinary skill in the art can determine the cell-based activity of a compound of the invention.

Suitable *in vivo* models for cancer are known to those of ordinary skill in the art. For further details of *in vivo* models for prostate adenocarcinoma, glioblastoma, lung carcinoma, and breast adenocarcinoma, see Biological Examples 5, 6, 7, 8, 9, and 10, *infra* and can be used to determine the cell-based activity of the Compounds of the Invention.

### General Synthesis

Compounds of this invention can be made by the synthetic procedures described below. The starting materials and reagents used in preparing these compounds are either available from commercial suppliers such as Aldrich Chemical Co. (Milwaukee, Wis.), or Bachem (Torrance, Calif.), or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), March's Advanced Organic Chemistry, (John Wiley and Sons, 4th Edition) and Larock's Comprehensive Organic Transformations (VCH Publishers Inc., 1989). These schemes are merely illustrative of some methods by which the compounds of this invention can be synthesized, and various modifications to these schemes can be made and will be suggested to one skilled in the art having referred to this disclosure. The starting materials and the intermediates of the reaction may be isolated and purified if desired using conventional techniques, including but not limited to filtration, distillation, crystallization, chromatography and the like. Such materials may be characterized using conventional means, including physical constants and spectral data.

Unless specified to the contrary, the reactions described herein take place at atmospheric pressure and over a temperature range from about -78 °C to about 150 °C, more specifically from about 0 °C. to about 125°C and more specifically at about room (or ambient) temperature, e.g., about 20 °C. Unless otherwise stated (as in the case of an hydrogenation), all reactions are performed under an atmosphere of nitrogen.

Prodrugs can be prepared by techniques known to one skilled in the art. These techniques generally modify appropriate functional groups in a given compound. These modified functional groups regenerate original functional groups by routine manipulation or *in vivo.* Amides and esters of the compounds of the present invention may be prepared according to conventional methods. A thorough discussion of prodrugs is provided in T. Higuchi and V. Stella, "Prodrugs as Novel Delivery Systems," Vol 14 of the A.C.S. Symposium Series, and in Bioreversible Carriers in Drug Design, ed. Edward B. Roche, American Pharmaceutical Association and Pergamon Press, 1987.

The compounds of the invention, or their pharmaceutically acceptable salts, may have asymmetric carbon atoms or quaternized nitrogen atoms in their structure. Compounds of Formula I that may be prepared through the syntheses described herein may exist as single stereoisomers, racemates, and as mixtures of enantiomers and diastereomers. The compounds may also exist as geometric isomers. All such single stereoisomers, racemates and mixtures thereof, and geometric isomers are intended to be within the scope of this invention. Some of the compounds of the invention may exist as tautomers. For example, where a ketone or aldehyde is present, the molecule may exist in the enol form; where an amide is present, the molecule may exist as the imidic acid; and where an enamine is present, the molecule may exist as an imine. All such tautomers are within the scope of the invention. In particular, imidazol-5-yl and

pyrazol-5-yl each can also exist in their respective tautomeric forms imidazol-4-yl and pyrazol-3-yl. Regardless of which structure or which terminology is used, each tautomer is included within the scope of the Invention.

The present invention also includes N-oxide derivatives and protected derivatives of compounds of Formula I. For example, when compounds of Formula I contain an oxidizable nitrogen atom, the nitrogen atom can be converted to an N-oxide by methods well known in the art. When compounds of Formula I contain groups such as hydroxy, carboxy, thiol or any group containing a nitrogen atom(s), these groups can be protected with a suitable "protecting group" or "protective group". A comprehensive list of suitable protective groups can be found in T.W. Greene, Protective Groups in Organic Synthesis, John Wiley & Sons, Inc. 1991. The protected derivatives of compounds of Formula I can be prepared by methods well known in the art.

Methods for the preparation and/or separation and isolation of single stereoisomers from racemic mixtures or non-racemic mixtures of stereoisomers are well known in the art. For example, optically active (R)- and (S)- isomers may be prepared using chiral synthons or chiral reagents, or resolved using conventional techniques. Enantiomers (R- and S-isomers) may be resolved by methods known to one of ordinary skill in the art, for example by: formation of diastereoisomeric salts or complexes which may be separated, for example, by crystallization; via formation of diastereoisomeric derivatives which may be separated, for example, by crystallization, selective reaction of one enantiomer with an enantiomer-specific reagent, for example enzymatic oxidation or reduction, followed by separation of the modified and unmodified enantiomers; or gas-liquid or liquid chromatography in a chiral environment, for example on a chiral support, such as silica with a bound chiral ligand or in the presence of a chiral solvent. It will be appreciated that where a desired enantiomer is converted into another chemical entity by one of the separation procedures described above, a further step may be required to liberate the desired enantiomeric form. Alternatively, specific enantiomer may be synthesized by asymmetric synthesis using optically active reagents, substrates, catalysts or solvents or by converting on enantiomer to the other by asymmetric transformation. For a mixture of enantiomers, enriched in a particular enantiomer, the major component enantiomer may be further enriched (with concomitant loss in yield) by recrystallization.

In addition, the compounds of the present invention can exist in unsolvated as well as solvated forms with pharmaceutically acceptable solvents such as water, ethanol, and the like. In general, the solvated forms are considered equivalent to the unsolvated forms for the purposes of the present invention.

The chemistry for the preparation of the compounds of this invention is known to those skilled in the art. In fact, there may be more than one process to prepare the compounds of the invention. For specific examples, see M. Barvian et al. J. Med. Chem. 2000, 43, 4606-4616; S. N. VanderWei et al. J. Med. Chem. 2005, 48, 2371-2387; P. L. Toogood et al. J. Med. Chem. 2005, 48, 2388-2406; J. Kasparec et al. Tetrahedron Letters 2003, 44, 4567-4570; and references cited therein. See also U.S. Pre-grant publication US2004/0009993 A1 (M. Angiolini et al.), and references cited therein. The following examples illustrate but do not limit the invention. All references cited herein are incorporated by reference in their entirety.

A Compound of Formula II where $R^1$ is 5- or 6-membered heterocycloalkyl which comprises one heteroatom and the heteroatom is -O-, $R^2$ is hydrogen, $R^4$ is methyl or ethyl, and $R^6$ is a 5- or 6- membered heteroaryl optionally substituted with 1, 2, 3, 4, or 5 $R^9$ groups (as defined in the Summary of the Invention for a Compound of Formula I) can be prepared according to Scheme 1.

## Scheme 1

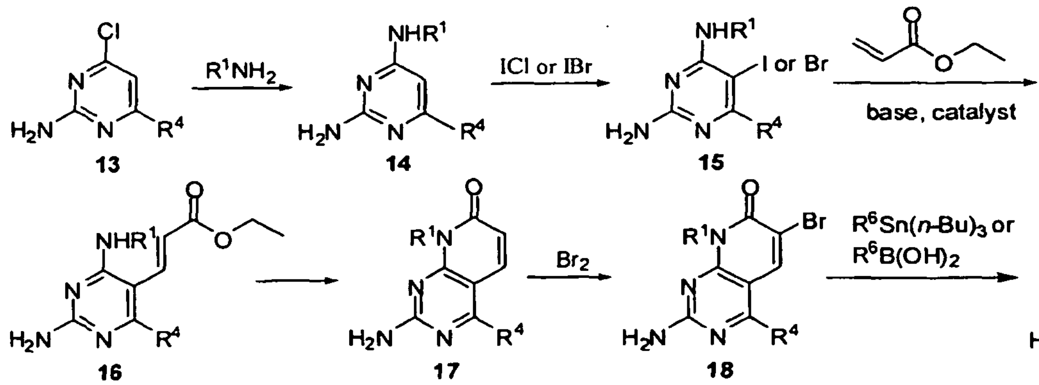


13 14 15 16 17 18 II

An intermediate of formula 14 is prepared by reacting a commercially-available intermediate of formula 13 with a commercially-available primary amine $R^1NH_2$ (as the free amine or salt thereof, such as an HCl salt) in solvents, such as water and such as a water/ethanol mixture, in the presence of a base such as TEA, and by heating the reaction. Intermediate **14** is then treated with iodine monochloride or iodine monobromide in a solvent such as methanol at around

0 °C and allowed to react at room temperature for approximately overnight or less as needed for the reaction to go to completion to form 15. After completion the residue can be triturated with acetone. Alternatively, after completion, the reaction mixture can be poured into 0.2 N sodium thiosulfate to quench excess iodine.

Intermediate 15 is then reacted in a solvent, such as DMA or DMF, with ethyl acrylate in the presence of a base, such as triethylamine, and in the presence of a catalyst, such as $Pd(OAc)_2$ in the presence of (+)BINAP or a catalyst such as $(Pd(PPh_3)_4$. The reaction is heated to approximately 95-100 °C and allowed to react for approximately overnight or less as needed for the reaction to go to completion to form 16. 16 is then optionally purified by column chromatography.

**17** is prepared by treating **16** with DBU optionally in the presence of a base such as DIEA at about room temperature. The reaction mixture is then heated to reflux or about 170 °C and allowed to proceed until completion, approximately 5-15 h. After evaporation of the solvent, the residue is triturated with acetone and collected by filtration to yield 17. Alternatively, the reaction is allowed to cool to room temperature and then purified directly by column chromatography.

**18** is prepared by reacting **17** with a brominating agent such as $Br_2$ in a solvent such as DCM at about room temperature. The reaction mixture is then stirred for approximately four hours to overnight. The resulting product is filtered and then suspended in a solvent such as DCM and treated with a base such as triethylamine. The organic layers are washed with water and dried over a drying agent such as $Na_2SO_4$ to yield **18.** Alternatively, after completion, the reaction mixture is partially concentrated and acetone is added, followed by concentrating and precipitating in a solvent such as ethyl acetate which can then be collected by filtration to yield **18.**

A Suzuki coupling can be performed on **18** using a boronic acid (or ester) of formula $R^6B(OH)_2$ in a solvent such as a DME-$H_2O$ mixture or such as a dioxane-$H_2O$ mixture, in the presence of a catalyst such as $Pd(dpppf)_2$ and in the presence of a base such as triethylamine. The reaction mixture is heated to reflux or about 95-100 °C for approximately 4 h. After cooling to room temperature, the reaction mixture is partitioned with water and ethyl acetate. After separation, the organic layer is dried over a drying agent such as $Na_2SO_4$ to yield a Compound of Formula **II**.

Alternatively, a Stille coupling can be performed on **18** (either as the free base or as a salt such as an HBr salt) using a tin reagent of formula $R^6Sn(n\text{-}Bu)_3$, in a solvent such as toluene, in the presence of a catalyst such as $Pd(PPh_3)_4$, and optionally in the presence of a base such as triethylamine of Hunig's base. The reaction is heated at about 80-110 °C for about four hours. After cooling to room temperature, the reaction mixture can be purified by column chromatography to yield a Compound of Formula II. Alternatively, after cooling to room temperature, 40% KF on alumina is added. The mixture is then filtered through Celite to remove the alumina and the Celite is then washed with a solvent such as ethyl acetate. The resulting filtrate can then be washed with 1 M aqueous KF and brine. The organic layers are dried over a drying agent such as $MgSO_4$, filtered and concentrated in vacuo. The residue can then be triturated with methylene chloride and hexane to yield a Compound of Formula II.

## Synthetic Examples

### Intermediate 6(a)

2-Amino-6-bromo-4-methyl-8-(tetrahydrofuran-3-yl)pyrido[2,3-*d*]pyrimidin-7(8*H*)-one hydrobromide

Step 1

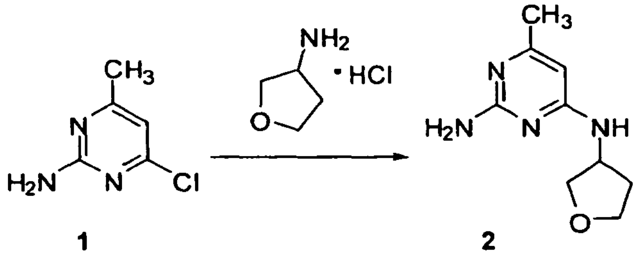

1 2

To a solution of 2-amino-4-chloro-6-methylpyrimidine (8.10 g, 56.63 mmol, Aldrich) and *R,S*-tetrahydrofuran-3-ylamine hydrochloride (7.0 g, 56.64 mmol, Small Molecules), $H_2O$ (70 mL), EtOH (30 mL) and triethylamine (28.0 mL, 198.2 mmol) were added. The reaction mixture was heated to a gentle reflux for 120 h . Monitoring of the reaction by LC/MS indicated that the reaction was complete. The reaction was allowed to cool to room temperature. The volatile

organics were removed under reduced pressure and the aqueous layer was washed with ethyl acetate (50 mL). The aqueous layer was reduced to about half the volume and dried on a lyophilizer overnight to afford a quantitative yield of 6-methyl-*N*4-(tetrahydrofuran-3-yl)pyrimidine-2,4-diamine. $^1H$ NMR (400 MHz, $CH_3OD$); δ 5.93 (s, 1H), 3.94 (m, 2H), 3.85 (m, 1H), 3.72 (m, 3H), 2.30 (m, 1H), 2.25 (s, 3H), 1.90 (m, 1H); MS (EI) for $C_9H_{14}N_4O$: 195.1 $[MH]^+$.

## Step 2

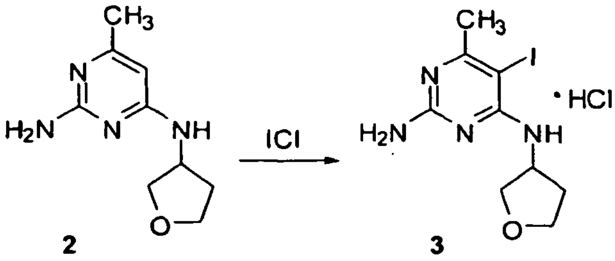


To the solution of 6-methyl-*N*4-(tetrahydrofuran-3-yl)pyrimidine-2,4-diamine (10.90 g, 56.18 mmol) in methanol (150 mL), which was cooled on an ice-water bath, was added ICl (1.0 M dichloromethane solution, 85 mL, 84.27 mmol, Aldrich). The reaction mixture was stirred at room temperature overnight and monitored by LC/MS. After completion, the reaction mixture was poured into 0.2 N sodium thiosulfate and stirred for 20 minutes to quench excess iodine. To this was added sat. $NaHCO_3$ aqueous solution to quench HI generated from the reaction. The reaction was extracted into dichloromethane (50mL x 6) and dried over $MgSO_4$. After filtration and evaporation of solvent on a rotary evaporator, it was purified by column chromatography (30 % ethyl acetate/hexane to 100% ethyl acetate) to afford solid 2.53 g (14.0 % yield) of 5-iodo-6-methyl-*N*4-(tetrahydrofuran-3-yl)pyrimidine-2,4-diamine as a brown powder. $^1H$ NMR (400 MHz, $CD_3OD$) δ 4.73 (m, 1H), 4.00 (m, 2H), 3.83 (m, 1H), 3.74 (m, 1H), 2.51 (s, 3H), 2.36 (m, 1H), 2.06 (m, 1H). MS (EI) for $C_9H_{13}IN_4O$:321.0 $[MH]^+$.

## Step 3

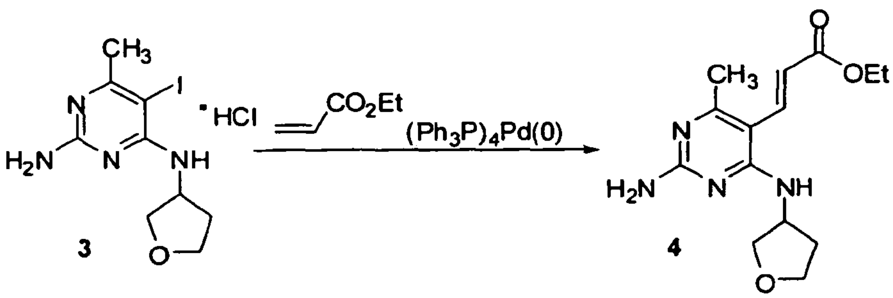


To a mixture of *N*4-ethyl-5-iodo-6-methylpyrimidine-2,4-diamine (1.80 g, 5.62 mmol), ethyl acrylate (1.84 mL, 16.86 mmol), triethylamine (2.40 mL, 16.86 mmol) in DMF (30 mL), which was pre-purged with nitrogen for 5 minutes, was added tetrakis(triphenylphosphine)palladium ($Pd(PPh_3)_4$, 650 mg, 10 mmol). The reaction mixture was heated to 95 °C and stirred overnight. After completion, the reaction mixture was poured into $H_2O$ and extracted into ethyl acetate (5 times). The organic layer was dried over $MgSO_4$ and concentrated to dryness on a rotary evaporator. The reaction mixture was purified by flash column chromatography (50% ethyl acetate/hexane to 100 % ethyl acetate) to give a yellow powder (1.45 g, 88 % yield) of (*E*)-ethyl 3-(2-amino-4-methyl-6-(tetrahydrofuran-3-ylamino)pyrimidin-5-yl)acrylate as a yellow solid. $^1H$ NMR (400 MHz, $CDCl_3$) δ 7.67(d, *J*=15.9Hz, 2H), 6.05(d, *J*=15.9Hz, 2H), 5.18 (d, 1H), 4.85 (bs, 2H), 4.68(m, 1H), 4.27(t, 3H), 3.97(m, 2H), 3.83(m, 1H), 3.68(m, 1H), 2.33(s, 3H), 1.84(m, 1H), 1.64(m, 1H). MS (EI) for $C_{14}H_{20}N_4O_3$: 293.1 $[MH]^+$.

Step 4

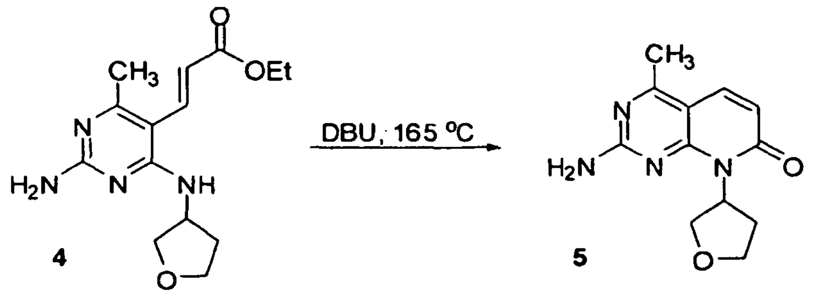


4 5

A round-bottomed flask was charged with ethyl (*E*)-ethyl 3-(2-amino-4-methyl-6-(tetrahydrofuran-3-ylamino) pyrimidin-5-yl)acrylate (1.45 g, 4.96 mmol) and 1,8-diazabicyclo[5.4.0]undec-7-ene (DBU, 3.0 mL, 19.84 mmol). The reaction mixture was stirred at 170 °C for 5 h, cooled to room temperature, and directly purified by column chromatography (100% ethyl acetate to 5% MeOH/ethyl acetate) to afford 2-amino-4-methyl-8-(tetrahydrofuran-3-yl)pyrido[2,3-d]pyrimidin-7(8*H*)-one as a brown solid (623.0 mg, 51 %). $^1$H NMR (400 MHz, $CDCl_3$); δ 7.68 (d, *J*=9.4Hz, 1H), 6.38 (d, *J*=9.4Hz, 1H), 6.24 (m, 1H), 5.19 (s, 2H), 4.36 (q, 1H), 4.12 (t, 1H), 4.01 (m, 2H), 2.57 (s, 3H), 2.51 (m, 1H), 2.15 (m, 1H) (s, 2H); MS (EI) for $C_{12}H_{14}N_4O_2$: 247.1 $[MH]^+$.

Step 5

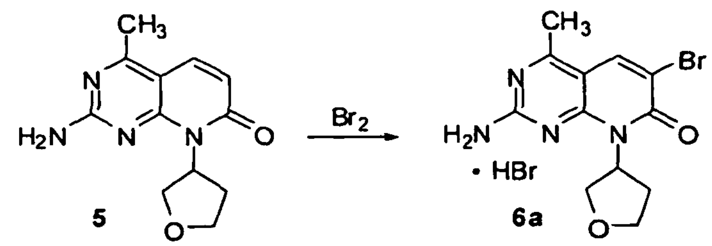


• HBr

5 6a

To a solution of 2-amino-4-methyl-8-(tetrahydrofuran-3-yl)pyrido[2,3-*d*]pyrimidin-7(8*H*)-one (125 mg, 0.508 mmol) in dichloromethane (10 mL) was added bromine (52 μL, 1.02 mmol). This reaction mixture was stirred for 4 h at room temperature and was monitored by LC-MS and TLC. After completion, the reaction mixture was concentrated to half of its original volume and acetone was added. The mixture was concentrated and ethyl acetate was added. The precipitate was collected by filtration and washed with ethyl acetate to give 2-amino-6-bromo-4-methyl-8-(tetrahydrofuran-3-yl)pyrido[2,3-*d*]pyrimidin-7(8*H*)-one hydrobromide as a yellowish powder, 185.0 mg (89 % yields). $^1$H NMR (400 MHz, DMSO-d6); δ 8.40 (s, 1H), 7.45 (br.,2H), 6.20 (m, 1H), 4.20 (q, 1H), 3.85 (m, 3H), 2.48 (m, 1H), 2.10 (s,3H), 2.08 (m, 1H); MS (EI) for $C_{12}H_{13}BrN_4O_2$: 324.1 $[M]^+$ , 326.1$[(M+2H)]^+$.

Intermediate 6(b): Similarly, 2-amino-6-bromo-4-methyl-8-[(3R)-tetrahydrofuran-3-yl]pyrido[2,3-*d*]pyrimidin-7 (8*H*)-one hydrobromide was prepared by replacing *R,S*-tetrahydrofuran-3-ylamine hydrochloride used in the preparation of Intermediate 6(a) with *R*-tetrahydrofuran-3-ylamine hydrochloride. $^1$H NMR (400 MHz, $d_6$-DMSO) δ 8.41 (s, 1H), 7.47 (br s, 1H), 6.22-6.10 (m, 1H), 6.01 (br s, 1H), 4.19 (q, 1H), 3.91-3.80 (m, 3H), 2.51 (s, 3H), 2.37-2.27 (m, 1H), 2.09-1.97 (m, 1H); MS (EI) for $C_{12}H_{13}BrN_4O_2$: 325, 327 $[MH]^+$ (Br isotope pattern). Analytical hplc purity 83%.

**Intermediate 6(c):** Similarly, 2-amino-6-bromo-4-methyl-8-[(3*S*)-tetrahydrofuran-3-yl]pyrido[2,3-*d*]pyrimidin-7 (8*H*)-one hydrobromide was prepared by replacing *R,S*-tetrahydrofuran-3-ylamine hydrochloride used in the preparation of Intermediate 6(a) with *S*-tetrahydrofuran-3-ylamine hydrochloride. $^1$H NMR (400 MHz, $d_6$-DMSO) δ 8.40 (s, 1H), 7.42 (br s, 1H), 6.61 (br s, 1H), 6.21-6.10 (m, 1H), 4.19 (q, 1H), 3.91-3.79 (m, 3H), 2.52 (s, 3H), 2.36-2.27 (m, 1H), 2.09-1.97 (m, 1H); MS (EI) for $C_{12}H_{13}BrN_4O_2$: 325, 327 $[MH]^+$ (Br isotope pattern). Analytical hplc purity 90%.

**Intermediate 6(d):** Similarly, 2-amino-6-bromo-4-methyl-8-(tetrahydro-2*H*-pyran-4-yl)pyrido[2,3-*d*]pyrimidin-7 (8*H*)-one hydrobromide was prepared by replacing *R,S*-tetrahydrofuran-3-ylamine hydrochloride used in the preparation of Intermediate 6(a) with tetrahydro-2*H*-pyran-4-amine. $^1$H NMR (400 MHz, $d_6$-DMSO) δ 8.40 (s, 1H), 7.52 (br s, 1H), 5.71-5.55 (m, 1H), 5.15 (br s, 1H), 3.97 (dd, 2H), 3.36 (t, 2H), 2.91-2.75 (m, 2H), 2.52 (s, 3H), 1.46 (dd, 2H); MS (EI) for $C_{13}H_{15}BrN_4O_2$: 339, 341 $[MH]^+$ (Br isotope pattern). Analytical HPLC purity of 92%.

**Intermediate 6(e):** Similarly, 2-amino-6-bromo-4-methyl-8-(tetrahydro-2*H*-pyran-3-yl)pyrido[2,3-*d*]pyrimidin-7 (8*H*)-one hydrobromide was prepared by replacing *R,S*-tetrahydrofuran-3-ylamine hydrochloride used in the preparation of Intermediate 6(a) with *R,S*-tetrahydro-2*H*-pyran-3-amine. $^1$H NMR (400 MHz, DMSO); δ 8.41 (s, 1H), 7.60 (bs, 2H),

5.53 (bs, 1H), 4.37 (m, 1H), 3.87 (m, 1), 3.39 (m, 2H), 2.75 (m, 1H), 1.69 (m, 3H); MS (EI) for $C_{13}H_{15}BrN_4O_2$: 338.9 (M+), 340.9 (M+2).

**Intermediate 6(f):** (*S*)-2-amino-6-bromo-4-methyl-8-(tetrahydro-2*H*-pyran-3-yl)pyrido[2,3-*d*]pyrimidin-7 (8*H*)-one can be prepared using the procedure described for Intermediate 6(a) by replacing *R,S*-tetrahydrofuran-3-ylamine hydrochloride used in the preparation of Intermediate 6(a) with (*S*)-tetrahydrofuran-3-ylamine hydrochloride.

**Intermediate 6(g):** (*R*)-2-amino-6-bromo-4-methyl-8-(tetrahydro-2H-pyran-3-y)pyndo[2,3-*d*]pyrimidin-7 (8*H*)-one can be prepared using the procedure described for Intermediate 6(a) by replacing *R,S*-tetrahydrofuran-3-ylamine hydrochloride used in the preparation of Intermediate 6(a) with (*R*)-tetrahydrofuran-3-ylamine hydrochloride.

**Example 1**

2-Amino-4-methyl-6-(1*H*-pyrazol-5-yl)-8-(tetrahydrofuran-3-yl)pyrido[2,3-*d*]pyrimidin-7(8*H*)-one

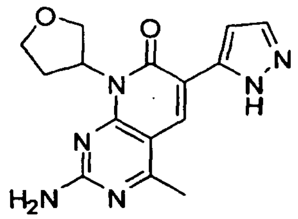

A mixture of 2-amino-6-bromo-4-methyl-8-(tetrahydrofuran-3-yl)pyrido[2,3-*d*]pyrimidin-7(8*H*)-one hydrobromide (100 mg, 0.247 mmol) prepared as described for Intermediate 6(a), 1*H*-pyrazole-5-yl boronic acid (41.4 mg, 0.371 mmol), $Pd(dpppf)_2$ (20.1 mg, 0.0247 mmol), TEA (100 mg, 0.99 mmol), dioxane (0.8mL), and water (0.2 mL) was heated to 95 °C for 3 hours (monitored by LC/MS). Cooling down to room temperature, the reaction mixture was partitioned with water and ethyl acetate. After separation, the organic layer was dried with $Na_2SO_4$. The product 2-amino-4-methyl-6-(1*H*-pyrazol-5-yl)-8-(tetrahydrofuran-3-yl)pyrido[2,3-*d*]pyrimidin-7(8*H*)-one (27.7g, 36%) was obtained by silica gel column chromatography. $^1$H NMR (300 MHz, DMSO-$d_6$): δ 13.0 (bs, 1H), 8.61 (s, 1H), 7.72(s, 1H), 7.25 (bd, 2H), 6.92 (S, 1H), 6.25 (m, 1H), 4.35~4.20(m, 1H), 4.05~3.75(m,3H), 2.6(s, 3H), 2.48~2.40 (m,1H), 2.13~2.0 (m, 1H). MS (EI) for $C_{15}H_{16}N_6O_2$: 313.2 (M+1$^+$).

The compounds in Examples 2, 3, 4, and 5 were prepared using the procedures described for Example 1 by replacing Intermediate 6(a) used in Example 1 with Intermediate 6(c), Intermediate 6(b), Intermediate 6(d), and Intermediate 6(e), respectively.

**Example 2:** 2-Amino-4-methyl-6-(1*H*-pyrazol-5-yl)-8-[(3*S*)-tetrahydrofuran-3-yl]pyrido[2,3-*d*]pyrimidin-7 (8*H*)-one. $^1$H NMR (300 MHz, $CDCl_3$): δ 7.70 (d, J = 9.0Hz, 1H), 6.40(d, J = 9.0Hz, 1H), 6.30~6.20 (m, 1H), 6.48~6.38 (m, 1H), 5.46 (bs, 2H), 4.44~4.00 (m, 4H), 2.56 (s, 3H), 2.55~2.46 (m,1H), 2.23~2.13 (m, 1H). MS (EI) for $C_{15}H_{16}N_6O_2$: 313.2 (M+1$^+$).

**Example 3:** 2-Amino-4-methyl-6-(1*H*-pyrazol-5-yl)-8-[(3*R*)-tetrahydrofuran-3-yl]pyrido[2,3-*d*]pyrimidin-7 (8*H*)-one. $^1$H NMR (300 MHz, $CD_3OD$): δ 8.39 (S, 1H), 7.62(s, 1H), 6.95 (s, 1H), 6.48~6.38 (m, 1H), 4.44~4.40 (m, 1H), 4.19~3.97 (m, 3H), 2.64 (s, 3H), 2.59~2.50 (m,1H), 2.23~2.15 (m, 1H). MS (EI) for $C_{15}H_{16}N_6O_2$: 313.2 (M+1$^+$).

**Example 4:** 2-Amino-4-methyl-6-(1*H*-pyrazol-5-yl)-8-(tetrahydro-2*H*-pyran-4-yl)pyrido[2,3-*d*]pyrimidin-7 (8*H*)-one. $^1$H NMR (300 MHz, DMSO-$d_6$): δ 13.0 (bs, 1H), 8.37 (bs, 1H), 7.7~7.2 (m, 3H), 6.95 (bs, 1H), 5.70 (m, 1H), 4.2~4.00 (m, 2H), 3.1~2.8 (m, 2H), 2.57(s, 3H), 1.50~1.40 (m, 2H), 1.0~0.8 (m, 2H). MS (EI) for $C_{16}H_{18}N_6O_2$: 327.2 (M+1$^+$).

**Example 5:** 2-Amino-4-methyl-6-(1*H*-pyrazol-5-yl)-8-(tetrahydro-2*H*-pyran-3-yl)pyrido[2,3-*d*]pyrimidin-7 (8*H*)-one. $^1$H NMR (300 MHz, DMSO-$d_6$): δ 13.0 (bs, 1H), 8.34 (bs, 1H), 7.49 (bs, 1H), 7.32 (bs, 1H), 7.21 (bs, 1H), 6.91 (bs, 1H), 5.60 (m, 1H), 4.49 (m, 1H), 3.86 (m, 1H), 3.66 (m, 1H), 3.40 (m, 1H), 2.44(s, 3H), 1.70~1.60 (m, 3H). MS (EI) for $C_{16}H_{18}N_6O_2$: 327.2 (M+1$^+$).

**Examples 6:** 2-Amino-4-methyl-6-(1*H*-pyrazol-5-yl)-8-[(3*S*)-tetrahydro-2*H*-pyran-3-yl]pyrido[2,3-*d*]pyrimidin-7 (8*H*)-one can be prepared using the procedures described above for Example 1 by replacing Intermediate 6(a) in Example 1 with Intermediate 6(f).

**Example 7:** 2-Amino-4-methyl-6-(1*H*-pyrazol-5-yl)-8-[(3*R*)-tetrahydro-2*H*-pyran-3-yl]pyrido[2,3-*d*]pyrimidin-7 (8*H*)-one can be prepared using the procedures described above for Example 1 by replacing Intermediate 6(a) in Example 1 with Intermediate 6(g).

**Example 8**

2-Amino-4-methyl-8-(tetrahydrofuran-3-yl)-6-(1,3-thiazol-2-yl)pyrido[2,3-*d*]pyrimidin-7(8*H*)-one

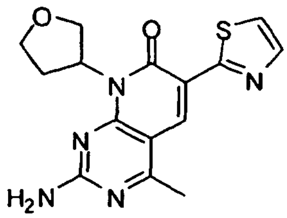

To a solution of 2-amino-6-bromo-4-methyl-8-(tetrahydrofuran-3-yl)pyrido[2,3-*d*]pyrimidin-7(8*H*)-one (60 mg, 0.185 mmol) prepared using procedures described for Intermediate 6(a), in toluene (5 mL) and tributylstannylthiazole (139 mg, 0.370 mmol) was added $Pd(PPh_3)_4$ (21 mg, 10 mol%). The reaction mixture was heated to 110 °C. After 4h, the reaction was cooled to room temperature and 40% KF on alumina was added. After 30 minutes stirring at room temperature, the alumina was removed by filtration on Celite and the Celite was washed with ethyl acetate. The resulting filtrate was washed consecutively with 1 M aqueous KF and brine. The organic layers were dried over $MgSO_4$, filtered and concentrated in vacuo. The residue was triturated with methylene chloride and hexane to give 2-amino-4-methyl-8-(tetrahydrofuran-3-yl)-6-(thiazol-2-yl)pyrido[2,3-*d*]pyrimidin-7(8*H*)-one (58 mg, 85% pure), which was purified by flash column chromatography to give the product as a yellow solid (24 mg, 40% yield). $^1H$ NMR (400 MHz, MeOD); δ 8.97 (s, 1H), 7.92 (d, *J*=3.4Hz, 1H), 7.61 (d, , *J*=3.4Hz, 1H), 6.45 (m, 1H), 4.43 (q, 1H), 4.19 (t, 1H), 4.01 (m, 2H), 2.69 (s, 3H), 2.57 (m, 1H), 2.22 (m, 1H); $^1H$ NMR (400 MHz, DMSO-d6); δ 8.84 (s, 1H), 7.95 (d, *J*=3.4Hz, 1H), 7.74 (d, *J*=3.4Hz, 1H), 7.55 (m, 2H), 6.27 (m, 1H), 4.30 (qr, 1H),4.00 (qn, 1H), 3.91 (m, 2H), 2.44 (m, 1H), 2.10 (m, 1H);MS (EI) for $C_{15}H_{15}N_5O_2S$: 330.1 $[MH]^+$. Analytical HPLC purity > 96%.

**Example 9:** 2-Amino-4-methyl-8-[(3*S*)-tetrahydrofuran-3-yl]-6-(1,3-thiazol-2-yl)pyrido[2,3-*d*]pyrimidin-7 (8*H*)-one (5.9mg, 4.9% yield) was prepared using the procedures described for Example 8 by replacing Intermediate 6 (a) in Example 8 with Intermediate 6(c). $^1H$ NMR (400MHZ, $CDCl_3$); d 8.7 (s, 1H), 7.95 (d, 1H), 7.46 (s, 1H), 6.38 (m, 1H), 5.38 (s, 2H), 4.46 (q, 1H), 4.25 (t, 1H), 4.06(m, 2H), 2.73 (s, 3H), 2.60(m, 1H), 2.24 (m, 1H); MS(EI) for $C_{15}H_{15}N5O_2S$: 330.1 ($MH^+$).

**Example 10:** 2-Amino-4-methyl-8-[(3R)-tetrahydrofuran-3-yl]-6-(1,3-thiazol-2-yl)pyrido[2,3-*d*]pyrimidin-7 (8*H*)-one (1.0 mg 0.8%) was prepared using the procedures described for Example 8 by replacing Intermediate 6(a) in Example 8 with Intermediate 6(b). $^1H$ NMR (400MHZ, $CDCl_3$); 8.70 (s, 1H), 7.95 (s, 1H), 7.47 (s, 1H), 6.39 (m, 1H), 5.37 (s, 2H), 4.44 (q, 1H), 4.22 (m, 1H), 4.06 (m, 2H), 2.73 (s, 3H), 2.61 (m, 1H), 2.25 (m, 1H);MS(EI) for $C_{15}H_{15}N5O_2S$: 330.1 ($MH^+$).

**Example 11:** 2-Amino-4-methyl-8-(tetrahydro-2*H*-pyran-4-yl)-6-(1,3-thiazol-2-yl)pyrido[2,3-*d*]pyrimidin-7 (8*H*)-one was prepared using the procedures described for Example 8 by replacing Intermediate 6(a) in Example 8 with Intermediate 6(d). MS(EI) for $C_{16}H_{17}N_5O_2S$: 344.2 ($MH^+$).

**Example 12:** 2-Amino-4-methyl-8-(tetrahydro-2*H*-pyran-3-yl)-6-(1,3-thiazol-2-yl)pyrido[2,3-d]pyrimidin-7 (8*H*)-one (8.2 mg, 9%) was prepared using the procedures described for Example 8 by replacing Intermediate 6(a) in Example 8 with and Intermediate 6(e). $^1H$ NMR (400 MHz, DMSO); δ 8.82 (s, 1H), 7.94 (d, 1H), 7.73 (d, 1H), 7.56 (s, 2H), 5.65 (bs, 1H), 4.51 (bs, 1H), 3.88 (m, 1H), 3.45 (m, 2H), 2.90 (m, 1H), 2.63 (s, 3H), 1.75 (m, 3H). MS (EI) for $C_{16}H_{17}N_SO_2S$: 344.2 ($MH^+$).

**Example 13:** 2-Amino-4-methyl-8-[(3*S*)-tetrahydro-2*H*-pyran-3-yl]-6-(1,3-thiazol-2-yl)pyrido[2,3-d]pyrimidin-7 (8*H*)-one can be prepared using the procedures described above for Example 8 by replacing Intermediate 6(a) in Example 8 with Intermediate 6(f), which can be prepared as described above.

**Example 14:** 2-amino-4-methyl-8-[(3*R*)-tetrahydro-2*H*-pyran-3-yl]-6-(1,3-thiazol-2-yl)pyrido[2,3-*d*]pyrimidin-7 (8*H*)-one can be prepared using the procedures described above for Example 8 by replacing Intermediate 6(a) in Example 8 with Intermediate 6(g), which can be prepared as described above.

**Example 15**

2-amino-6-{(1-[(ethyloxy)methyl]-1*H*-imidazol-2-yl)-4-methyl-8-(tetrahydrofuran-3-yl)pyrido[2,3-*d*]pyrimidin-7(8*H*)-one trifluoroacetate salt

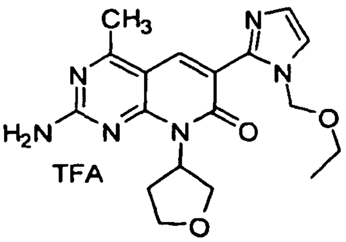

A mixture of 2-amino-6-bromo-4-methyl-8-(tetrahydrofuran-3-yl)pyrido[2,3-*d*]pyrimidin-7(8*H*)-one hydrobromide salt (150 mg, 0.369 mmol) prepared using procedures as described for Intermediate 6(a), triethylamine (0.052 mL, 0.373 mmol), and 1-ethoxymethyl-2-tributylstaruiyl-1*H*-imidazole (85% pure; 720 mg, 1.47 mmol) prepared as described by Xie, L. et al. US 2003220348), in toluene (5 mL) was sparged with nitrogen for 5 minutes. $Pd(PPh_3)_4$ (854 mg, 0.738 mmol) was added and the reaction mixture was heated at 110 °C. After 1h, the reaction was cooled to room temperature and was sequentially purified by flash column chromatography ($SiO_2$~60 mL) (using EtOAc to 50% MeOH in EtOAc with 1% triethylamine as eluent). The material from the purest fractions was further purified by preparative HPLC using trifluoroacetic acid as the eluent buffer to afford 2-amino-6-{(1-[(ethyloxy)methyl]-1*H*-imidazol-2-yl)-4-methyl-8-(tetrahydrofuran-3-yl)pyrido[2,3-*d*]pyrimidin-7(8*H*)-one trifluoroacetate salt (40.5 mg, 23% yield) as an off-white solid. $^1H$ NMR (400 MHz, $d_6$-DMSO) δ 8.59 (s, 1H), 7.99 (d, 1H), 7.87 (d, 1H), 7.85-7.70 (br d, 2H), 6.26-6.16 (m, 1H), 5.54 (s, 2H), 4.21 (q, 1H), 3.98-3.86 (m, 3H), 3.51 (q, 2H), 2.54 (s, 3H), 2.48-2.35 (m, 1H), 2.16-2.04 (m, 1H), 1.09 (t, 3H); MS (EI) for $C_{18}H_{22}N_6O_3$: 371 $[MH]^+$. Analytical HPLC purity > 93%.

**Example 16:** 2-Amino-6-{1-[(ethyloxy)methyl]-1*H*-imidazol-2-yl}-4-methyl-8-[(3*S*)-tetrahydrofuran-3-yl]pyrido [2,3-d]pyrimidin-7(8*H*)-one trifluoroacetate salt (26 mg, 18% yield) was prepared using the procedures described for Example 15 by replacing Intermediate 6(a) in Example 15 with Intermediate 6(c). $^1H$ NMR (400 MHz, $CD_3OD$) δ 8.70 (s, 1H), 7.87 (d, 1H), 7.68 (d, 1H), 6.45-6.35 (m, 1H), 5.60 (s, 2H), 4.35 (q, 1H), 4.13 (t, 1H), 4.03-3.95 (m, 2H), 3.67 (q, 2H), 2.62 (s, 3H), 2.56-2.45 (m, 1H), 2.28-2.16 (m, 1H), 1.22 (t, 3H); MS (EI) for $C_{18}H_{22}N_6O_3$: 371 $[MH]^+$.

**Example 17:** 2-Amino-6-{1-[(ethyloxy)methyl]-1*H*-imidazol-2-yl}-4-methyl-8-[(3*R*)-tetrahydrofuran-3-yl]pyrido [2,3-*d*]pyrimidin-7(8*H*)-one trifluoroacetate salt (10.7 mg, 6% yield) was prepared using the procedures described for Example 15 by replacing Intermediate 6(a) in Example 15 with Intermediate 6(b). $^1H$ NMR (400 MHz, $CD_3OD$) δ 8.70 (s, 1H), 7.86 (d, 1H), 7.68 (d, 1H), 6.45-6.35 (m, 1H), 5.60 (s, 2H), 4.35 (q, 1H), 4.13 (t, 1H), 4.03-3.94 (m, 2H), 3.67 (q, 2H), 2.62 (s, 3H), 2.55-2.45 (m, 1H), 2.27-2.16 (m, 1H), 1.22 (t, 3H); MS (EI) for $C_{18}H_{22}N_6O_3$: 371 $[MH]^+$. Analytical HPLC purity > 92%.

**Example 18:** 2-Amino-6-{1-[(ethyloxy)methyl]-1*H*-imidazol-2-yl}-4-methyl-8-(tetrahydro-2*H*-pyran-4-yl)pyrido [2,3-*d*]pyrimidin-7(8*H*)-one can be prepared using the procedures in Example 15 by replacing Intermediate 6(a) in Example 15 with Intermediate 6(d), prepared as described above.

**Example 19:** 2-Amino-6-{1-[(ethyloxy)methyl]-1*H*-imidazol-2-yl}-4-methyl-8-(tetrahydro-2*H*-pyran-3-yl)pyrido [2,3-*d*]pyrimidin-7(8*H*)-one can be prepared using the procedures in Example 15 by replacing Intermediate 6(a) in Example 15 with Intermediate 6(e), prepared as described above.

**Example 20:** 2-Amino-6-{1-[(ethyloxy)methyl]-1*H*-imidazol-2-yl}-4-methyl-8-[(3*S*)-tetrahydro-2*H*-pyran-3-yl] pyrido[2,3-*d*]pyrimidin-7(8*H*)-one can be prepared using the procedures in Example 15 by replacing Intermediate 6(a) in Example 15 with Intermediate 6(f), which can be prepared as described above.

**Example 21:** 2-Amino-6-{1-[(ethyloxy)methyl]-1*H*-imidazol-2-yl}-4-methyl-8-[(3*R*)-tetrahydro-2*H*-pyran-3-yl] pyrido[2,3-*d*]pyrimidin-7(8*H*)-one can be prepared using the procedures in Example 15 by replacing Intermediate 6(a) in Example 15 with Intermediate 6(g), which can be prepared as described above.

**Example 22:** 2-Amino-6-(1*H*-imidazol-2-yl)-4-methyl-8-(tetrahydrofuran-3-yl)pyrido[2,3-*d*]pyrimidin-7(8*H*)-one can be prepared from the product of Example 15 using procedures known to one of ordinary skill in the art, in particular, by treating with a suitable Lewis acid and a nucleophile.

**Example 23:** 2-amino-6-(1*H*-imidazol-2-yl)-4-methyl-8-[(3*S*)-tetrahydrofuran-3-yl]pyrido[2,3-*d*]pyrimidin-7 (8*H*)-one can be prepared from the product of Example 16 using procedures known to one of ordinary skill in the art, in particular, by treating with a suitable Lewis acid and a nucleophile.

**Example 24:** 2-amino-6-(1*H*-imidazol-2-yl)-4-methyl-8-[(3*R*)-tetrahydrofuran-3-yl]pyrido[2,3-*d*]pyrimidin-7 (8*H*)-one can be prepared from the product of Example 17 using procedures known to one of ordinary skill in the art, in particular, by treating with a suitable Lewis acid and a nucleophile.

**Example 25:** 2-amino-6-(1*H*-imidazol-2-yl)-4-methyl-8-(tetrahydro-2*H*-pyran-4-yl)pyrido[2,3-*d*]pyrimidin-7 (8*H*)-one can be prepared from the product of Example 18 using procedures known to one of ordinary skill in the art, in particular, by treating with a suitable Lewis acid and a nucleophile.

**Example 26:** 2-amino-6-(1*H*-imidazol-2-yl)-4-methyl-8-(tetrahydro-2*H*-pyran-3-yl)pyrido[2,3-*d*]pyrimidin-7 (8*H*)-one can be prepared from the product of Example 19 using procedures known to one of ordinary skill in the art, in

particular, by treating with a suitable Lewis acid and a nucleophile.

**Example 27:** 2-amino-6-(1*H*-imidazol-2-yl)-4-methyl-8-[(3*S*)-tetrahydro-2*H*-pyran-3-yl]pyrido[2,3-*d*]pyrimidin-7(8*H*)-one can be prepared from the product of Example 20 using procedures known to one of ordinary skill in the art, in particular, by treating with a suitable Lewis acid and a nucleophile.

**Example 28:** 2-amino-6-(1*H*-imidazol-2-yl)-4-methyl-8-[(3*R*)-tetrahydro-2*H*-pyran-3-yl]pyrido[2,3-*d*]pyrimidin-7(8*H*)-one can be prepared from the product of Example 21 using procedures known to one of ordinary skill in the art, in particular, by treating with a suitable Lewis acid and a nucleophile.

**Example 29**

2-amino-4-methyl-8-(tetrahydro-2*H*-pyran-3-yl)-6-(1,3-thiazol-5-yl)pyrido[2,3-*d*]pyrimidin-7-(8*H*)-one

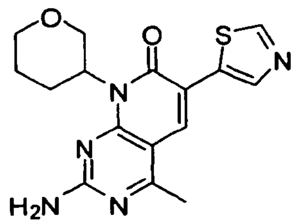

A mixture of 2-amino-6-bromo-4-methyl-8-(tetrahydro-2*H*-pyran-3-yl)pyrido[2,3-*d*]pyrimidin-7(8*H*)-one hydrobromide salt (155 mg, 0.369 mmol) prepared using procedures as described for Intermediate 6(e), triethylamine (0.052 mL, 0.373 mmol) and 5-(tributylstannyl)thiazole (700 mg, 1.87 mmol) in toluene (5 mL) was sparged with nitrogen for 5 minutes. $Pd(PPh_3)_4$ (427 mg, 0.369 mmol) was added and the reaction mixture was heated at 110 °C. After 1h, the reaction was cooled to room temperature and was purified by flash column chromatography ($SiO_2$ ~60 mL) (using 0-10% MeOH in EtOAc as eluent). The purest fractions were combined and concentrated and the residue was triturated with methanol. The solid was collected by filtration, was washed with methanol and then was dried to afford 2-amino-4-methyl-8-(tetrahydro-2*H*-pyran-3-yl)-6-(1,3-thiazol-5-yl)pyrido[2,3-*d*]pyrimidin-7(8*H*)-one (20 mg, 16% yield) as a tan solid. $^1$H NMR (400 MHz, $d_6$-DMSO) $\delta$ 9.02 (s, 1H), 8.64 (s, 1H), 8.52 (s, 1H), 7.40 (s, 2H), 5.64 (br s, 1H), 4.48 (br s, 1H), 3.88 (d, 1H), 3.69 (dd, 1H), 3.49-3.35 (m, 1H), 2.95-2.78 (m, 1H), 2.63 (s, 3H), 1.82-1.65 (m, 3H); MS (EI) for $C_{16}H_{17}N_5O_2S$: 344 $[MH]^+$. Analytical hplc purity > 90%.

**Example 30:** 2-Amino-4-methyl-8-(tetrahydrofuran-3-yl)-6-(1,3-thiazol-5-yl)pyrido[2,3-*d*]pyrimidin-7(8*H*)-one was prepared using the procedures described for Example 29 by replacing Intermediate 6(e) in Example 29 with Intermediate 6(a). $^1$H NMR (400 MHz, DMSO-d6); $\delta$ 9.04 (s, 1H), 8.68 (s, 1H), 8.54 (s, 1H), 7.38 (s, 2H), 6.26 (m, 1H), 4.27 (qr, 1H), 3.91 (m, 3H), 2.64 (s, 3H), 2.43 (m, 1H), 2.06 (m, 1H);MS (EI) for $C_{15}H_{15}N_5O_2S$: 330.03 $[MH]^+$. Analytical HPLC purity > 89%.

**Example 31:** 2-Amino-4-methyl-8-[(3*S*)-tetrahydrofuran-3-yl]-6-(1,3-thiazol-5-yl)pyrido[2,3-*d*]pyrimidin-7(8*H*)-one was prepared using the procedures described for Example 29 by replacing Intermediate 6(e) in Example 29 with Intermediate 6(c). $^1$H NMR (400 MHz, DMSO-d6); $\delta$ 9.05 (s, 1H), 8.62 (s, 1H), 8.56 (s, 1H), 7.41 (br, 2H), 6.28 (m, 1H), 4.30 (q, 1H), 3.90 (m, 3H), 2.50 (s, 3H), 2.40 (m, 1H), 2.10(m, 1H). MS (EI) for $C_{15}H_{15}N_5O_2S$: 330.1. Analytical HPLC > 87% purity.

**Example 32:** 2-Amino-4-methyl-8-[(3*R*)-tetrahydrofuran-3-yl]-6-(1,3-thiazol-5-yl)pyrido[2,3-*d*]pyrimidin-7(8*H*)-one (6.1 mg, 7.5% yield) was prepared using the procedures described for Example 29 by replacing Intermediate 6(e) in Example 29 with Intermediate 6(b). $^1$H NMR (400 MHz, d6-DMSO): 9.03 (s, 1H), 8.66 (s, 1H), 8.54 (s, 1H), 7.38 (br s, 2H), 6.31-6.21 (m, 1H), 4.28 (q, 1H), 3.99-3.84 (m, 3H), 2.64 (s, 3H), 2.44-2.38 (m, 1H), 2.14-2.03 (m, 1H); MS (EI) for $C_{15}H_{15}N_5O_2S$: 330 $(MH^+)$.

**Example 33:** 2-amino-4-methyl-8-(tetrahydro-2*H*-pyran-4-yl)-6-(1,3-thiazol-5-yl)pyrido[2,3-*d*]pyrimidin-7(8*H*)-one was prepared using the procedures described for Example 29 by replacing Intermediate 6(e) in Example 29 with Intermediate 6(d). $^1$H NMR (400 MHz, DMSO-d6); $\delta$ 9.03 (s, 1H), 8.62 (s, 1H), 8.56 (s, 1H), 7.39 (br, 2H), 5.78 (m, 1H), 4.02 (dd, 2H), 3.41 (t, 2H), 2.98 (m, 2H), 2.61 (s, 3H), 1.50 (brd, 2H). MS (EI) for $C_{16}H_{17}N_5O_2S$: 344.2. Analytical HPLC > 95% purity.

**Example 34:** 2-Amino-4-methyl-8-[(3*S*)-tetrahydro-2*H*-pyran-3-yl]-6-(1,3-thiazol-5-yl)pyrido[2,3-*d*]pyrimidin-7(8*H*)-one can be prepared using the procedures described above for Example 29 by replacing Intermediate 6(e) in Example 29 with Intermediate 6(f).

**Example 35:** 2-amino-4-methyl-8-[(3*R*)-tetrahydro-2*H*-pyran-3-yl]-6-(1,3-thiazol-5-yl)pyrido[2,3-*d*]pyrimidin-7(8*H*)-one can be prepared using the procedures described above for Example 29 by replacing Intermediate 6(e) in Example 29 with Intermediate 6(g).

**Biological Examples**

Biological Example 1

**PI3Kalpha Luciferase-Coupled Chemiluminescence Assay Protocol**

PI3Kα activity was measured as the percent of ATP consumed following the kinase reaction using luciferase-luciferi*N*-coupled chemiluminescence. Reactions were conducted in 384-well white, medium binding microtiter plates (Greiner). Kinase reactions were initiated by combining test compounds, ATP, substrate (PIP2), and kinase in a 20 μL volume in a buffer solution. The standard PI3Kalpha assay buffer was composed 50 mM Tris, pH 7.5, 1 mM EGTA, 10 mM MgCl, 1 mM DTT and 0.03% CHAPS. The standard assay concentrations for enzyme, ATP, and substrate were 0.5-1.1 nM, 1 μM, and 7.5 μM, respectively. The reaction mixture was incubated at ambient temperature for approximately 2 h. Following the kinase reaction, a 10 μL aliquot of luciferase-luciferin mix (Promega Kinase-Glo) was added and the chemiluminescence signal measured using a Victor2 plate reader (Perkin Elmer). Total ATP consumption was limited to 40-60% and IC50 values of control compounds correlate well with literature references.

Compounds of the invention were tested in this assay and demonstrated the ability to bind to PI3K. In particular, Compounds of the invention demonstrated a PI3K-binding affinity of about 0.06 μM or less; a subset of these Compounds demonstrated a PI3K-binding affinity of about 0.05 μM or less; a subset of these Compounds demonstrated a PI3K-binding affinity of about 0.04 μM or less; a subset of these Compounds demonstrated a PI3K-binding affinity of about 0.03 μM or less; a subset of these Compounds demonstrated a PI3K-binding affinity of about 0.02 μM or less; a subset of these Compounds demonstrated a PI3K-binding affinity of about 0.01 μM or less.

In one embodiment of the invention, the PI3K inhibitor is selected from the compounds in Table 1 having a PI3K-binding affinity of about 0.1 μM or less. In another embodiment, the PI3K inhibitor is selected from the compounds in Table 1 having a PI3K-binding affinity of about 0.06 μM or less. In another embodiment, the PI3K inhibitor is selected from the compounds in Table 1 having a PI3K-binding affinity of about 0.05 μM or less. In another embodiment, the PI3K inhibitor is selected from the compounds in Table 1 having a PI3K-binding affinity of about 0.04 μM or less. In another embodiment, the PI3K inhibitor is selected from the compounds in Table 1 having a PI3K-binding affinity of about 0.03 μM or less. In another embodiment, the PI3K inhibitor is selected from the compounds in Table 1 having a PI3K-binding affinity of about 0.02 μM or less. In another embodiment, the PI3K inhibitor is selected from the compounds in Table 1 having a PI3K-binding affinity of about 0.01 μM or less.

**Biological Example 2**

Phospho AKT assayPC-3 cells were seeded in 96-well plates at 24,000 cells/well. Cells were cultured for 2 days, then treated with compounds in serum-free medium for 3 hr. EGF (100 ng/mL) was added for the last 10 min. Cells were lysed in RIPA buffer. Phospho T308 Akt (Cell Signaling Technology, 7144) and total Aktl (Cell Signaling Technology, 7142) were quantified by ELISA performed according to the manufacturer's protocol. The readings of phospho Akt were normalized to total Akt readings.(RIPA: 50mM Tris, pH 7.5, 150 mM NaCl, 1% Triton X-100, 0.5% sodium deoxycholate, 0.1% SDS, 1mM EDTA, 1mM Na3V04, 1mM NaF, 1 mM β glycerol phosphate, Complete protease inhibitors (Roche, 11 873 580 001), and phosphatase inhibitor cocktail 1 (Sigma, P2850).

**Biological Example 3**

Phospho S6 assay

PC3 cells were seeded on 96-well plates at 8,000 cells/well. For each experiment, cells were seeded and treated in duplicated plates: one plate for phospho S6 CellELISA, and one plate for total S6 CellELISA. Cells were cultured on the plates for 3 days, then treated with compounds in serum-free medium for 3 hr in triplicate. Cells were fixed with 4% formaldehyde, quenched with 0.6% $H_2O$, blocked with 5% BSA, incubated with either phospho S6 antibody or total S6 antibody overnight, incubated with goat-anti-rabbit-IgG-HRP for 1 hr, and developed in chemiluminescent substrate.

**Biological Example 4**

PIP assay

MCF-7 cells grown in 10-cm dishes are starved for 3 hours in DMEM, and then treated with compounds for 20 minutes. In the last 2 minutes of the incubation with the compounds, EGF (100 ng/mL) is added to stimulate the production of PIP3. The medium is aspirated and the cells are scraped with 10% trichloroacetic acid. The lipids are extracted from the pellet after the cell lysates are centrifuged. PIP3 in the cellular lipid extraction is quantified with the AlphaScreen assay in which Grpl-PH is used as the PIP3 specific probe. The amount of cellular PIP3 is calculated from the standard curve of diC PI (3,4,5) P3.

**Biological Example 5-10**

In vivo models

Female and male athymic nude mice (NCr) 5-8 weeks of age and weighing approximately 20 g are used in the following model. Prior to initiation of a study, the animals are allowed to acclimate for a minimum of 48 h. During these studies, animals are provided food and water ad libitum and housed in a room conditioned at 70-75°F and 60% relative humidity. A 12 h light and 12 h dark cycle is maintained with automatic timers. All animals are examined daily for compound-induced or tumor-related deaths.

PC-3 human prostate adenocarcinoma cells are cultured in vitro in DMEM (Mediatech) supplemented with 20% Fetal Bovine Serum (Hyclone), Penicillin-Streptomycin and non-essential amino acids at 37°C in a humidified 5% CO atmosphere. On day 0, cells are harvested by trypsinization and $3x10^6$ cells (passage 13,99% viability) in 0.1 mL of ice-cold Hank's balanced salt solution are implanted subcutaneously into the hindflank of 5-8 week old male nude mice. A transponder is implanted in each mouse for identification, and animals are monitored daily for clinical symptoms and survival. Body weights are recorded daily.

U-87 MG human glioblastoma cells are cultured in vitro in DMEM (Mediatech) supplemented with 10% Fetal Bovine Serum (Hyclone), Penicillin-Streptomycin and non-essential amino acids at 37°C in a humidified 5% CO atmosphere. On day 0, cells are harvested by trypsinization and $2x10^6$ cells (passage 5, 96% viability) in 0.1 mL of ice-cold Hank's balanced salt solution are implanted intradermally into the hindflank of 5-8 week old female nude mice. A transponder is implanted in each mouse for identification, and animals are monitored daily for clinical symptoms and survival. Body weights are recorded daily.

A549 human lung carcinoma cells are cultured in vitro in DMEM (Mediatech) supplemented with 10% Fetal Bovine Serum (Hyclone), Penicillin-Streptomycin and non-essential amino acids at 37°C in a humidified 5% CO atmosphere. On day 0, cells are harvested by trypsinization and $10x10^6$ cells (passage 12,99% viability) in 0.1 mL of ice-cold Hank's balanced salt solution are implanted intradermally into the hindflank of 5-8 week old female nude mice. A transponder is implanted in each mouse for identification, and animals are monitored daily for clinical symptoms and survival. Body weights are recorded daily.

MDA-MB-468 human breast adenocarcinoma cells, passage number <6, are maintained and propagated in log-phase growth in Dulbecco's Modification of Eagles's Medium (DMEM; Mediatech) containing L-Glutamine supplemented with 10% Fetal Bovine Serum (Hyclone), Penicillin-Streptomycin and non-essential amino acids at 37 °C in a humidified, 5% CO atmosphere. On day 0, cells are harvested by trypsinization, and $10 \times 10^6$ cells (passage 10, 98% viability) in 50% cold Hanks balanced salt solution/50% Matrigel (100 μL total volume per mouse) are implanted subcutaneously into the mammary fat pads of female nude mice.

Calu-6 human lung anaplastic carcinoma cells are cultured *in vitro* in DMEM (Mediatech) supplemented with 10% Fetal Bovine Serum (Hyclone), Penicillin-Streptomycin and non-essential amino acids at 37 °C in a humidified, 5% CO atmosphere. On day 0, cells are harvested by trypsinization, and $5x10^6$ cells (passage #8, 96% viability) in 0.1 mL ice-cold Hank's balanced salt solution are implanted intradermally in the hind-flank of 5-8 week old female athymic nude mice. A transponder is implanted in each mouse for identification, and animals are monitored daily for clinical symptoms and survival. Body weights are recorded daily.

For subcutaneous or intradermal tumors, the mean tumor weight of each animal in the respective control and treatment groups is determined twice weekly during the study. Tumor weight (TW) is determined by measuring perpendicular diameters with a caliper, using the following formula:

$$\text{tumor weight (mg)} = [\text{tumor volume} = \text{length (mm)} \times \text{width}^2\ (\text{mm}^2)]/2$$

These data are recorded and plotted on a tumor weight vs. days post-implantation line graph and presented graphically as an indication of tumor growth rates. Percent inhibition of tumor growth (TGI) is determined with the following formula:

$$\left[1-\left(\frac{(X_f - X_0)}{(Y_f - X_0)}\right)\right]*100$$

where $X_0$ = average TW of all tumors on day 0

$X_f$= TW of treated group on Day f

$Y_f$= TW of vehicle control group on Day f

If tumors regress below their starting sizes, then the percent tumor regression is determined with the following formula:

$$\left[1-\left(\frac{(X_0 - X_f)}{X_0}\right)\right]*100$$

Tumor size is calculated individually for each tumor to obtain a mean ± SEM value for each experimental group. Statistical significance is determined using the 2-tailed Student's t-test (significance defined as P<0.05).

The foregoing invention has been described in some detail by way of illustration and example, for purposes of clarity and understanding. The invention has been described with reference to various specific embodiments and techniques. However, it should be understood that many variations and modifications may be made while remaining within the scope of the invention. It will be obvious to one of skill in the art that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims, along with the full scope of equivalents to which such claims are entitled.

## Claims

1. A Compound of Formula I

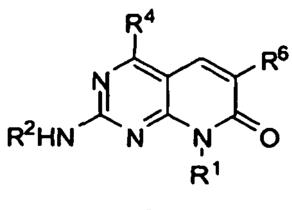

I

optionally as a pharmaceutically acceptable salt, additionally optionally as a solvate, and additionally optionally as a hydrate thereof, where
$R^1$ is 5- or 6- membered heterocycloalkyl;
$R^2$ is hydrogen or alkyl;
$R^4$ is alkyl;
$R^6$ is 5- or 6-membered heteroaryl optionally substituted with 1, 2, 3, 4, or 5 $R^9$ groups;
each $R^9$, when present, is independently halo, alkyl, haloalkyl, alkoxy, haloalkoxy, cyano, amino, alkylamino, dialkylamino, alkoxyalkyl, carboxyalkyl, alkoxycarbonyl, aminoalkyl, cycloalkyl, aryl, arylalkyl, aryloxy, heterocycloalkyl, or heteroaryl and where the cycloalkyl, aryl, heterocycloalkyl, and heteroaryl, each either alone or as part of another group within $R^9$, are independently optionally substituted with 1, 2, 3, or 4 groups selected from halo,

alkyl, haloalkyl, hydroxy, alkoxy, haloalkoxy, amino, alkylamino, and dialkylamino.

**2.** The Compound of Claim 1, wherein $R^1$ is a 5- or 6-membered heterocycloalkyl where the heterocycloalkyl comprises one heteroatom which is -O-; where the Compound is optionally as a pharmaceutically acceptable salt, additionally optionally as a solvate, and additionally optionally as a hydrate thereof.

**3.** The Compound of Claim 1 or 2, where $R^6$ is a 5-membered heteroaryl optionally substituted with 1, 2, or 3 $R^9$ groups; where the Compound is optionally as a pharmaceutically acceptable salt, additionally optionally as a solvate, and additionally optionally as a hydrate thereof.

**4.** The Compound of Claim I or 2, wherein $R^6$ is pyrazolyl, imidazolyl, thienyl, furanyl, pyrrolyl, or thiazolyl, each of which is optionally substituted with 1, 2, or 3 $R^9$ groups;
where the Compound is optionally as a pharmaceutically acceptable salt, additionally optionally as a solvate, and additionally optionally as a hydrate thereof.

**5.** The Compound of Claim 1 or 2, wherein $R^6$ is pyrazolyl, imidazolyl, or thiazolyl, each of which is optionally substituted with 1, 2, or 3 $R^9$ groups; where the Compound is optionally as a pharmaceutically acceptable salt, additionally optionally as a solvate, and additionally optionally as a hydrate thereof.

**6.** The Compound of Claim 1 or 2, wherein $R^6$ is a 6-membered heteroaryl optionally substituted with 1, 2, or 3 $R^9$ groups; where the Compound is optionally as a pharmaceutically acceptable salt, additionally optionally as a solvate, and additionally optionally as a hydrate thereof.

**7.** The Compound of Claims 1, 2, 3, 4, 5 or 6, where $R^2$ is hydrogen; where the Compound is optionally as a pharmaceutically acceptable salt, additionally optionally as a solvate, and additionally optionally as a hydrate thereof.

**8.** The Compound of Claims 1, 2, 3, 4, 5 or 6, where $R^2$ is ethyl or methyl; where the Compound is optionally as a pharmaceutically acceptable salt, additionally optionally as a solvate, and additionally optionally as a hydrate thereof.

**9.** The Compound of Claims 1, 2, 3, 4, 5, 6, 7 or 8, where $R^4$ is methyl; where the Compound is optionally as a pharmaceutically acceptable salt, additionally optionally as a solvate, and additionally optionally as a hydrate thereof.

**10.** The Compound of Claim 1 selected from

| **Name** |
|---|
| 2-amino-4-methyl-6-(1*H*-pyrazol-5-yl)-8-(tetrahydrofuran-3-yl)pyrido[2,3-*d*]pyrimidin-7(8 *H*)-one; |
| 2-amino-4-methyl-6-(1*H*-pyrazol-5-yl)-8-[(3*S*)-tetrahydrofuran-3-yl]pyrido[2,3-*d*]pyrimidin -7(8*H*)-one; |
| 2-amino-4-methyl-6-(1*H*-pyrazol-5-yl)-8-[(3*R*)-tetrahydrofuran-3-yl]pyrido[2,3-*d*]pyrimidin -7(8*H*)-one; |
| 2-amino-4-methyl-6-(1*H*-pyrazol-5-yl)-8-(tetrahydro-2*H*-pyran-4-yl)pyrido[2,3-*d*]pyrimidin -7(8*H*)-one; |
| 2-amino-4-methyl-6-(1*H*-pyrazol-5-yl)-8-(tetrahydro-2*H*-pyran-3-yl)pyrido[2,3-*d*]pyrimidin -7(8*H*)-one; |
| 2-amino-4-methyl-6-(1*H*-pyrazol-5-yl)-8-[(3*S*)-tetrahydro-2*H*-pyran-3-yl]pyrido[2,3-*d*]pyri midin-7(8*H*)-one; |
| 2-amino-4-methyl-6-(1*H*-pyrazol-5-yl)-8-[(3*R*)-tetrahydro-2*H*-pyran-3-yl]pyrido[2,3-*d*]pyri midin-7(8*H*)-one; |
| 2-amino-4-methyl-8-(tetrahydrofuran-3-yl)-6-(1,3-thiazol-2-yl)pyrido[2,3-*d*]pyrimidin-7(8 *H*)-one; |
| 2-amino-4-methyl-8-[(3*S*)-tetrahydrofuran-3-yl]-6-(1,3-thiazol-2-yl)pyrido[2,3-*d*]pyrimidin-7(8*H*)-one; |
| 2-amino-4-methyl-8-[(3*R*)-tetrahydrofuran-3-yl]-6-(1,3-thiazol-2-yl)pyrido[2,3-*d*]pyrimidin -7(8*H*)-one; |
| 2-amino-4-methyl-8-(tetrahydro-2*H*-pyran-4-yl)-6-(1,3-thiazol-2-yl)pyrido[2,3-*d*]pyrimidin -7(8*H*)-one; |
| 2-amino-4-methyl-8-(tetrahydro-2*H*-pyran-3-yl)-6-(1,3-thiazo(-2-yl)pyrido[2,3-*d*]pyrimidin -7(8*H*)-one; |
| 2-amino-4-methyl-8-[(3*S*)-tetrahydro-2*H*-pyran-3-yl]-6-(1,3-thiazol-2-yl)pyrido[2,3-*d*]pyri midin-7(8*H*)-one; |
| 2-amino-4-methyl-8-[(3*R*)-tetrahydro-2*H*-pyran-3-yl]-6-(1,3-thiazol-2-yl)pyrido[2,3-*d*]pyri midin-7(8*H*)-one; |
| 2-amino-6-(1*H*-imidazol-2-yl)-4-methyl-8-(tetrahydrofuran-3-yl)pyrido[2,3-*d*]pyrimidin-7( 8*H*)-one; |

(continued)

| Name |
|---|
| 2-amino-6-(1*H*-imidazol-2-yl)-4-methyl-8-[(3*S*)-tetrahydrofuran-3-yl]pyrido[2,3-*d*]pyrimidi n-7(8*H*)-one; |
| 2-amino-6-(1*H*-imidazol-2-yl)-4-methyl-8-[(3*R*)-tetrahydrofuran-3-yl]pyrido[2,3-*d*]pyrimidi n-7(8*H*)-one; |
| 2-amino-6-(1*H*-imidazol-2-yl)-4-methyl-8-(tetrahydro-2*H*-pyran-4-yl)pyrido[2,3-*d*]pyrimidi n-7(8*H*)-one; |
| 2-amino-6-(1*H*-imidazol-2-yl)-4-methyl-8-(tetrahydro-2*H*-pyran-3-yl)pyrido[2,3-*d*]pyrimidi n-7(8*H*)-one; |
| 2-aminl-6-(1*H*-imidazol-2-yl)-4-methyl-8-[(3*S*)-tetrahydro-2*H*-pyran-3-yl]pyrido[2,3-*d*]pyr imidin-7(8*H*)-one; |
| 2-amino-6-(1*H*-imidazol-2-yl)-4-methyl-8-[(3*R*)-tetrahydro-2*H*-pyran-3-yl]pyrido[2,3-*d*]pyr imidin-7(8*H*)-one; |
| 2-amino-4-methyl-8-(tetrahydrofuran-3-yl)-6-(1,3-thiazol-5-yl)pyrido[2,3-*d*]pyrimidin-7(8 *H*)-one; |
| 2-amino-4-methyl-8-[(3*S*)-tetrahydrofuran-3-yl]-6-(1,3-thiazol-5-yl)pyrido[2,3-*d*]pyrimidin-7(8*H*)-one; |
| 2-amino-4-methyl-8-[(3*R*)-tetrahydrofuran-3-yl]-6-(1,3-thiazol-5-yl)pyrido[2,3-*d*]pyrimidin -7(8*H*)-one; |
| 2-amino-4-methyl-8-(tetrahydro-2*H*-pyran-4-yl)-6-(1,3-thiazol-5-yl)pyrido[2,3-*d*]pyrimidin -7(8*H*)-one; |
| 2-amino-4-methyl-8-(tetrahydro-2*H*-pyran-3-yl)-6-(1,3-thiazol-5-yl)pyrido[2,3-*d*]pyrimidin -7(8*H*)-one; |
| 2-amino-4-methyl-8-[(3*S*]-tetrahydro-2*H*-pyran-3-yl]-6-(1,3-thiazol-5-yl)pyrido[2,3-*d*]pyri midin-7(8*H*)-one; and |
| 2-amino-4-methyl-8-[(3*R*)-tetrahydro-2*H*-pyran-3-yl]-6-(1,3-thiazol-5-yl)pyrido[2,3-*d*]pyri midin-7(8*H*)-one |

where the Compound is optionally as a pharmaceutically acceptable salt, additionally optionally as a solvate, and additionally optionally as a hydrate thereof.

**11.** A pharmaceutical composition which comprises a Compound of any one of Claims 1 to 10, and a pharmaceutically acceptable carrier, excipient, or diluent; where the Compound is optionally as a pharmaceutically acceptable salt, additionally optionally as a solvate, and additionally optionally as a hydrate thereof.

**12.** A Compound of any one of Claims 1 to 10, where the Compound is optionally as a pharmaceutically acceptable salt, additionally optionally as a solvate, and additionally optionally as a hydrate thereof, for use in medicine.

**13.** A Compound of any one of Claims 1 to 10, for use in the treatment of cancer, where the Compound is optionally as a pharmaceutically acceptable salt, additionally optionally as a solvate, and additionally optionally as a hydrate thereof.

**14.** The Compound of Claim 13 where the cancer is breast cancer, colon cancer, rectal cancer, endometrial cancer, gastric carcinoma, glioblastoma, hepatocellular carcinoma, small cell lung cancer, non-small cell lung cancer, melanoma, ovarian cancer, cervical cancer, pancreatic cancer, prostate carcinoma, acute myelogenous leukemia (AML), chronic myelogenous leukemia (CML), non-Hodgkin's lymphoma, or thyroid carcinoma.

**15.** A Compound of Claim 13, where the Compound is optionally as a pharmaceutically acceptable salt, additionally optionally as a solvate, and additionally optionally as a hydrate thereof, wherein said use is in combination with one or more treatments selected from surgery, one or more chemotherapeutic agents, one or more hormone therapies, one or more antibodies, one or more immunotherapies, radioactive iodine therapy, and radiation.

**16.** A method of preparing a Compound of Claim 1, comprising:

(a) reacting an intermediate of formula 1

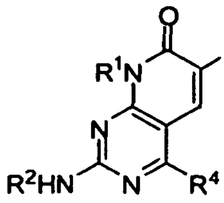

1

where $R^1$, $R^2$, and $R^4$ are as defined in Claim 1, with an intermediate of formula $R^6Sn(n\text{-}Bu)_3$ or $R^6B(OH)_2$ where $R^6$ and $R^9$ are as defined in Claim 1 to yield a Compound of Claim 1;
(b) optionally further modifying one of the $R^1$, $R^2$, $R^4$, and $R^6$ groups; and
(c) optionally forming a pharmaceutically acceptable salt, solvate, and/or hydrate thereof.

**Patentansprüche**

1. Eine Verbindung der Formel I

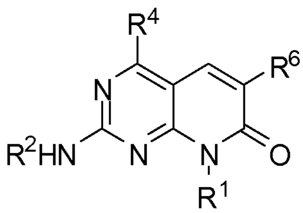

I

optional als ein pharmazeutisch akzeptables Salz, zusätzlich optional als ein Solvat, und zusätzlich optional als ein Hydrat davon, wobei
$R^1$ ein 5- oder 6-gliedriges Heterocycloalkyl ist;
$R^2$ Wasserstoff oder Alkyl ist;
$R^4$ Alkyl ist;
$R^6$ ein 5- oder 6-gliedriges Heteroaryl ist, das optional mit 1, 2, 3, 4 oder 5 $R^9$-Gruppen substituiert ist;
jedes $R^9$, falls vorhanden, unabhängig Halogen, Alkyl, Halogenalkyl, Alkoxy, Halogenalkoxy, Cyan, Amino, Alkylamino, Dialkylamino, Alkoxyalkyl, Carboxyalkyl, Alkoxycarbonyl, Aminoalkyl, Cycloalkyl, Aryl, Arylalkyl, Aryloxy, Heterocycloalkyl oder Heteroaryl ist und wobei das Cycloalkyl, Aryl, Heterocycloalkyl und Heteroaryl, jeweils entweder allein oder als Teil einer anderen Gruppe innerhalb von $R^9$, unabhängig optional mit 1, 2, 3 oder 4 Gruppen substituiert sind, ausgewählt aus Halogen, Alkyl, Halogenalkyl, Hydroxy, Alkoxy, Halogenalkoxy, Amino, Alkylamino und Dialkylamino.

2. Verbindung gemäß Anspruch 1, wobei $R^1$ ein 5- oder 6-gliedriges Heterocycloalkyl ist, wobei das Heterocycloalkyl ein Heteroatom beinhaltet, das -O- ist; wobei die Verbindung optional als ein pharmazeutisch akzeptables Salz, zusätzlich optional als ein Solvat und zusätzlich optional als ein Hydrat davon vorliegt.

3. Verbindung gemäß Anspruch 1 oder 2, wobei $R^6$ ein 5-gliedriges Heteroaryl ist, das optional mit 1, 2 oder 3 $R^9$-Gruppen substituiert ist; wobei die Verbindung optional als ein pharmazeutisch akzeptables Salz, zusätzlich optional als ein Solvat und zusätzlich optional als ein Hydrat davon vorliegt.

4. Verbindung gemäß Anspruch 1 oder 2, wobei $R^6$ Pyrazolyl, Imidazolyl, Thienyl, Furanyl, Pyrrolyl oder Thiazolyl ist, von denen jedes optional mit 1, 2 oder 3 $R^9$-Gruppen substituiert ist;
wobei die Verbindung optional als ein pharmazeutisch akzeptables Salz, zusätzlich optional als ein Solvat und zusätzlich optional als ein Hydrat davon vorliegt.

5. Verbindung gemäß Anspruch 1 oder 2, wobei $R^6$ Pyrazolyl, Imidazolyl oder Thiazolyl ist, von denen jedes optional mit 1, 2 oder 3 $R^9$-Gruppen substituiert ist; wobei die Verbindung optional als ein pharmazeutisch akzeptables Salz, zusätzlich optional als ein Solvat und zusätzlich optional als ein Hydrat davon vorliegt.

**6.** Verbindung gemäß Anspruch 1 oder 2, wobei $R^6$ ein 6-gliedriges Heteroaryl ist, das optional mit 1, 2 oder 3 $R^9$-Gruppen substituiert ist; wobei die Verbindung optional als ein pharmazeutisch akzeptables Salz, zusätzlich optional als ein Solvat und zusätzlich optional als ein Hydrat davon vorliegt.

**7.** Verbindung gemäß Anspruch 1, 2, 3, 4, 5 oder 6, wobei $R^2$ Wasserstoff ist; wobei die Verbindung optional als ein pharmazeutisch akzeptables Salz, zusätzlich optional als ein Solvat und zusätzlich optional als ein Hydrat davon ist vorliegt.

**8.** Verbindung gemäß Anspruch 1, 2, 3, 4, 5 oder 6, wobei $R^2$ Ethyl oder Methyl ist; wobei die Verbindung optional als ein pharmazeutisch akzeptables Salz, zusätzlich optional als ein Solvat und zusätzlich optional als ein Hydrat davon vorliegt.

**9.** Verbindung gemäß Anspruch 1, 2, 3, 4, 5, 6, 7 oder 8, wobei $R^4$ Methyl ist; wobei die Verbindung optional als ein pharmazeutisch akzeptables Salz, zusätzlich optional als ein Solvat und zusätzlich optional als ein Hydrat davon vorliegt.

**10.** Verbindung gemäß Anspruch 1, ausgewählt aus

| Name |
|---|
| 2-Amino-4-methyl-6-(1*H*-pyrazol-5-yl)-8-(tetrahydrofuran-3-yl)pyrido[2,3-*d*]pyrimidin-7(8*H*)-on; |
| 2-Amino-4-methyl-6-(1*H*-pyrazol-5-yl)-8-[(3*S*)-tetrahydrofuran-3-yl]pyrido[2,3-*d*]pyrimidin-7(8*H*)-on; |
| 2-Amino-4-methyl-6-(1*H*-pyrazol-5-yl)-8-[(3*R*)-tetrahydrofuran-3-yl]pyrido[2,3-*d*]pyrimidin-7(8*H*)-on; |
| 2-Amino-4-methyl-6-(1*H*-pyrazol-5-yl)-8-(tetrahydro-2*H*-pyran-4-yl)pyrido[2,3-*d*]pyrimidin-7(8*H*)-on; |
| 2-Amino-4-methyl-6-(1*H*-pyrazol-5-yl)-8-(tetrahydro-2*H*-pyran-3-yl)pyrido[2,3-*d*]pyrimidin-7(8*H*)-on; |
| 2-Amino-4-methyl-6-(1 H-pyrazol-5-yl)-8-[(3S)-tetrahydro-2H-pyran-3-yl]pyrido[2,3-*d*]pyrimidin-7(8*H*)-on; |
| 2-Amino-4-methyl-6-(1*H*-pyrazol-5-yl)-8-[(3*R*)-tetrahydro-2*H*-pyran-3-yl]pyrido[2,3-*d*]pyrimidin-7(8*H*)-on; |
| 2-Amino-4-methyl-8-(tetrahydrofuran-3-yl)-6-(1,3-thiazol-2-yl)pyrido[2,3-*d*]pyrimidin-7(8*H*)-on; |
| 2-Amino-4-methyl-8-[(3*S*)-tetrahydrofuran-3-yl]-6-(1,3-thiazol-2-yl)pyrido[2,3-*d*]pyrimidin-7(8*H*)-on; |
| 2-Amino-4-methyl-8-[(3*R*)-tetrahydrofuran-3-yl]-6-(1,3-thiazol-2-yl)pyrido[2,3-*d*]pyrimidin-7(8*H*)-on; |
| 2-Amino-4-methyl-8-(tetrahydro-2*H*-pyran-4-yl)-6-(1,3-thiazol-2-yl)pyrido[2,3-*d*]pyrimidin-7(8*H*)-on; |
| 2-Amino-4-methyl-8-(tetrahydro-2*H*-pyran-3-yl)-6-(1,3-thiazol-2-yl)pyrido[2,3-*d*]pyrimidin-7(8*H*)-on; |
| 2-Amino-4-methyl-8-[(3*S*)-tetrahydro-2*H*-pyran-3-yl]-6-(1,3-thiazol-2-yl)pyrido[2,3-*d*]pyrimidin-7(8*H*)-on; |
| 2-Amino-4-methyl-8-[(3R)-tetrahydro-2H-pyran-3-yl]-6-(1,3-thiazol-2-yl)pyrido[2,3-*d*]pyrimidin-7(8*H*)-on; |
| 2-Amino-6-(1H-imidazol-2-yl)-4-methyl-8-(tetrahydrofuran-3-yl)pyrido[2,3-*d*]pyrimidin-7(8*H*)-on; |
| 2-Amino-6-(1*H*-imidazol-2-yl)-4-methyl-8-[(3*S*)-tetrahydrofuran-3-yl]pyrido[2,3-*d*]pyrimidin-7(8*H*)-on; |
| 2-Amino-6-(1*H*-imidazol-2-yl)-4-methyl-8-[(3*R*)-tetrahydrofuran-3-yl]pyrido[2,3-*d*]pyrimidin-7(8*H*)-on; |
| 2-Amino-6-(1*H*-imidazol-2-yl)-4-methyl-8-(tetrahydro-2*H*-pyran-4-yl)pyrido[2,3-*d*]pyrimidin-7(8*H*)-on; |
| 2-Amino-6-(1*H*-imidazol-2-yl)-4-methyl-8-(tetrahydro-2*H*-pyran-3-yl)pyrido[2,3-*d*]pyrimidin-7(8*H*)-on; |
| 2-Amino-6-(1*H*-imidazol-2-yl)-4-methyl-8-[(3*S*)-tetrahydro-2*H*-pyran-3-yl]pyrido[2,3-*d*]pyrimidin-7(8*H*)-on; |
| 2-Amino-6-(1*H*-imidazol-2-yl)-4-methyl-8-[(3*R*)-tetrahydro-2*H*-pyran-3-yl]pyrido[2,3-*d*]pyrimidin-7(8*H*)-on; |
| 2-Amino-4-methyl-8-(tetrahydrofuran-3-yl)-6-(1,3-thiazol-5-yl)pyrido[2,3-*d*]pyrimidin-7(8*H*)-on; |
| 2-Amino-4-methyl-8-[(3*S*)-tetrahydrofuran-3-yl]-6-(1,3-thiazol-5-yl)pyrido[2,3-*d*]pyrimidin-7(8*H*)-on; |
| 2-Amino-4-methyl-8-[(3*R*)-tetrahydrofuran-3-yl]-6-(1,3-thiazol-5-yl)pyrido[2,3-*d*]pyrimidin-7(8*H*)-on; |
| 2-Amino-4-methyl-8-(tetrahydro-2*H*-pyran-4-yl)-6-(1,3-thiazol-5-yl)pyrido[2,3-*d*]pyrimidin-7(8*H*)-on; |
| 2-Amino-4-methyl-8-(tetrahydro-2*H*-pyran-3-yl)-6-(1,3-thiazol-5-yl)pyrido[2,3-*d*]pyrimidin-7(8*H*)-on; |

(fortgesetzt)

| Name |
|---|
| 2-Amino-4-methyl-8-[(3*S*)-tetrahydro-2*H*-pyran-3-yl]-6-(1,3-thiazol-5-yl)pyrido[2,3-*d*]pyrimidin-7(8*H*)-on; und |
| 2-Amino-4-methyl-8-[(3R)-tetrahydro-2H-pyran-3-yl]-6-(1,3-thiazol-5-yl)pyrido[2,3-*d*]pyrimidin-7(8*H*)-on |

wobei die Verbindung optional als ein pharmazeutisch akzeptables Salz, zusätzlich optional als ein Solvat und zusätzlich optional als ein Hydrat davon vorliegt.

**11.** Eine pharmazeutische Zusammensetzung, die eine Verbindung gemäß einem der Ansprüche 1 bis 10 und einen pharmazeutisch akzeptablen Träger, einen pharmazeutisch akzeptablen Hilfsstoff oder ein pharmazeutisch akzeptables Verdünnungsmittel beinhaltet; wobei die Verbindung optional als ein pharmazeutisch akzeptables Salz, zusätzlich optional als ein Solvat und zusätzlich optional als ein Hydrat davon vorliegt.

**12.** Verbindung gemäß einem der Ansprüche 1 bis 10, wobei die Verbindung optional als ein pharmazeutisch akzeptables Salz, zusätzlich optional als ein Solvat und zusätzlich optional als ein Hydrat davon vorliegt, zur Verwendung in der Medizin.

**13.** Verbindung gemäß einem der Ansprüche 1 bis 10, zur Verwendung in (bei) der Behandlung von Krebs, wobei die Verbindung optional als ein pharmazeutisch akzeptables Salz, zusätzlich optional als ein Solvat und zusätzlich optional als ein Hydrat davon vorliegt.

**14.** Verbindung gemäß Anspruch 13, wobei der Krebs Brustkrebs, Kolonkrebs, Rektalkrebs, Endometriumkrebs, Magenkarzinom, Glioblastom, Leberzellkarzinom, kleinzelliger Lungenkrebs, nicht-kleinzelliger Lungenkrebs, Melanom, Eierstockkrebs, Gebärmutterhalskrebs, Bauchspeicheldrüsenkrebs, Prostatakrebs, akute myeloische Leukämie (AML), chronische myeloische Leukämie (CML), Non-Hodgkin-Lymphom oder Schilddüsenkarzinom ist.

**15.** Verbindung gemäß Anspruch 13, wobei die Verbindung optional als ein pharmazeutisch akzeptables Salz, zusätzlich optional als ein Solvat und zusätzlich optional als ein Hydrat davon vorliegt, wobei die Verwendung in Kombination mit einer oder mehreren Behandlungen ist, ausgewählt aus Chirurgie, einem oder mehr chemotherapeutischen Mitteln, einer oder mehr Hormontherapien, einem oder mehr Antikörpern, einer oder mehr Immuntherapien, radioaktiver Jodtherapie und Bestrahlung.

**16.** Ein Verfahren zur Herstellung einer Verbindung gemäß Anspruch 1, beinhaltend:

(a) Zur-Reaktion-Bringen eines Intermediats der Formel 1

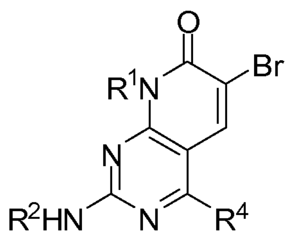

1

wobei $R^1$, $R^2$ und $R^4$ wie in Anspruch 1 definiert sind, mit einem Intermediat (mit) der Formel $R^6Sn(n\text{-}Bu)_3$ oder $R^6B(OH)_2$, wobei $R^6$ und $R^9$ wie in Anspruch 1 definiert sind, um eine Verbindung gemäß Anspruch 1 zu ergeben;
(b) optional weiteres Modifizieren von einer der $R^1$-, $R^2$-, $R^4$- und $R^6$-Gruppen; und
(c) optional Bilden eines pharmazeutisch akzeptablen Salzes, Solvats und/oder Hydrats davon.

**Revendications**

**1.** Un composé de Formule I

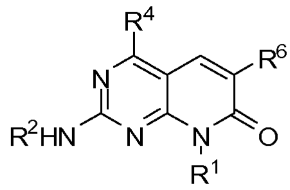

I

facultativement sous forme de sel pharmaceutiquement acceptable, facultativement encore sous forme de solvate, et facultativement encore sous forme d'hydrate de celui-ci, où
$R^1$ est un hétérocycloalkyle à 5 ou 6 membres ;
$R^2$ est hydrogène ou alkyle ;
$R^4$ est alkyle ;
$R^6$ est un hétéroaryle à 5 ou 6 membres facultativement substitué par 1, 2, 3, 4 ou 5 groupes $R^9$;
chaque $R^9$, lorsqu'il est présent, est indépendamment halo, alkyle, haloalkyle, alcoxy, haloalcoxy, cyano, amino, alkylamino, dialkylamino, alcoxyalkyle, carboxyalkyle, alcoxycarbonyle, aminoalkyle, cycloalkyle, aryle, arylalkyle, aryloxy, hétérocycloalkyle, ou hétéroaryle et où les cycloalkyle, aryle, hétérocycloalkyle, et hétéroaryle, chacun soit seul, soit faisant partie d'un autre groupe au sein de $R^9$, sont indépendamment facultativement substitués par 1, 2, 3, ou 4 groupes sélectionnés parmi halo, alkyle, haloalkyle, hydroxy, alcoxy, haloalcoxy, amino, alkylamino, et dialkylamino.

2. Le composé de la revendication 1, dans lequel $R^1$ est un hétérocycloalkyle à 5 ou 6 membres où l'hétérocycloalkyle comprend un hétéroatome qui est -O- ; où le composé est facultativement sous forme de sel pharmaceutiquement acceptable, facultativement encore sous forme de solvate, et facultativement encore sous forme d'hydrate de celui-ci.

3. Le composé de la revendication 1 ou de la revendication 2, où $R^6$ est un hétéroaryle à 5 membres facultativement substitué par 1, 2, ou 3 groupes $R^9$ ; où le composé est facultativement sous forme de sel pharmaceutiquement acceptable, facultativement encore sous forme de solvate, et facultativement encore sous forme d'hydrate de celui-ci.

4. Le composé de la revendication 1 ou de la revendication 2, dans lequel $R^6$ est pyrazolyle, imidazolyle, thiényle, furanyle, pyrrolyle, ou thiazolyle, chacun d'entre eux est facultativement substitué par 1, 2 ou 3 groupes $R^9$ ; où le composé est facultativement sous forme de sel pharmaceutiquement acceptable, facultativement encore sous forme de solvate, et facultativement encore sous forme d'hydrate de celui-ci.

5. Le composé de la revendication 1 ou de la revendication 2, dans lequel $R^6$ est pyrazolyle, imidazolyle, ou thiazolyle, chacun d'entre eux est facultativement substitué par 1, 2, ou 3 groupes $R^9$ ; où le composé est facultativement sous forme de sel pharmaceutiquement acceptable, facultativement encore sous forme de solvate, et facultativement encore sous forme d'hydrate de celui-ci.

6. Le composé de la revendication 1 ou de la revendication 2, dans lequel $R^6$ est un hétéroaryle à 6 membres facultativement substitué par 1, 2, ou 3 groupes $R^9$ ; où le composé est facultativement sous forme de sel pharmaceutiquement acceptable, facultativement encore sous forme de solvate, et facultativement encore sous forme d'hydrate de celui-ci.

7. Le composé des revendications 1, 2, 3, 4, 5 ou 6, où $R^2$ est hydrogène ; où le composé est facultativement sous forme de sel pharmaceutiquement acceptable, facultativement encore sous forme de solvate, et facultativement encore sous forme d'hydrate de celui-ci.

8. Le composé des revendications 1, 2, 3, 4, 5 ou 6, où $R^2$ est éthyle ou méthyle ; où le composé est facultativement sous forme de sel pharmaceutiquement acceptable, facultativement encore sous forme de solvate, et facultativement encore sous forme d'hydrate de celui-ci.

9. Le composé des revendications 1, 2, 3, 4, 5, 6, 7 ou 8, où $R^4$ est méthyle ; où le composé est facultativement sous forme de sel pharmaceutiquement acceptable, facultativement encore sous forme de solvate, et facultativement encore sous forme d'hydrate de celui-ci.

10. Le composé de la revendication 1 sélectionné parmi

| Nom |
| --- |
| 2-amino-4-méthyl-6-(1*H*-pyrazol-5-yl)-8-(tétrahydrofuran-3-yl)pyrido[2,3-*d*]pyrimidin-7(8*H*)-one ; |
| 2-amino-4-méthyl-6-(1*H*-pyrazol-5-yl)-8-[(3*S*)-tétrahydrofuran-3-yl]pyrido[2,3-*d*]pyrimidin-7 (8*H*)-one ; |
| 2-amino-4-méthyl-6-(1*H*-pyrazol-5-yl)-8-[(3*R*)-tétrahydrofuran-3-yl]pyrido[2,3-*d*]pyrimidin-7 (8*H*)-one ; |
| 2-amino-4-méthyl-6-(1*H*-pyrazol-5-yl)-8-(tétrahydro-2*H*-pyran-4-yl)pyrido[2,3-*d*]pyrimidin-7 (8*H*)-one ; |
| 2-amino-4-méthyl-6-(1*H*-pyrazol-5-yl)-8-(tétrahydro-2*H*-pyran-3-yl)pyrido[2,3-*d*]pyrimidin-7 (8*H*)-one ; |
| 2-amino-4-méthyl-6-(1*H*-pyrazol-5-yl)-8-[(3*S*)-tétrahydro-2*H*-pyran-3-yl]pyrido[2,3-*d*]pyrimi din-7(8*H*)-one ; |
| 2-amino-4-méthyl-6-(1*H*-pyrazol-5-yl)-8-[(3*R*)-tétrahydro-2H-pyran-3-yl]pyrido[2,3-*d*]pyrimi din-7(8*H*)-one ; |
| 2-amino-4-méthyl-8-(tétrahydrofuran-3-yl)-6-(1,3-thiazol-2-yl)pyrido[2,3-*d*]pyrimidin-7(8*H*)-one; |
| 2-amino-4-méthyl-8-[(3*S*)-tétrahydrofuran-3-yl]-6-(1,3-thiazol-2-yl)pyrido[2,3-*d*]pyrimidin-7(8*H*)-one ; |
| 2-amino-4-méthyl-8-[(3*R*)-tétrahydrofuran-3-yl]-6-(1,3-thiazol-2-yl)pyrido[2,3-*d*]pyrimidin-7(8*H*)-one ; |
| 2-amino-4-méthyl-8-(tétrahydro-2*H*-pyran-4-yl)-6-(1,3-thiazol-2-yl)pyrido[2,3-*d*]pyrimidin-7(8*H*)-one ; |
| 2-amino-4-méthyl-8-(tétrahydro-2*H*-pyran-3-yl)-6-(1,3-thiazol-2-yl)pyrido[2,3-*d*]pyrimidin-7(8*H*)-one ; |
| 2-amino-4-méthyl-8-[(3*S*)-tétrahydro-2*H*-pyran-3-yl]-6-(1,3-thiazol-2-yl)pyrido[2,3-*d*]pyrimi din-7(8*H*)-one ; |
| 2-amino-4-méthyl-8-[(3*R*)-tétrahydro-2*H*-pyran-3-yl]-6-(1,3-thiazol-2-yl)pyrido[2,3-*d*]pyrimi din-7(8*H*)-one ; |
| 2-amino-6-(1*H*-imidazol-2-yl)-4-méthyl-8-(tétrahydrofuran-3-yl)pyrido[2,3-*d*]pyrimidin-7(8*H*)-one ; |
| 2-amino-6-(1*H*-imidazol-2-yl)-4-méthyl-8-[(3*S*)-tétrahydrofuran-3-yl]pyrido[2,3-*d*]pyrimidin-7(8*H*)-one ; |
| 2-amino-6-(1*H*-imidazol-2-yl)-4-méthyl-8-[(3*R*)-tétrahydrofuran-3-yl]pyrido[2,3-*d*]pyrimidin-7(8*H*)-one ; |
| 2-amino-6-(1*H*-imidazol-2-yl)-4-méthyl-8-(tétrahydro-2*H*-pyran-4-yl)pyrido[2,3-*d*]pyrimidin-7(8*H*)-one ; |
| 2-amino-6-(1*H*-imidazol-2-yl)-4-méthyl-8-(tétrahydro-2*H*-pyran-3-yl)pyrido[2,3-*d*]pyrimidin-7(8*H*)-one ; |
| 2-amino-6-(1*H*-imidazol-2-yl)-4-méthyl-8-[(3*S*)-tétrahydro-2*H*-pyran-3-yl]pyrido[2,3-*d*]pyrim idin-7(8*H*)-one ; |
| 2-amino-6-(1*H*-imidazol-2-yl)-4-méthyl-8-[(3*R*)-tétrahydro-2*H*-pyran-3-yl]pyrido[2,3-*d*]pyrim idin-7(8*H*)-one ; |
| 2-amino-4-méthyl-8-(tétrahydrofuran-3-yl)-6-(1,3-thiazol-5-yl)pyrido[2,3-d]pyrimidin-7(8*H*)-one ; |
| 2-amino-4-méthyl-8-[(3*S*)-tétrahydrofuran-3-yl]-6-(1,3-thiazol-5-yl)pyrido[2,3-*d*]pyrimidin-7(8*H*)-one ; |
| 2-amino-4-méthyl-8-[(3*R*)-tétrahydrofuran-3-yl]-6-(1,3-thiazol-5-yl)pyrido[2,3-*d*]pyrimidin-7(8*H*)-one ; |
| 2-amino-4-méthyl-8-(tétrahydro-2*H*-pyran-4-yl)-6-(1,3-thiazol-5-yl)pyrido[2,3-d]pyrimidin-7(8*H*)-one ; |
| 2-amino-4-méthyl-8-(tétrahydro-2*H*-pyran-3-yl)-6-(1,3-thiazol-5-yl)pyrido[2,3-*d*]pyrimidin-7(8*H*)-one ; |
| 2-amino-4-méthyl-8-[(3*S*)-tétrahydro-2*H*-pyran-3-yl]-6-(1,3-thiazol-5-yl)pyrido[2,3-*d*]pyrimi din-7(8*H*)-one; et |
| 2-amino-4-méthyl-8-[(3R)-tétrahydro-2*H*-pyran-3-yl]-6-(1,3-thiazol-5-yl)pyrido[2,3-*d*]pyrimi din-7(8*H*)-one |

où le composé est facultativement sous forme de sel pharmaceutiquement acceptable, facultativement encore sous forme de solvate, et facultativement encore sous forme d'hydrate de celui-ci.

**11.** Une composition pharmaceutique qui comprend un composé de l'une quelconque des revendications 1 à 10, et un véhicule, excipient, ou diluant pharmaceutiquement acceptables ; où le composé est facultativement sous forme de sel pharmaceutiquement acceptable, facultativement encore sous forme de solvate, et facultativement encore sous forme d'hydrate de celui-ci.

**12.** Un composé de l'une quelconque des revendications 1 à 10, où le composé est facultativement sous forme de sel pharmaceutiquement acceptable, facultativement encore sous forme de solvate, et facultativement encore sous forme d'hydrate de celui-ci, destiné à être utilisé en médecine.

**13.** Un composé de l'une quelconque des revendications 1 à 10, destiné à être utilisé pour le traitement du cancer, où

le composé est facultativement sous forme de sel pharmaceutiquement acceptable, facultativement encore sous forme de solvate, et facultativement encore sous forme d'hydrate de celui-ci.

**14.** Le composé de la revendication 13 où le cancer est un cancer du sein, un cancer du côlon, un cancer du rectum, un cancer de l'endomètre, un carcinome de l'estomac, un glioblastome, un carcinome hépatocellulaire, un cancer du poumon à petites cellules, un cancer du poumon non à petites cellules, un mélanome, un cancer de l'ovaire, un cancer du col de l'utérus, un cancer du pancréas, un carcinome de la prostate, une leucémie myéloïde aiguë (LMA), une leucémie myéloïde chronique (LMC), un lymphome non hodgkinien, ou un carcinome de la thyroïde.

**15.** Un composé de la revendication 13, où le composé est facultativement sous forme de sel pharmaceutiquement acceptable, facultativement encore sous forme de solvate, et facultativement encore sous forme d'hydrate de celui-ci, dans lequel ladite utilisation se fait en combinaison avec un ou plusieurs traitements sélectionnés parmi la chirurgie, un ou plusieurs agents chimiothérapeutiques, une ou plusieurs hormonothérapies, un ou plusieurs anticorps, une ou plusieurs immunothérapies, le traitement par l'iode radioactif, et le rayonnement.

**16.** Une méthode de préparation d'un composé de la revendication 1, comprenant les étapes suivantes :

(a) faire réagir un intermédiaire de Formule 1

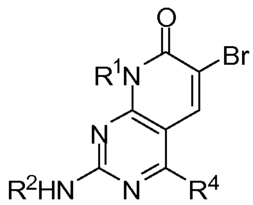

**1**

où $R^1$, $R^2$, et $R^4$ sont tels que définis à la revendication 1, avec un intermédiaire de formule $R^6Sn(n\text{-}Bu)_3$ ou $R^6B(OH)_2$ où $R^6$ et $R^9$ sont tels que définis à la revendication 1 pour obtenir un composé de la revendication 1 ;
(b) facultativement, modifier plus avant l'un des groupes $R^1$, $R^2$, $R^4$, et $R^6$ ; et
(c) facultativement, former un sel pharmaceutiquement acceptable, un solvate, et/ou un hydrate de celui-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60911160 B
- WO 9634867 A
- WO 2005105801 A
- WO 2006071819 A
- WO 05117909 A
- WO 2006108059 A
- WO 2005020921 A
- WO 2006033943 A
- WO 2005030140 A
- WO 06108059 A
- WO 2006014325 A
- WO 2004006846 A
- WO 2004050681 A
- WO 2004072051 A
- WO 2005028434 A
- WO 2007035620 A
- WO 2006091963 A
- WO 06074057 A
- WO 2005112932 A
- WO 2004101583 A
- US 7160867 B
- US 910720 A
- WO 2006074057 A
- US 722719 A
- US 722291 A
- US 571140 A
- WO 2005117909 A
- US 568173 A
- US 573336 A
- US 533555 A
- US 568789 A
- US 522004 A
- US 4107288 A
- US 5145684 A
- US 20040009993 A1, M. Angiolini
- US 2003220348 A, Xie, L.


### Non-patent literature cited in the description


- **CAMPBELL et al.** *Cancer Res,* 2004, vol. 64, 7678-7681
- **LEVINE et al.** *Clin Cancer Res,* 2005, vol. 11, 2875-2878
- **WANG et al.** *Hum Mutat,* 2005, vol. 25, 322
- **LEE et al.** *Gynecol Oncol,* 2005, vol. 97, 26-34
- **BACHMAN et al.** *Cancer Biol Ther,* 2004, vol. 3, 772-775
- **LI et al.** *Breast Cancer Res Treat,* 2006, vol. 96, 91-95
- **SAAL et al.** *Cancer Res,* 2005, vol. 65, 2554-2559
- **SAMUELS ; VELCULESCU.** *Cell Cycle,* 2004, vol. 3, 1221-1224
- **SAMUELS et al.** *Science,* 2004, vol. 304, 554
- **VELHO et al.** *Eur J Cancer,* 2005, vol. 41, 1649-1654
- **ODA et al.** *Cancer Res.,* 2005, vol. 65, 10669-10673
- **BYUN et al.** *Int J Cancer,* 2003, vol. 104, 318-327
- **LEE et al.** *Oncogene,* 2005, vol. 24, 1477-1480
- **TANG et al.** *Lung Cancer,* 2006, vol. 51, 181-191
- **MASSION et al.** *Am J Respir Crit Care Med,* 2004, vol. 170, 1088-1094
- **WU et al.** *J Clin Endocrinol Metab,* 2005, vol. 90, 4688-4693
- **SUJOBERT et al.** *Blood,* 1997, vol. 106, 1063-1066
- **HICKEY ; COTTER.** *J Biol Chem,* 2006, vol. 281, 2441-2450
- **HARTMANN et al.** *Acta Neuropathol (Berl),* 2005, vol. 109, 639-642
- **GOODMAN ; GILMAN et al.** The Pharmacological Basis of Therapeutics. Pergamon Press, 1990
- Remington's Pharmaceutical Sciences. Mack Publishing Company, 1985
- **S. M. BERGE et al.** Pharmaceutical Salts. *J. Pharm. Sci.,* 1977, vol. 66, 1-19
- **T. HIGUCHI ; V. STELLA.** Pro-drugs as Novel Delivery Systems. A.C.S. Symposium Series, vol. 14
- Bioreversible Carriers in Drug Design. American Pharmaceutical Association and Pergamon Press, 1987
- Remington's Pharmaceutical Sciences. Mack Publishing Company, 1990
- Fieser and Fieser's Reagents for Organic Synthesis. John Wiley and Sons, 1991, vol. 1-17
- Rodd's Chemistry of Carbon Compounds. Elsevier Science Publishers, 1989, vol. 1-5

- Organic Reactions. John Wiley and Sons, 1991, vol. 1-40
- March's Advanced Organic Chemistry. John Wiley and Sons
- Larock's Comprehensive Organic Transformations. VCH Publishers Inc, 1989
- **T.W. GREENE.** Protective Groups in Organic Synthesis. John Wiley & Sons, Inc, 1991
- **M. BARVIAN et al.** *J. Med. Chem.,* 2000, vol. 43, 4606-4616
- **S. N. VANDERWEI et al.** *J. Med. Chem.,* 2005, vol. 48, 2371-2387
- **P. L. TOOGOOD et al.** *J. Med. Chem.,* 2005, vol. 48, 2388-2406
- **J. KASPAREC et al.** *Tetrahedron Letters,* 2003, vol. 44, 4567-4570